# EUROPEAN PATENT APPLICATION

(11) **EP 4 837 974 A2**
(43) Date of publication of application: **11.11.2026**
(21) Application number: 26166061.7
(22) Date of filing: 19.03.2026
(51) Int. Cl.: C12Q 1/6886

(54) **METHODS FOR PREDICTING RESPONSIVENESS TO DARATUMUMAB IN A CANCER SUBJECT**

(30) Priority: 20.03.2025 US 202563774825 P
(71) Applicant: Allarity Therapeutics, Inc., Boston MA 02108 (US)
(72) Inventor: KNUDSEN, Steen, 3370 Melby (DK)
(74) Representative: Mewburn Ellis LLP

(57) **Abstract**

Featured are methods, devices, and kits for detecting gene expression in a subject with a cancer and methods for determining responsiveness of a subject with a cancer to a treatment, such as treatment with daratumumab. Also disclosed are methods of treating a subject patient with a cancer by administering a treatment, e.g., treatment with daratumumab, in particular when the subject is determined to be responsive to the treatment based on the expression of the biomarkers described herein.

## Description

### SEQUENCE LISTING

The instant application contains a Sequence Listing which has been submitted electronically in XML format and is hereby incorporated by reference in its entirety. Said XML copy, created on March 13, 2025, is named "51167-034001_Sequence_Listing_3_13_25.xml" and is 58,266 bytes in size.

### FIELD OF THE INVENTION

The use of biomarkers to predict the responsiveness of a subject (e.g., a subject having a cancer) to a cancer therapy (e.g., a cancer therapy for treating the cancer).

### BACKGROUND

Gene expression measurements in a tissue sample have been used to facilitate the determination of a disease state in a subject or a subject's responsiveness to a therapy for a disease. For example, gene expression measurements can be used to reveal the presence of cancer in a subject, as well as, in some cases, the type, stage, and origin of the cancer. Gene expression may even have a role in predicting the efficacy of a cancer therapy. In recent years, the Cancer Cell Line Encyclopedia (CCLE) has tested the baseline gene expression of about 1000 human cancer cell lines, including using DNA microarrays. Various studies have explored the relationship between gene expression and therapeutic effect using CCLE data as a baseline for comparison.

During cancer treatment, critical time is often lost due to a trial and error approach to finding an effective therapy. In addition, cancer cells often develop resistance to a previously effective therapy. In such situations, patient outcome would be greatly improved by early detection of such resistance.

Thus, there exists a need in the art for methods and devices that can predict the responsiveness of a cancer patient to a medical treatment (e.g., a cancer therapy).

### SUMMARY OF THE INVENTION

Featured are methods for detecting the level of expression of one or more biomarkers (e.g., one, two, three, four, five, ten, fifteen, twenty, twenty five, twenty six, or twenty seven) or all of the biomarkers shown in Tables 1 and/or 2, such as NR3C1 (SEQ ID NO: 1) and/or CCND1 (SEQ ID NO: 28), respectively, in a subject, such as a subject with a cancer (e.g., a subject with multiple myeloma (MM) or non-Hodgkin's lymphoma (NHL); e.g., a subject with MM) in order to determine the responsiveness of the subject to treatment with daratumumab or a pharmaceutically acceptable composition thereof . Also featured are methods of treating cancer in a subject in need thereof (e.g., a subject with MM or NHL or a recurrence thereof; e.g., a subject with MM or a recurrence thereof) that include administering daratumumab to the subject, in which the subject is or has been determined to be responsive to daratumumab according to the methods described herein. The methods described herein may be *in vitro* methods. The methods described herein may be *in vitro* diagnostic methods.

Exemplary types of cancer for which responsiveness to daratumumab can be determined include, e.g., multiple myeloma or non-Hodgkin's lymphoma (e.g., a diffuse large B cell lymphoma, follicular lymphoma, mantle cell lymphoma, or Burkitt's lymphoma). In a particular embodiment, the cancer is multiple myeloma (MM).

In a first aspect, the disclosure features a method of determining responsiveness of a subject with a cancer to treatment with daratumumab that includes: (a) determining the level of expression of one or more biomarkers of sensitivity selected from the biomarkers of Table 1; and/or (b) determining the level of expression of one or more biomarkers of resistance selected from the biomarkers of Table 2. In particular, the methods include determining the level of expression of NR3C1 (SEQ ID NO: 1) as the biomarker of sensitivity and CCND1 (SEQ ID NO: 28) as the biomarker of resistance.

In some embodiments, the subject is determined to be responsive to daratumumab if: (a) the level of expression of the one or more biomarkers of sensitivity is substantially similar to the level of expression of the one or more biomarkers of sensitivity in a cell or tissue known to be sensitive to daratumumab (or relative to a reference level of the biomarker(s) of sensitivity, such as the level of expression of the one or more biomarkers of sensitivity in a sample from a reference subject with the same cancer as the subject); (b) the level of expression of the one or more biomarkers of resistance is substantially similar to the level of expression of the one or more biomarkers of resistance in a cell or tissue known to be sensitive to daratumumab (or relative to a reference level of the biomarker(s) of resistance, such as the level of expression of the one or more biomarkers of resistance in a sample from a reference subject with the same cancer as the subject); (c) the level of expression of the one or more biomarkers of sensitivity is substantially dissimilar to the level of expression of the one or more biomarkers of sensitivity in a cell or tissue known to be resistant to daratumumab; and/or (d) the level of expression of the one or more biomarkers of resistance is substantially dissimilar to the level of expression of the one or more biomarkers of resistance in a cell or tissue known to be resistant to daratumumab.

In some embodiments, in the methods described herein, (a) sensitivity of a cell or tissue known to be sensitive to daratumumab is based on Cancer Cell Line Encyclopedia (CCLE) data of B cell lymphoma cell lines; and/or (b) resistance of a cell or tissue known to be resistant to daratumumab is based on CCLE data of B cell lymphoma cell lines.

In some embodiments, the methods described herein further include administering daratumumab to the subject determined to be sensitive to daratumumab.

In a second aspect, the disclosure features a method of treating a cancer in a subject in need thereof by administering daratumumab to the subject, wherein the subject has been determined to be responsive to daratumumab according to any of the methods described herein (e.g., the method of the first aspect).

In a third aspect, the disclosure features a method of treating a subject with a cancer including: (a) determining the level of expression of one or more biomarkers of sensitivity and/or determining the level of expression of one or more biomarkers of resistance; and (b) administering daratumumab to the subject based on the level of expression of the one or more biomarkers of sensitivity and/or the one or more biomarkers of resistance.

In some embodiments, the subject is administered daratumumab if: (a) the level of expression of the one or more biomarkers of sensitivity is substantially similar to the level of expression of the one or more biomarkers of sensitivity in a cell or tissue known to be sensitive to daratumumab (or relative to a reference level of the biomarker(s) of sensitivity, such as the level of expression of the one or more biomarkers of sensitivity in a sample from a reference subject with the same cancer as the subject); (b) the level of expression of the one or more biomarkers of resistance is substantially similar to the level of expression of the one or more biomarkers of resistance in a cell or tissue known to be sensitive to daratumumab (or relative to a reference level of the biomarker(s) of resistance, such as the level of expression of the one or more biomarkers of resistance in a sample from a reference subject with the same cancer as the subject); (c) the level of expression of the one or more biomarkers of sensitivity is substantially dissimilar to the level of expression of the one or more biomarkers of sensitivity in a cell or tissue known to be resistant to daratumumab; and/or (d) the level of expression of the one or more biomarkers of resistance is substantially dissimilar to the level of expression of the one or more biomarkers of resistance in a cell or tissue known to be resistant to daratumumab.

In some embodiments, in the methods described herein, (a) sensitivity of a cell or tissue known to be sensitive to daratumumab is based on CCLE data of B cell lymphoma cell lines; and/or (b) resistance of a cell or tissue known to be resistant to daratumumab is based on CCLE data of B cell lymphoma cell lines.

In some embodiments, the methods described herein further include administering one or more therapies other than daratumumab to the subject, wherein, optionally, the one or more therapies include surgery, radiation, or a therapeutic agent.

In some embodiments, the therapeutic agent includes a corticosteroid, an antipyretic, an antihistamine, a chemotherapeutic agent, or a combination thereof. In some embodiments, (a) the corticosteroid is methylprednisolone or dexamethasone; (b) the antipyretic is acetaminophen/paracetamol; and/or (c) the antihistamine is diphenhydramine.

In some embodiments, the methods described herein further include administering to the subject lenalidomide, dexamethasone, bortezomib, melphalan, prednisone, thalidomide, pomalidomide, or a combination thereof. In some embodiments, the methods described herein include administration to the subject of one of the following combination therapies: (a) daratumumab, lenalidomide, and dexamethasone; (b) daratumumab, bortezomib, melphalan, and prednisone; (c) daratumumab, bortezomib, thalidomide, and dexamethasone; (d) daratumumab, bortezomib, and dexamethasone; or (e) daratumumab, pomalidomide, and dexamethasone.

In some embodiments, daratumumab is administered intravenously. In some embodiments, daratumumab is administered via intravenous infusion.

In some embodiments, a dose of daratumumab is administered once a week, once every two weeks, once every three weeks, and/or once every four weeks. In some embodiments, the dose of daratumumab administered to the subject is about 16 mg/kg. In some embodiments, the dose of daratumumab is administered as two sub-doses, wherein each sub-dose is half the amount of the dose (e.g., 8 mg/kg for each sub-dose). In some embodiments, the second sub-dose is administered one day after administration of the first sub-dose.

In some embodiments, daratumumab is administered in a dosing schedule including: (a) 1 dose per week for weeks 1-8, followed by 1 dose every two weeks starting on week 9 for weeks 9-24, and then followed by 1 dose every four weeks thereafter starting on week 25; (b) 1 dose per week for weeks 1-6, followed by 1 dose every three weeks starting on week 7 for weeks 7-54, and then followed by 1 dose every four weeks thereafter starting on week 55; (c) 1 dose per week for weeks 1-9, followed by 1 dose every three weeks starting on week 10 for weeks 10-24, and then followed by 1 dose every four weeks thereafter starting on week 25; (d) 1 dose per week for weeks 1-8, followed by 1 dose every two weeks starting on week 9 for weeks 9-16, followed by a pause, and then followed by 1 dose every two weeks for weeks 1-8 after reinitiation of dosing; or (e) 1 dose split into two sub-doses, each including half the amount of the dose, administered on Days 1 and 2 of week 1, 1 dose per week for weeks 2-8, followed by 1 dose every two weeks starting on week 9 for weeks 9-24, and then followed by 1 dose every four weeks thereafter starting on week 25.

In some embodiments, the level of expression of the one or more biomarkers of sensitivity and/or the level of expression one or more biomarkers of resistance is determined using a device. In some embodiments, the device is a microarray. In some embodiments, the microarray is a deoxyribonucleic acid (DNA)-based platform. In some embodiments, the device includes at least two, at least three, at least four, at least five, at least six, at least seven, at least eight, at least nine, at least ten, or more single-stranded nucleic acid molecules of capable of hybridizing to the one or more biomarkers of sensitivity and/or the one or more biomarkers of resistance. In some embodiments, the one or more single-stranded nucleic acid molecules of the device have a length in the range of 10-100 nucleotides.

In some embodiments, the level of expression of the one or more biomarkers of sensitivity and/or the level of expression of the one or more biomarkers of resistance is determined by microarray analysis, mass spectrometry, a nucleic acid amplification method, a nucleic acid sequencing method, an imaging-based method, or a gel-based method. In some embodiments, (a) the nucleic acid amplification method includes polymerase chain reaction (PCR), reverse transcriptase PCR (RT-PCR), or quantitative real-time PCR (qPCR); or (b) the nucleic acid sequencing method includes pyrosequencing, nanopore sequencing, sequencing by synthesis, sequencing by expansion, sequencing by ligation, or next generation sequencing; (c) the imaging-based method includes fluorescence *in situ* hybridization, enzyme-linked immunosorbent assay (ELISA), nanostring, or immunohistochemistry; or (d) the gel-based method includes a Northern blot, a Western blot, or a Southern blot.

In some embodiments, the level of expression of the one or more biomarkers of sensitivity and/or the level of expression of the one or more biomarkers of resistance is an RNA expression level or a protein expression level. In some embodiments, the RNA expression level is an mRNA expression level.

In some embodiments, the method includes converting the level of expression of the one or more biomarkers of sensitivity and/or the level of expression of the one or more biomarkers of resistance into a mean score, wherein the mean score indicates the responsiveness of the subject to daratumumab. In some embodiments, the method further includes subtracting the mean score for the one or more biomarkers of resistance from the mean score for the one or more biomarkers of sensitivity to obtain a difference score, wherein the difference score indicates the responsiveness of the subject to daratumumab. In some embodiments, the level of expression of the one or more biomarkers of sensitivity and/or the level of expression of the one or more biomarkers of resistance is determined in a bone marrow sample or a blood sample obtained from the subject. In some embodiments, the bone marrow sample and/or the blood sample includes plasma cells.

In some embodiments, the mean score for the one or more biomarkers of sensitivity and/or the difference score above a cutoff value indicates that the subject is responsive to daratumumab. In some embodiments, the cutoff value is established as the 50^{th} percentile, 60th percentile, 70^{th} percentile, 80^{th} percentile, 90^{th} percentile, or greater of a set of reference mean scores or a set of reference difference scores determined in a reference population. In some embodiments, the reference population includes individuals having the same cancer as the subject. In some embodiments, the set of reference mean scores or the set of reference difference scores is: (a) a set including the mean scores for the one or more biomarkers of sensitivity calculated for each individual of the reference population; (b) a set including the mean scores for the one or more biomarkers of resistance calculated for each individual of the reference population; or (c) a set including the difference scores calculated for each individual of the reference population. In some embodiments, the reference mean score or the reference difference score is determined in a reference bone marrow sample or a reference blood sample obtained from the individuals of the reference population. In some embodiments, the reference bone marrow sample or the reference blood sample includes plasma cells.

In some embodiments, (a) the biomarkers of sensitivity are selected from at least 5, at least 10, at least 15, at least 20, at least 25, or all 27 of the biomarkers of Table 1; and/or the biomarkers of resistance are selected from at least 5, at least 10, at least 15, at least 20, at least 25, or all 26 of the biomarkers of Table 2; (b) the biomarkers of sensitivity are selected from one or more of SEQ ID NOs: 1-27 and/or the biomarkers of resistance are selected from one or more of SEQ ID NOs: 28-53; and/or (c) the biomarker of sensitivity is NR3C1 (SEQ ID NO: 1); and/or the biomarker of resistance is CCND1 (SEQ ID NO: 28). In some embodiments, the biomarker of sensitivity is NR3C1 (SEQ ID NO: 1) and the biomarker of resistance is CCND1 (SEQ ID NO: 28).

In some embodiments, the cancer is multiple myeloma. In some embodiments, the subject has not received prior therapy for the multiple myeloma. In some embodiments, the subject has relapsed after or is refractory to at least one prior therapy for treating the multiple myeloma. In some embodiments, the at least one prior therapy includes an alkylating agent, a proteasome inhibitor, and/or an immunomodulatory agent. In some embodiments, the at least one prior therapy includes bortezomib, lenalidomide, pomalidomide, carfilzomib, autologous stem cell transplant therapy, or a combination thereof. In some embodiments, the subject has relapsed after or is refractory to one, two, or three prior lines of therapy.

In some embodiments, the cancer is a B cell lymphoma. In some embodiments, the B cell lymphoma is a non-Hodgkin's lymphoma (NHL). In some embodiments, the subject is ineligible for autologous stem cell transplant therapy.

### Definitions

As used herein, "a" or "an" means "at least one" or "one or more" unless otherwise indicated. In addition, the singular forms "a", "an", and "the" include plural referents unless the context clearly dictates otherwise.

As used herein, "about" refers to an amount that is ± 10% of the recited value.

As used herein, the term "daratumumab" refers to an anti-CD38 monoclonal antibody described in the World Health Organization's (WHO) Recommended International Nonproprietary Names: List 63, WHO Drug Information, Vol. 24, No. 1, 2010). Daratumumab is an immunoglobulin G1 kappa (IgG1κ) human monoclonal antibody that binds to CD38 antigen. It is produced in Chinese Hamster Ovary (CHO) cells using recombinant DNA technology. The molecular weight of daratumumab is approximately 148 kDa. Daratumumab may be sold under the commercial name DARZALEX^{®}. Daratumumab has the CAS number 945721-28-8. Daratumumab has been granted orphan drug status for multiple myeloma, diffuse large B cell lymphoma, follicular lymphoma, and mantle cell lymphoma. Daratumumab has a heavy chain complementarity determining region 1 (HCDR-1) sequence of SFAMS (SEQ ID NO: 54); an HCDR-2 sequence of AISGSGGGTYYADSVKG (SEQ ID NO: 55); an HCDR-3 sequence of DKILWFGEPVFDY (SEQ ID NO: 56); a light chain complementarity determining region 1 (LCDR-1) sequence of RASQSVSSYLA (SEQ ID NO: 57); an LCDR-2 sequence of DASNRAT (SEQ ID NO: 58); and an LCDR-3 sequence of QQRSNWPPT (SEQ ID NO: 59). Daratumumab has the following variable heavy chain domain (VH) and variable light chain domain (VL) sequences:
VH (SEQ ID NO: 60): 

.
VL (SEQ ID NO: 61): 

.
Additionally, Daratumumab has the heavy chain sequence (SEQ ID NO: 62): 
EVQLLESGGGLVQPGGSLRLSCAVSGFTFNSFAMSWVRQAPGKGLEWVSAISGSGGGTYYADSVK
GRFTISRDNSKNTLYLQMNSLRAEDTAVYFCAKDKILWFGEPVFDYWGQGTLVTVSSASTKGPSVFP
LAPSSKSTSGGTAALGCLVKDYFPEPVTVSWNSGALTSGVHTFPAVLQSSGLYSLSSVVTVPSSSLG
TQTYICNVNHKPSNTKVDKRVEPKSCDKTHTCPPCPAPELLGGPSVFLFPPKPKDTLMISRTPEVTCV
VVDVSHEDPEVKFNWYVDGVEVHNAKTKPREEQYNSTYRVVSVLTVLHQDWLNGKEYKCKVSNKA
 
LPAPIEKTISKAKGQPREPQVYTLPPSREEMTKNQVSLTCLVKGFYPSDIAVEWESNGQPENNYKTTP
PVLDSDGSFFLYSKLTVDKSRWQQGNVFSCSVMHEALHNHYTQKSLSLSPGK.
And the light chain sequence (SEQ ID NO: 63):

EIVLTQSPATLSLSPGERATLSCRASQSVSSYLAWYQQKPGQAPRLLIYDASNRATGIPARFSGSGSG
TDFTLTISSLEPEDFAVYYCQQRSNWPPTFGQGTKVEIKRTVAAPSVFIFPPSDEQLKSGTASVVCLL
NNFYPREAKVQWKVDNALQSGNSQESVTEQDSKDSTYSLSSTLTLSKADYEKHKVYACEVTHQGLS
SPVTKSFNRGEC.

By "biomarker" is meant a nucleic acid molecule (e.g., an mRNA or its complement, for example, a cDNA) or a protein encoded by the nucleic acid molecule present in, or from, a cell or tissue (e.g., tumor tissue). The expression of the biomarker correlates to the responsiveness (e.g., sensitivity or resistance) of the cell or tissue (and thus, the patient containing the cell or tissue or the patient from which the cell or tissue was obtained) to a cancer treatment (e.g., daratumumab). In particular, a biomarker of sensitivity is a nucleic acid molecule (e.g., a mRNA or its complement) expressed from any one of the genes shown in Table 1, or the protein encoded by the nucleic acid molecule, and a biomarker of resistance is a nucleic acid molecule (e.g., a mRNA or its complement) expressed from any one of the genes shown in Table 2, or the protein encoded by the nucleic acid molecule.

The terms "cancer" and "cancerous" refer to or describe the physiological condition in mammals (e.g., humans) that is typically characterized by unregulated cell proliferation. Examples of cancer include, but are not limited to, multiple myeloma (MM) or non-Hodgkin's lymphoma (NHL; e.g., a diffuse large B cell lymphoma, follicular lymphoma, mantle cell lymphoma, or Burkitt's lymphoma). Methods provided herein for the treatment of cancer include the treatment of MM and NHL. In a particular instance, the methods described herein are used in the treatment of MM and/or the determination of whether a patient with MM will be responsive to a cancer therapy (e.g., to daratumumab).

The terms "expression level" and "level of expression," as used herein interchangeably, refer to the amount of a gene product in a cell, tissue, biological sample, organism, or patient, e.g., amounts of DNA, RNA (e.g. messenger RNA (mRNA) of), or protein encoded by a given gene.

"Gene" as used herein indicates a coding or noncoding gene whose activity can be determined by measuring the produced RNA. Examples include protein coding genes, microRNAs, small nuclear RNAs and other RNAs with catalytic, regulatory or coding properties.

To "inhibit growth" as used herein means causing a reduction in cell growth (e.g., cancer cell growth (e.g., multiple myeloma (MM) or non-Hodgkin's lymphoma (NHL) cell lines); e.g., MM cell lines) *in vivo* or *in vitro* by, e.g., 10%, 20%, 30%, 40%, 50%, 60%, 70%, 80%, 90%, 95%, or 99% or more, as evident by a reduction in the proliferation of cells exposed to a treatment (e.g., daratumumab), relative to the proliferation of cells in the absence of the treatment. Growth inhibition may be the result of a treatment (e.g., daratumumab) that induces apoptosis in a cell, induces necrosis in a cell, slows cell cycle progression, disrupts cellular metabolism, induces cell lysis, or induces some other mechanism that reduces the proliferation of cells.

"Microarray" as used herein means a device employed by any method that quantifies one or more subject oligonucleotides, e.g., RNA, DNA, cDNA, or analogues thereof, at a time. For example, many DNA microarrays, including those made by Affymetrix (e.g., an Affymetrix HG-U133A array), use several probes for determining the expression of a single gene. The DNA microarray may contain oligonucleotide probes that may be, e.g., full-length cDNAs complementary to an RNA or cDNA fragments that hybridize to part of an RNA. The DNA microarray may also contain modified versions of DNA or RNA, such as locked nucleic acids or LNA. Exemplary RNAs include mRNA, miRNA, and miRNA precursors.

As used herein, the term "percent (%) sequence identity" refers to the percentage of nucleic acid residues of a candidate sequence, e.g., a probe or primer of the invention, that are identical to the nucleic acid residues of a reference sequence, e.g., a biomarker sequence of the invention, after aligning the sequences and introducing gaps, if necessary, to achieve the maximum percent sequence identity (e.g., gaps can be introduced in one or both of the candidate and reference sequences for optimal alignment and non-homologous sequences can be disregarded for comparison purposes). Alignment for purposes of determining percent sequence identity can be achieved in various ways that are within the skill in the art, for instance, using computer software, such as BLAST, BLAST-2, BLAST-P, BLAST-N, BLAST-X, WU-BLAST-2, ALIGN, ALIGN-2, CLUSTAL, MEGALIGN (DNASTAR). In addition, those skilled in the art can determine appropriate parameters for measuring alignment, including any algorithms needed to achieve optimal alignment over the length of the sequences being compared.

"CCLE" or "Cancer Cell Line Encyclopedia" as used herein refers to a database maintained by the CCLE of cancer cell line expressions, e.g., as maintained on broadinstitute.org and depmap.org. In some instances, the CCLE cancer cell line expression was determined by microarray (e.g., using Affymetrix^{®}).

The terms "patient" and "subject," as used interchangeably herein, refer to any animal (e.g., a mammal, such as a human). A patient to be treated or tested for responsiveness to a treatment (e.g., daratumumab) according to the methods described herein may be one who has been diagnosed with a cancer, such as multiple myeloma (MM) or non-Hodgkin's lymphoma (NHL). In a particular instance, the patient has been diagnosed with MM. Diagnosis may be performed by any method or techniques known in the art, such as x-ray, MRI, or biopsy, and confirmed by a physician. To minimize exposure of a patient to drug treatments that may not be therapeutic, the patient may be determined to be either responsive or non-responsive to a cancer treatment, such as daratumumab, according to the methods described herein.

"Resistance" as used herein means that a cell (e.g., a cancer cell) or a tissue (e.g., a tumor) *in vitro* or *in vivo* (e.g., in a subject with a cancer, such as a human) is tolerant to treatment with an anti-cancer agent (e.g., daratumumab), e.g., the cell or tissue (e.g., tumor tissue) is able to survive and grow despite exposure to (e.g., treatment with) an anti-cancer agent (e.g., daratumumab). Resistance may arise via exploitation by a cell or tissue (e.g., a tumor tissue) of one or more of drug inactivation, drug target alteration, drug efflux, DNA damage repair, cell death inhibition, cell cycle regulation, epithelial-mesenchymal transition (EMT), epigenetics, and other mechanisms. A "resistant" cell or tissue refers to a cell (e.g., a cancer cell) or a tissue (e.g., a tumor), respectively, *in vitro* or *in vivo* (e.g., in a subject with a cancer, such as a human) that has acquired and/or exhibits resistance to a treatment (e.g., daratumumab). For example, a resistant cell or tissue (e.g., a tumor tissue) is one that, upon exposure of the cell (e.g., a cancer cell) or the tissue (e.g., a tumor), respectively, to a cancer therapeutic (e.g., daratumumab) exhibits an inhibition in growth of the cell or tumor of less than 30%, 25%, 20%, 15%, 10%, 5%, or 1% relative to the growth of a cell or tissue not exposed to the treatment. Resistance to treatment may be determined by a cell lysis assay, e.g., a calcein release assay or a calcein acetoxymethyl ester (calcein-AM) release ADCC assay (e.g., wherein target cancer cells are incubated with calcein-AM and peripheral blood mononuclear cells (PBMCs) containing natural killer (NK) effector cells), and fluorescence from calcein-AM is measured to determine % cell lysis (e.g., after normalizing for spontaneous calcein-AM release and as compared to total calcein-AM release by, e.g., addition of detergent). In this assay, greater fluorescence indicates a greater number of viable cells.

The terms "sensitivity" and "responsiveness," as used herein, refer to the likelihood that a cancer treatment (e.g., daratumumab) has (e.g., induces) a desired effect, or alternatively refers to the strength of a desired effect caused or induced by the treatment in a cell (e.g., a cancer cell) or a tissue (e.g., a tumor) *in vitro* or *in vivo* (e.g., in a subject with a cancer, such as a human; e.g., a subject with MM). For example, the desired effect can include lysis of cancer cells *in vitro* by more than 30%, 35%, 40%, 45%, 50%, 55%, 60%, 65%, 70%, 75%, 80%, 85%, 90%, 95%, or 100% of the cancer cells of a sample. The desired effect can also include reduction in tumor mass by, e.g., about 30%, 35%, 40%, 45%, 50%, 55%, 60%, 65%, 70%, 75%, 80%, 85%, 90%, 95%, or 100%. "Sensitive" and "responsive" as used herein refer to a cell (e.g., a cancer cell) or a tissue (e.g., a tumor) *in vitro* or *in vivo* (e.g., in a subject with a cancer, such as a human) that is responsive to exposure to a therapeutic (e.g., daratumumab). Responsiveness to treatment may be determined by a cell lysis assay, e.g., a cell-based assay, which measures the % lysis of cancer cells, such as the calcein release assay described herein.

The term "sample" or "biological sample," as used herein, refers to any specimen (such as cells, tissue (e.g., a tissue sample obtained by biopsy; e.g., a bone marrow biopsy; i.e., a bone marrow sample), blood, serum, plasma, urine, cerebrospinal fluid, or pancreatic fluid) taken from a subject. Preferably, the sample is taken from a portion of the body affected by a cancer (e.g., a biopsy of the cancer tissue). Biopsy may involve fine needle aspiration biopsy, core needle biopsy (e.g., stereotactic core needle biopsy, vacuum-assisted core biopsy, or magnetic resonance imaging (MRI) guided biopsy), or surgical biopsy (e.g., incisional biopsy or excisional biopsy). The sample may undergo additional purification and processing, for example, to remove cell debris and other unwanted molecules. Additional processing may further involve amplification, e.g., using PCR (e.g., RT-PCR). The standard methods of sample purification, such as removal of unwanted molecules, are known in the art.

"Substantially similar" or "corresponds," as used herein with respect to a numerical value of a parameter of one or more of the biomarker(s) of sensitivity and/or resistance (e.g., biomarker expression level, difference score, or mean score), e.g., as determined in a test sample (e.g., a tumor biopsy) from a cancer patient, means that the numerical value of the parameter in the test sample is ± 0-30% of the numerical value of the parameter in a reference sample (e.g., a cell (e.g., a cancer cell) or tissue (e.g., a tumor), such as a cell or a tissue obtained from a subject having the same cancer as a subject from whom the test sample was obtained) known to be sensitive or resistant to daratumumab). For example, a numerical value of a parameter in a test sample may be substantially similar to, or may correspond to, the numerical value of the parameter in a reference sample if the parameter values of the test and reference samples differ by, e.g., less than 30%, less than 29%, less than 28%, less than 27%, less than 26%, less than 25%, less than 24%, less than 23%, less than 22%, less than 21%, less than 20%, less than 19%, less than 18%, less than 17%, less than 16%, less than 15%, less than 14%, less than 13%, less than 12%, less than 11%, less than 10%, less than 9%, less than 8%, less than 7%, less than 6%, less than 5%, less than 4%, less than 3%, less than 2%, or less than 1%.

"Substantially dissimilar," as used herein with respect to a numerical value of a parameter of one or more of the biomarker(s) of sensitivity and/or resistance (e.g., biomarker expression level, difference score, or mean score), e.g., as determined in a test sample (e.g., a tumor biopsy) from a cancer patient, means that the numerical value of the parameter in the test sample deviates by greater than 30% from the numerical value of the parameter in a reference sample (e.g., a cell (e.g., a cancer cell) or tissue (e.g., a tumor), such as a cell or a tissue obtained from a subject having the same cancer as a subject from whom the test sample was obtained) known to be sensitive or resistant to daratumumab). For example, a numerical value of a parameter in a test sample may be substantially dissimilar to the numerical value of the parameter in a reference sample if the parameter values of the test and reference samples differ by, e.g., greater than 30%, 35%, 40%, 45%, 50%, 55%, 60%, 65%, 70%, 75%, 80%, 85%, 90%, 95%, or 100%.

"Treatment," "medical treatment," to "treat," and "therapy," as used interchangeably herein, refer to administering or exposing a patient (e.g., a human) with a cancer (e.g., multiple myeloma or non-Hodgkin's lymphoma), a cancer cell, or a tumor to an anti-cancer agent (e.g., a drug, a protein, an antibody, a nucleic acid, a chemotherapeutic agent, or a radioactive agent; e.g., an antibody; e.g., daratumumab), or to some other form of medical intervention used to treat or prevent a disease, disorder, or condition (e.g., surgery, cryotherapy, radiation therapy, or combinations thereof). In particular, a medical treatment can include daratumumab. For example, the cancer to be treated is multiple myeloma or non-Hodgkin's lymphoma (e.g., follicular lymphoma, diffuse large B cell lymphoma, mantle cell lymphoma, or Burkitt's lymphoma). Radiation therapy includes the administration of a radioactive agent to a patient or exposure of a patient to radiation. The radiation may be generated from sources such as particle accelerators and related medical devices or agents that emit, e.g., X-radiation, gamma radiation, or electron (Beta radiation) beams. A treatment may be or further include surgery, e.g., to remove a tumor from a subject or living organism.

Other features and advantages of the disclosure will be apparent from the following Detailed Description, the drawings, and the claims.

### BRIEF DESCRIPTION OF THE DRAWINGS

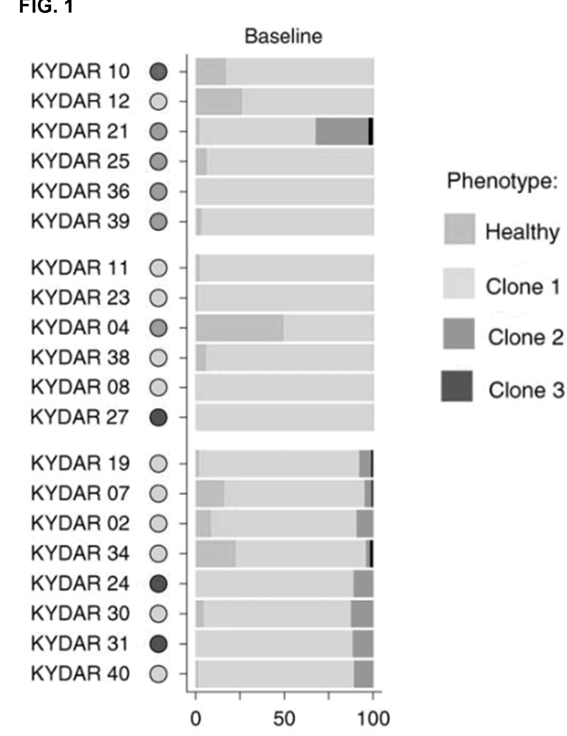

**FIG. 1** shows barplots from Yael et al. (Nature Medicine. 27(3):491-503, 2021) depicting malignant PC clonality at baseline (pretreatment).

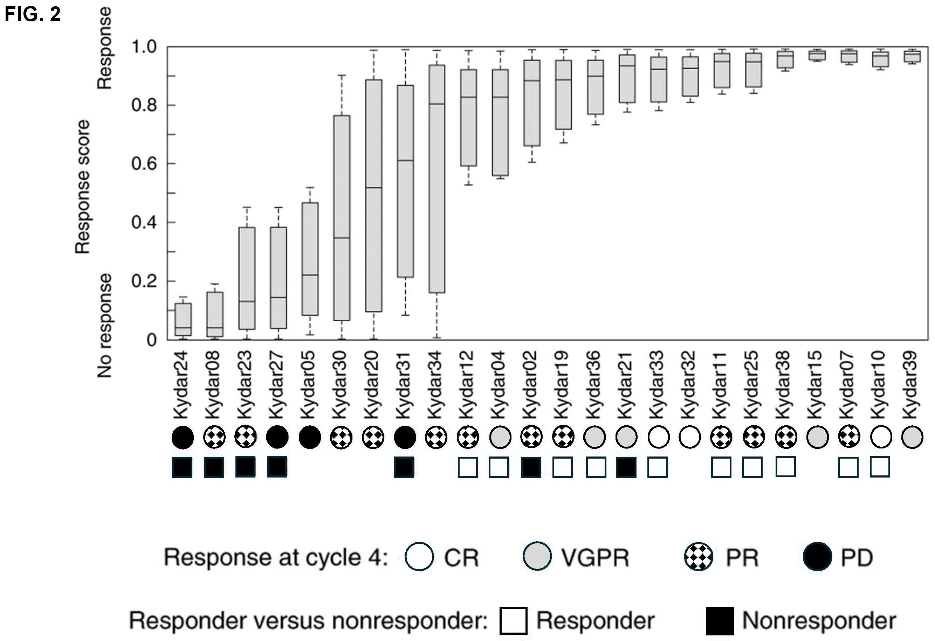

**FIG. 2** is a boxplot from Yael et al. (Nature Medicine. 27(3):491-503, 2021) showing the response score of individual cells from 24 individual patients. A shallow neural network classifier was used in Yael et al (i.e., not a DRP) to predict the response to treatment after 4 months. The center line represents the median, the box limits the upper and lower quartiles, and the whiskers 1.5× the interquartile range. CR: complete response; VGPR: very good partial response; PR: partial response; PD: progressive disease.

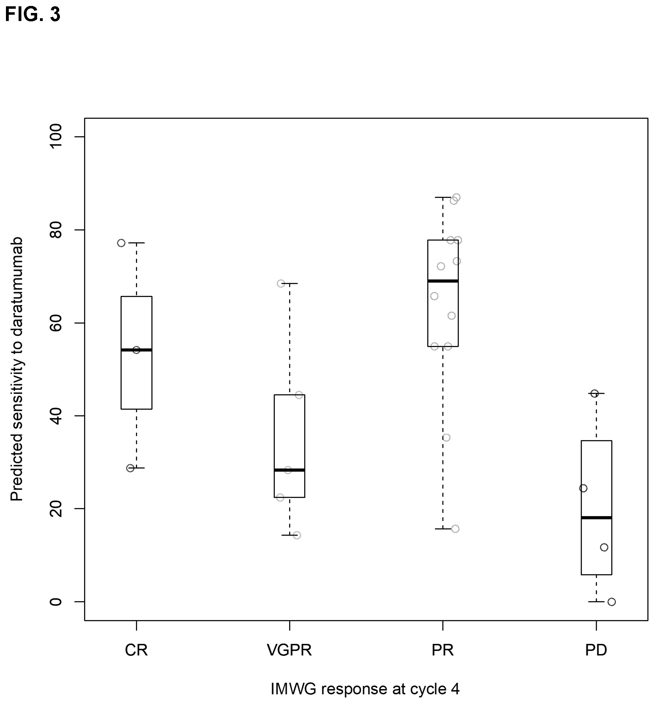

**FIG. 3** is a graph showing DRP-Daratumumab scores in clone 1 from 24 KYDAR patients where cycle 4 response evaluation was performed. The Pearson correlation between DRP score and numerically encoded response categories is -0.26 (95% CI -0.60 - 0.16). Note that 6 PR responses are transient in cycle 4. CR: complete response; VGPR: very good partial response; PR: partial response; PD: disease progression.

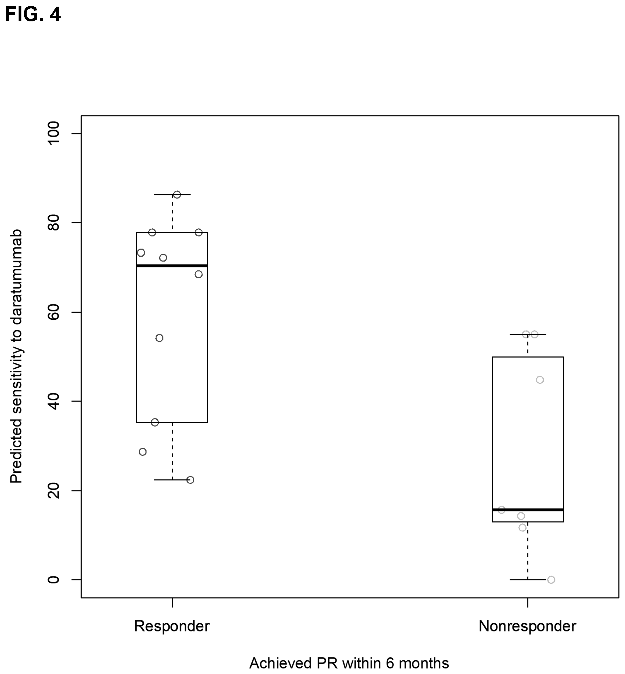

**FIG. 4** is a graph showing DRP-Daratumumab scores in clone 1 from 17 KYDAR patients where response evaluation was performed. The Pearson correlation between DRP score and numerically encoded response categories is -0.59 (95% CI -0.83 - -0.15).

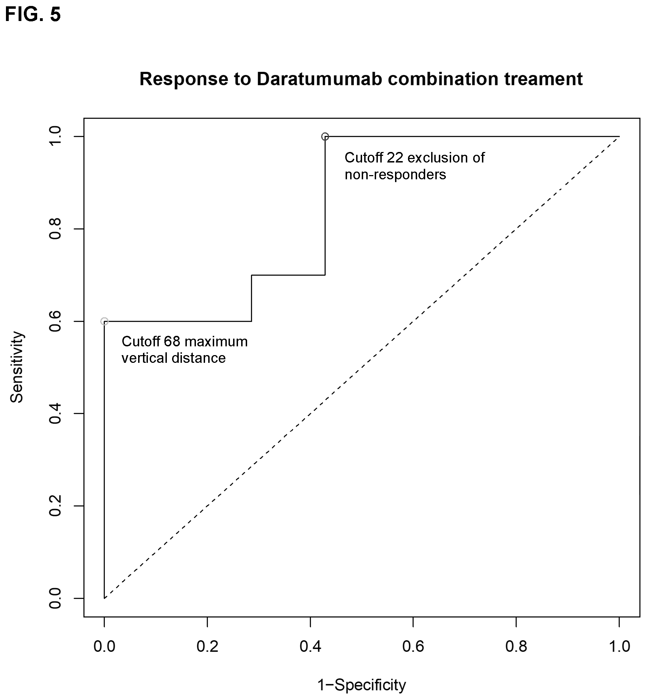

**FIG. 5** is a graph showing the receiver operating characteristic (ROC) curve of DRP-Daratumumab scores in clone 1 from KYDAR subjects where response evaluation was performed. The area under the curve is 0.84. A perfect classifier has an area of 1, a coin flip has an area of 0.5 (dotted line. Cutoff 22 and Cutoff 68 have a similar maximum vertical distance, which are considered optimums in classification theory. If using percentiles instead of a linear transformation of DRP score, the cutoffs 22 and 68 correspond to the 10th and 57th percentile of the KYDAR clone 1 population, respectively.

### DETAILED DESCRIPTION

The disclosure features methods of determining whether a subject with a cancer (e.g., multiple myeloma (MM) or non-Hodgkin's lymphoma (NHL); e.g., MM) will likely to responsive to treatment with daratumumab and methods for treating a subject that has been determined to be responsive to treatment with daratumumab by administering to the subject an effective amount of daratumumab. The methods include determining the levels of expression of sensitivity (e.g., one or more, or all, of the biomarkers shown in Table 1) and/or the levels of expression of resistance (e.g., one or more, or all, of the biomarkers shown in Table 2) to determine whether a subject with a cancer (e.g., MM or NHL; e.g., MM) will likely be responsive to daratumumab. A device, such as a microarray, with one or more single-stranded oligonucleotide probes that have substantial identity (e.g., at least 85%, 90%, 95%, 99%, or 100% sequence identity) to a sequence that is complementary or identical to the nucleic acid sequence of one or more (e.g., all) biomarkers shown in Tables 1 and/or 2 can be used according to the method described herein to assess the responsiveness of a subject with a cancer to treatment with daratumumab. For example, the probes can be used to detect one or more (e.g., one, two, three, four, five, ten, fifteen, twenty, twenty five, or all twenty seven) of the biomarkers of sensitivity listed in Table 1, such as NR3C1 (SEQ ID NO: 1), in a sample (e.g., a tumor sample) from a patient having cancer. Additionally, the probes can be used to detect one or more (e.g., one, two, three, four, five, ten, fifteen, twenty, twenty-five, or all twenty-six) of the biomarkers of resistance listed in Table 2, such as CCND1 (SEQ ID NO: 28), in a sample (e.g., a tumor sample) from a subject with cancer. Accordingly, the disclosure features individual biomarkers (e.g., NR3C1 (SEQ ID NO: 1) or CCND1 (SEQ ID NO: 28)) and sets of biomarkers, such as those shown in Tables 1 and/or 2, that can be used to determine the responsiveness of a subject with cancer to treatment with daratumumab at various stages of disease progression (e.g., a subject diagnosed with the cancer or a subject after cancer recurrence) and at different times during the treatment process (e.g., prior to administration of any cancer treatment, after administration of one or more cancer treatments other than daratumumab, prior to administration of daratumumab, or during administration of daratumumab).

Methods are described herein for identifying biomarkers (e.g., biomarkers of sensitivity and biomarkers of resistance) of drug responsiveness, detecting biomarker gene expression in a subject with cancer, determining the responsiveness of a subject with cancer to daratumumab based on the levels of expression of the biomarkers of sensitivity and/or biomarkers of resistance, and treating cancer in the subject with daratumumab. Also described are devices and kits for use in these methods.

The biomarkers of sensitivity and resistance to daratumumab are described in detail in Tables 1 and 2, respectively, below.

**Table 1. mRNA biomarkers of sensitivity to daratumumab.**

| Affymetrix IDs refer to the array type HG-U133_Plus_2. Sequences are representative probes from a probeset of typically 11 probe sequences. More than one probeset can target the same gene. | | | | |
|---|---|---|---|---|
| **Gene** | **Affymetrix ID** | **Correlation** | **Representative Probe Sequence** | **SEQ ID NO:** |
| NR3C1 | 211671_s_at | 0.605 | CTAGACTTTCTGTTGGTGTATCCCC | 1 |
| PARP8 | 219033_at | 0.598 | GCACACGATTCTTTTTCGTCTATGA | 2 |
| PSMB10 | 202659_at | 0.589 | GTGCTAGAAGACCGGTTCCAGCCGA | 3 |
| NR3C1 | 201865_x_at | 0.57 | AACCACCAGTGCCCAAAGTCTGTGT | 4 |
| ZNF593 | 204175_at | 0.56 | GGCGGCCGGACTTGGATGAGATTCA | 5 |
| ZPR1 | 200054_at | 0.554 | GAGCCTATTGTGTTACTTGACCTTA | 6 |
| TXNIP | 201010_s_at | 0.553 | GCTGAAACTGGTCTACTGTGTCTCT | 7 |
| ARHGEF2 | 207629_s_at | 0.551 | CCCTGCAACATCCGTCTTGGAGGAG | 8 |
| IL10RA | 204912_at | 0.543 | AGTACTCTTAGGTGCCAGTCTGGTA | 9 |
| TMEM123 | 211967_at | 0.521 | CCTTGAAAGTTTCCTCTGCATTGGG | 10 |
| TRIB2 | 202478_at | 0.519 | GACAAGGCGGCCATGGATTTGCCCT | 11 |
| LINC00623 | 222001_x_at | 0.518 | GGACACCTGCGCGTTAGGAGATTCT | 12 |
| LINC00869 | | | | |
| TAGLN2 | 200916_at | 0.514 | TGCCTACCATGGTCTGGGGCTTGAG | 13 |
| MCCC2 | 209624_s_at | 0.512 | CAGCCCTCAACGCACCAATAGAGAA | 14 |
| LOC101928789 | 207777_s_at | 0.494 | CAAGCTTTATTCAGGACACACTTCT | 15 |
| SP140 | | | | |
| SP110 | 208392_x_at | 0.493 | CTGAATGTGAGTTCCTCCTCTTGAA | 16 |
| - | 217152_at | 0.489 | GCTGGTCTAGGTGAAGTGGTGTTTA | 17 |
| MBP | 210136_at | 0.488 | AGAGAGCCACATTCTCAGGGTCTCT | 18 |
| STAT6 | 201331_s_at | 0.487 | GGTGGAGGCTTCTGATACGTGTATG | 19 |
| HIVEP2 | 212642_s_at | 0.487 | AAGAGTGTCACACTCCTGGCTATCT | 20 |
| SP110 | 208012_x_at | 0.483 | TGGATTTTCACTGTTCTAAGTCCCC | 21 |
| SEL1L3 | 212314_at | 0.48 | TTGACTTCCAAGACCTAGCAGTTAT | 22 |
| SP110 | 209762_x_at | 0.465 | TCACCTCCACATTTGGGCATGGCAT | 23 |
| EVI2B | 211742_s_at | 0.461 | TCCATCCTCAAATGACTCTTTTTTC; | 24 |
| NUP88 | 202900_s_at | 0.456 | GATACCTGATCAACTTCGACATTTG | 25 |
| SEL1L3 | 212311_at | 0.456 | GCAGTATTTCCAGTCTGTCTCAGCA | 26 |
| KIAA0247 | 202181_at | 0.454 | TGCCCTGGCTTCTTCAGGTTATCGC | 27 |

**Table 2. mRNA biomarkers of resistance to daratumumab.**

| Affymetrix IDs refer to the array type HG-U133_Plus_2. Sequences are representative probes from a probeset of typically 11 probe sequences. More than one probeset can target the same gene. | | | | |
|---|---|---|---|---|
| **Gene ID** | **Affymetrix ID** | **Correlation** | **Probe sequence** | **SEQ ID NO:** |
| CCND1 | 208712_at | -0.607 | AACCAAAAGAATTTGCACCCCGCTG | 28 |
| PSMC3IP | 213951_s_at | -0.606 | TAAAACGAGCATTTGGAGGGCCCAC | 29 |
| SF3B1 | 214305_s_at | -0.589 | ACACCAATTGGCACACCAGCCATGA | 30 |
| SF3B1 | 201070_x_at | -0.587 | TCCTCCAGCTGGTTATGTTCCTATT | 31 |
| CCL20 | 205476_at | -0.574 | GGTTTCACTTGCACATCATGGAGGG | 32 |
| UPP1 | 203234_at | -0.573 | GAAGGGCAAGGCCGTCTGGATGGGG | 33 |
| KPNB1 | 208974_x_at | -0.570 | AGCCGCCAACAGCAGCGCTGTTAGT | 34 |
| TMEM2 | 218113_at | -0.566 | TTCTAAACCCCCGACGCATAAAGTG | 35 |
| FKBP9 | 212169_at | -0.559 | TCAACAGAGTATTTCCCTTGGCCGA | 36 |
| PDIA4 | 208658_at | -0.548 | TCTGATTGCTTCCTGTTGAGCACAA | 37 |
| SMYD2 | 212922_s_at | -0.545 | AAGCCCTACAGTAAGCACTATCCTT | 38 |
| PTN | 211737_x_at | -0.540 | CAAGCCCAAACCTCAAGCAGAATCT | 39 |
| GPR171 | 207651_at | -0.538 | TTGCCTTGTAATTCGACAGCTCTAC | 40 |
| SLC20A1 | 201920_at | -0.522 | AGAGCATGCTCTGCGTTGTTGGTTT | 41 |
| TMEM97 | 212281_s_at | -0.522 | TCAGCTTTCTCTAAGTCTTTGCTCA | 42 |
| GUSB | 202605_at | -0.510 | CCCACTCAGTAGCCAAGTCACAATG | 43 |
| HYPK MIR1282 SERF2 SERF2-C15ORF63 | 218680_x_at | -0.509 | GAAGGTCCCGGGAGCAGAAAGCCAA | 44 |
| IL6 | 205207_at | -0.499 | TGTTAATGGGCATTCCTTCTTCTGG | 45 |
| CAV1 | 212097_at | -0.498 | ATGTGCTATACTGCATACTTTTTAA | 46 |
| KPNB1 | 213507_s_at | -0.493 | TGTAAAAGGCAGCCTGGCCTTTGCT | 47 |
| CAV1 | 203065_s_at | -0.486 | GCACATCTGGGCAGTTGTACCATGC | 48 |
| MFGE8 | 210605_s_at | -0.481 | GACACAGGCAGCTTCCAAAATATAT | 49 |
| TRAF3 | 221571_at | -0.470 | GTAGCCGCTTGCTGTGAAGACACAT | 50 |
| RPS6KA3 | 203843_at | -0.463 | AGCTGATAGCACAAGTTCTGGCGAC | 51 |
| CALU | 200755_s_at | -0.455 | GAACCTGCCATTACCTGGGCAAGGA | 52 |
| TRBC1 | 213193_x_at | -0.455 | TAGAGCAGACTGTGGCTTTACCTCG | 53 |

### Cancer Types

The methods, devices, and kits of the disclosure can be used to determine whether a subject, e.g., a subject with a cancer (e.g., multiple myeloma or non-Hodgkin's lymphoma, among others), will respond to treatment with daratumumab. In addition, methods are provided for treating a subject (e.g., a subject with a cancer, as described herein) who has been determined to be responsive to treatment with daratumumab according to the methods described herein, e.g., by administering an effective amount of daratumumab to the subject. Non-limiting examples of cancers contemplated for the methods described herein include multiple myeloma and non-Hodgkin's lymphoma (e.g., follicular lymphoma, diffuse large B cell lymphoma, mantle cell lymphoma, and Burkitt's lymphoma).

In particular, the methods are useful for determining whether a subject, e.g., a subject with a cancer, e.g., multiple myeloma and non-Hodgkin's lymphoma (e.g., follicular lymphoma, diffuse large B cell lymphoma, mantle cell lymphoma, and Burkitt's lymphoma), will likely respond to treatment with daratumumab. The methods can be used to exclude from treatment with daratumumab those subjects determined to be unlikely to respond to treatment with daratumumab based on the methods described herein.

In some instances, the subject has not received prior therapy for multiple myeloma. In some instances, the subject has relapsed after or is refractory to at least one prior therapy for multiple myeloma. In some instances, the at least one prior therapy includes an alkylating agent, a proteasome inhibitor, and/or an immunomodulatory agent. In some instances, the at least one prior therapy includes bortezomib, lenalidomide, pomalidomide, carfilzomib, autologous stem cell transplant therapy, or combinations thereof. In some instances, the subject has relapsed after or is refractory to one, two, or three prior lines of therapy.

### Methods for Detecting Biomarker Gene Expression in a Cancer Patient

A subject with cancer can be assessed for sensitivity or resistance to daratumumab by detecting gene expression of a biomarker (e.g., one or more of the biomarkers of Tables 1 and/or 2) in a biological sample obtained from the cancer patient (e.g., a subject with a cancer, such as, e.g., multiple myeloma or non-Hodgkin's lymphoma, or a recurrence thereof). The biological sample can include, for example, cells, tissue (e.g., a tissue sample obtained by biopsy; e.g., a bone marrow sample or a bone marrow biopsy sample), blood, serum, plasma, urine, sputum, cerebrospinal fluid, lymph tissue or fluid, or pancreatic fluid. For example, the biological sample can be fresh frozen or formalin-fixed paraffin embedded (FFPE) tissue obtained from the subject, such as a tumor sample (e.g., a biopsy) from the tissue of interest (e.g., bone marrow). In a preferred embodiment, the biological sample is a bone marrow sample. The bone marrow sample may include plasma cells.

### RNA Extraction and Biomarker Expression Measurement

Cell samples or tissue samples (e.g., a bone marrow sample or a reference bone marrow sample) may be snap frozen in liquid nitrogen until processing or fixed in formalin and embedded in paraffin. RNA may be extracted using, e.g., Trizol Reagent from Invitrogen following manufacturer's instructions, and detected directly or converted to cDNA for detection. RNA may be amplified using, e.g., MessageAmp kit from Ambion following manufacturer's instructions. Amplified RNA may be quantified using, e.g., HG-U133A or HG-U133_Plus2 GeneChip from Affymetrix Inc. and compatible apparatus e.g. GCS3000Dx from Affymetrix, using the manufacturer's instructions. The Affymetrix array typically contains 11 probes (also known as a probe set) specific to each gene. In general, confidence in a prediction of responsiveness or non-responsiveness increases with an increase in the number of probes used in the analysis. In Tables 1 and 2, a representative probe of the typical 11 probes is shown for each gene. The resulting biomarker expression measurements may be further analyzed as described herein. The procedures described can be implemented using, e.g., R software available from R-Project and supplemented with packages available from Bioconductor.

One or more of the biomarkers shown in Tables 1 and/or 2 (e.g., NR3C1 (SEQ ID NO: 1) or CCND1 (SEQ ID NO: 28)) may be measured in a biological sample (e.g., a tumor sample) obtained from the cancer subject (e.g., a subject with any of the cancer types described herein, such as a subject with a recurrence of cancer) using, e.g., polymerase chain reaction (PCR), reverse transcriptase PCR (RT-PCR), quantitative real-time PCR (qPCR), an array (e.g., a microarray), a genechip, pyrosequencing, nanopore sequencing, sequencing by synthesis, sequencing by expansion, single molecule real time technology, sequencing by ligation, microfluidics, infrared fluorescence, next generation sequencing (e.g., RNA-Seq techniques), Northern blots, Western blots, Southern blots, NanoString nCounter technologies (e.g., those described in U.S. Patent Application Nos. US 2011/0201515, US 2011/0229888, and US 2013/0017971, each of which is incorporated by reference in its entirety), proteomic techniques (e.g., mass spectrometry or protein arrays), and combinations thereof.

### Devices

A device of the disclosure can be used for detecting the level of expression of one or more biomarkers shown in Table(s) 1 and/or 2. The device may include at least one single-stranded nucleic acid (e.g., a probe) having at least 85% sequence identity (e.g., 85%, 90%, 95%, 97%, 98%, 99%, or 100% sequence identity) to a nucleic acid sequence that is complementary or identical to at least 5 (e.g., at least 10, at least 15, at least 20, or more) consecutive nucleotides of one or more biomarkers shown in Table(s) 1 and/or 2 (e.g., NR3C1 (SEQ ID NO: 1) or CCND1 (SEQ ID NO: 28)), in which the at least one single-stranded nucleic acid is sufficient for the detection of the level of expression of the one or more biomarkers. The device may be used to detect the level of expression of a given biomarker by specific hybridization between the single-stranded nucleic acid and the biomarker (e.g., an mRNA, genomic DNA, or non-coding RNA), a nucleic acid encoding the biomarker (e.g., an mRNA), or a complementary nucleic acid thereof. The device may be, or may include, a microarray. The device may also include or be used with reagents and materials for next generation sequencing (e.g., sequencing by synthesis). The device may also include or be used with NanoString reagents and at least one nCounter cartridge. The device may be, or include a protein array, which contains one or more protein binding moieties (e.g., proteins, antibodies, nucleic acids, aptamers, affibodies, lipids, phospholipids, small molecules, labeled variants of any of the above, and any other moieties useful for protein detection as well known in the art) capable of detectably binding to the polypeptide product(s) of one or more biomarkers shown in Table(s) 1 and/or 2. For example, the device may have single-stranded nucleic acid molecule(s) having the sequence of or complementary to each of the biomarkers of sensitivity selected from the biomarkers of Table 1 and/or for each of the biomarkers of resistance selected from the biomarkers of Table 2 that are affixed to the device and can be used to detect the level of expression of the biomarkers, e.g., by hybridization.

### Microarrays

The level of expression of the biomarkers (e.g., the biomarkers listed in Table(s) 1 and/or 2 (e.g., NR3C1 or CCND1) may be determined using high-throughput expression profiling platforms, such as microarrays. In particular, a microarray for use in the methods for assessing the responsiveness of a subject with cancer (e.g., a subject with recurrence of cancer) to daratumumab contains or is produced by generating oligonucleotide probes (e.g., DNA, cDNA, or RNA probes) capable of hybridizing to one or more biomarkers of interest (e.g., one or more of the biomarkers of Table(s) 1 and/or 2) or the complement sequences thereof. Each probe can have, e.g., at least 10, 15, 20, 25, 30, or more contiguous nucleic acid residues (e.g., at least 15) that are complementary or identical to a nucleic acid sequence of a selected biomarker. The probe nucleic acid sequence can also have at least 85% (e.g., 90%, 95%, 99%, or 100%) sequence identity to the nucleic acid sequence of the gene coding the biomarker (e.g., NR3C1 (e.g., SEQ ID NO: 1) or CCND1 (e.g., SEQ ID NO: 28)) or the complement sequence thereof. In particular, the probe sequences can be complementary to all or a portion of the nucleic acid sequence of the biomarker(s).

For example, microarrays of the disclosure for determining daratumumab responsiveness can include probes for one or more (e.g., at least 1, 5, 10, 15, or 20 (e.g., at least the top 1, 5, 10, 15, or 20), or more (e.g., all)) biomarkers of sensitivity shown in Table 1, such as NR3C1 (e.g., SEQ ID NO: 1).

Microarrays of the disclosure for determining daratumumab responsiveness can also include probes for one or more (e.g., at least 1, 5, 10, 15, or 20 (e.g., at least the top 1, 5, 10, 15, or 20), or more (e.g., all)) biomarkers of resistance listed in Table 2, such as CCND1 (e.g., SEQ ID NO: 28).

A microarray probe may be single-stranded or double-stranded. The probe may be labeled (e.g., detectably labeled with a fluorescent molecule, dye molecule, small molecule, epitope tag, barcode sequence, polypeptide, or any other detectable molecule). Probes can be detectably labeled and immobilized on a solid support to form the microarray. For example, probes can be either prefabricated and spotted to the surface or directly synthesized on to the surface (*in situ*) of the microarray. The microarray can also be configured such that the sequence and position of each member (e.g., probe) of the array is known. For example, a selection of biomarkers whose expression correlates with an increased likelihood of responsiveness to daratumumab can be arrayed on a solid support. Hybridization of a labeled probe with a particular target nucleic acid (e.g., an mRNA corresponding to one or more biomarkers of Table(s) 1 and/or 2) indicates that the sample from which the mRNA was derived expresses that biomarker (e.g., the biomarker of sensitivity or resistance to daratumumab).

### PCR-based techniques

As few as one and up to 25 or more of the biomarkers (e.g., 1 to 25 (e.g., the top 1 to 25) or 10 to 25 (e.g., the top 10 to 25), or at least the top 25 of the biomarkers listed in Table(s) 1 and/or 2) may be used to determine responsiveness of a cancer patient to daratumumab using the methods described herein. Tissue or cell samples from a cancer patient (e.g., a patient having recurrence of cancer, such as, e.g., multiple myeloma or non-Hodgkin's lymphoma) can be conveniently assayed for gene expression levels using nucleic acid amplification methods, such as polymerase chain reaction (PCR). Such PCR-based techniques may include reverse transcription PCR (RT-PCR), quantitative real-time PCR (qPCR), reverse transcription qPCR (RT-qPCR), or quantitative loop-mediated isothermal amplification (q-LAMP). For example, an mRNA corresponding to a biomarker of Table 1 or 2 can be detected in a biological sample by (a) producing cDNA from the sample by reverse transcription using at least one primer; (b) amplifying the cDNA so produced using a target polynucleotide as sense and antisense primers to amplify target cDNAs therein; and (c) detecting the presence of the amplified target cDNA using polynucleotide probes. The primers and probes including the target sequences shown in Table(s) 1 and/or 2, such as NR3C1 (SEQ ID NO: 1) or CCND1 (SEQ ID NO: 28), may be used to detect the level of expression of one or more of the indicated biomarkers using PCR. The methods can include one or more steps that allow determination of the levels of target mRNA in a biological sample (e.g., by simultaneously examining the levels of a comparative control mRNA sequence or "housekeeping" gene, such as an actin family member or GAPDH). The primers for these PCR-based techniques may be labeled for detection according to methods known in the art.

### Sequencing

The level of expression of the biomarkers shown in Table(s) 1 and/or 2, such as NR3C1 (SEQ ID NO: 1) or CCND1 (SEQ ID NO: 28), may be determined using sequencing technologies, such as next generation sequencing platforms (e.g., RNA-Seq), as described in Mortazavi et al., Nat. Methods 5: 621-628, 2008, hereby incorporated by reference. RNA-Seq is a robust technology for monitoring expression by direct sequencing of the RNA molecules in a sample. This methodology may include fragmentation of RNA to an average length of, e.g., 200 nucleotides, conversion to cDNA by random priming, and synthesis of double-stranded cDNA (e.g., using the Just cDNA DoubleStranded cDNA Synthesis Kit from Agilent Technology). The cDNA may then be converted into a molecular library for sequencing by addition of sequence adapters for each library (e.g., from Illumina^{®}/Solexa), and the resulting 50 to 100 nucleotide reads are mapped onto the genome. Exemplary sequencing platforms suitable for use according to the methods include, e.g., 454 pyrosequencing, Illumina sequencing by synthesis, SOLiD sequencing, Ion Torrent sequencing, and PacBio RS sequencing.

### Methods of Determining the Responsiveness of a Subject to Daratumumab

The disclosure features methods for the detection and screening of a subject with a cancer (e.g., a subject with cancer or a recurrence thereof) that may be responsive to daratumumab. The methods involve detecting the level of expression of one or more of the biomarkers shown in Table(s) 1 and/or 2 (e.g., NR3C1 (SEQ ID NO: 1) or CCND1 (SEQ ID NO: 28)). The methods may be used for predicting a subject's responsiveness to daratumumab, and may optionally include treating the subject with daratumumab (e.g., at one or more times throughout the progression of cancer and/or in cases of recurrence, such as, e.g., after a first line treatment, a second line treatment, and/or a third line treatment, in which one or more of the first, second, and third lines of treatment do not result in stable disease (SD), a partial response (PR), or a complete response (CR)).

The disclosure provides individual biomarkers (e.g., NR3C1 (SEQ ID NO: 1) or CCND1 (SEQ ID NO: 28)) and sets of biomarkers (e.g., two or more of the biomarkers listed in Table(s) 1 and/or 2), the levels of expression of which, as detected in a biological sample (e.g., a tumor sample, such as a biopsy) obtained from a cancer patient (e.g., a human with cancer, such as MM and NHL; e.g., a human with MM), are indicative of responsiveness to daratumumab.

The biomarkers were identified using methods similar to those previously described in, e.g., Chen et al. (Mol. Cancer Ther. 11:34-33, 2012), Wang et al. (J. Nat. Cancer Inst. 105: 1284-1291, 2013), and Knudsen et al. (PLoS One, 9: e87415, 2014), Buhl et al (PLoS One 13(3):e0194609, 2018), each of which are incorporated by reference herein in their entirety. In particular, an algorithm based on growth inhibition values (GI50) of a cell line (e.g., CCLE cells) is used. The cell line is subjected to treatment with daratumumab and baseline gene expression is determined (e.g., by microarray analysis, RT-PCR, qPCR, or next generation sequencing). After normalization, genes with, e.g., a Pearson correlation coefficient greater than 0.25 or below -0.25 can be classified as biomarkers of sensitivity or resistance, respectively. In particular, a correlation coefficient of 0.25 or greater is a statistically significant cut-off known in the art for establishing whether the levels of expression of, e.g., the genes shown in Table(s) 1 and/or 2, correlate with the likelihood of cancer treatment sensitivity, such as sensitivity to daratumumab, as described in Yael et al., Nature Medicine. 27(3):491-503, 2021, hereby incorporated by reference.

Alternatively, after normalization, genes, or means of genes, or differences between means of genes (e.g., the difference between the mean level of expression of one or more biomarkers of sensitivity and the mean level of expression of one or more biomarkers of resistance), that have a value above a cutoff value of the 50^{th} percentile or greater (e.g., 60^{th} percentile, 70^{th} percentile, 80^{th} percentile, or 90^{th} percentile, or greater) in a reference subject or reference population, such as a reference subject or reference population with the same diagnosis as the subject (e.g., the same type of cancer as the subject) indicates that the sample, such as, e.g., a tumor sample (or the subject from whom the sample was taken), is predicted to be responsive or non-responsive to the treatment, e.g., treatment with daratumumab. In particular, a subject determined to have a level of expression of the one or more biomarkers of sensitivity and/or resistance that is above a cutoff value (e.g., a 50^{th} percentile, a 60^{th} percentile, a 70^{th} percentile, an 80^{th} percentile, or a 90^{th} percentile or greater) of the level of the biomarkers of sensitivity and/or resistance (or a mean score of the biomarker level or a difference score of the biomarker level) in a reference subject or reference population determined to be sensitive to daratumumab indicates that the subject is also likely to be responsive to daratumumab. The higher the cutoff value, the greater the likelihood the subject will be sensitive to the treatment.

### Comparison of Biomarker Expression Levels

One or more biomarkers of sensitivity and/or resistance, as described herein, obtained from a biological sample of subject with a cancer (e.g., MM or NHL; e.g., MM) can be used to predict the responsiveness of the subject to treatment with daratumumab. The methods involve measuring the level of expression of the biomarker(s) in the biological sample. A single biomarker (e.g., any one or more of the biomarkers of Table 1 or 2, such as NR3C1 (SEQ ID NO: 1) and/or CCND1 (SEQ ID NO: 28)) or a set of biomarkers (e.g., one, two, three, four, five, ten, fifteen, twenty, twenty five, or all of the biomarkers of Tables 1 and/or 2 (e.g., the top one, the top two, the top three, the top four, the top five, the top ten, the top fifteen, the top twenty, the top twenty five, or all of the biomarkers of Tables 1 and/or 2)) may be used to determine the responsiveness of a cancer patient (e.g., a patient with cancer recurrence) to treatment with daratumumab. After determining the level of expression of the biomarker(s) in a sample (e.g., a tumor sample) from the cancer patient, the level of expression of the biomarker(s) in the sample may be compared to the level of expression of the biomarker(s) in a cell (e.g., a cancer cell) or tissue (e.g., a tumor tissue, such as the same type of tumor as that of the cancer patient) known to be sensitive to treatment with daratumumab. If the level of expression of the biomarker(s) in the sample from the cancer patient is substantially similar to the level of expression of the biomarker(s) in the cell or tissue (e.g., tumor tissue) known to be sensitive to daratumumab, then the cancer patient is predicted to be responsive to treatment with daratumumab. Alternatively, if the level of expression of the biomarker(s) in the sample from the cancer patient is substantially dissimilar to the level of expression of the biomarker(s) in the cell or tissue (e.g., tumor tissue) known to be sensitive to daratumumab, then the cancer patient is predicted to be non-responsive to treatment with daratumumab.

The level of expression of the biomarker(s) (e.g., NR3C1 (SEQ ID NO: 1) or CCND1 (SEQ ID NO: 28)) in a sample from the cancer patient may also be compared to the level of expression of the biomarker(s) in a cell (e.g., a cancer cell) or tissue (e.g., a tumor tissue, such as the same type of tumor as that of the cancer patient) known to be resistant to treatment with daratumumab. If the level of expression of the biomarker(s) in the sample from the cancer patient is substantially similar to the level of expression of the biomarker(s) in the cell or tissue (e.g., a tumor tissue) known to be resistant to daratumumab, then the cancer patient is predicted to be non-responsive to treatment with daratumumab. Alternatively, if the level of expression of the biomarker(s) in the sample from the cancer patient is substantially dissimilar to the level of expression of the biomarker(s) in the cell or tissue (e.g., a tumor tissue) known to be resistant to daratumumab, then the cancer patient is predicted to be responsive to treatment with daratumumab.

The responsiveness of a cancer patient (e.g., a patient with cancer recurrence) to daratumumab can also be predicted by comparing the level of expression of a biomarker (e.g., NR3C1 (SEQ ID NO: 1) or CCND1 (SEQ ID NO: 28)) to the level of expression of the biomarker in one or more cells or tissues (e.g., from a cancer patient population, e.g., with the same cancer as that of the subject patient) known to be sensitive to treatment with daratumumab, and one or more cells or tissues (e.g., from a cancer patient population) known to be resistant to treatment with daratumumab. In particular, the patient may be responsive to treatment with daratumumab if the level of expression of the biomarker(s) is substantially similar to the level of expression of the biomarker(s) in a cell or tissue (e.g., a tumor tissue) known to be sensitive to treatment with daratumumab (e.g., the same type of cell or tissue from the patient), and if the level of expression of the biomarker(s) is substantially dissimilar to the level of expression of the biomarker(s) in a cell or tissue (e.g., a tumor tissue) known to be resistant to treatment with daratumumab. Alternatively, the patient may be non-responsive to treatment with daratumumab if the level of expression of the biomarker(s) is substantially similar to the level of expression of the biomarker(s) in a cell or tissue (e.g., a tumor tissue) known to be resistant to treatment with daratumumab, and if the level of expression of the biomarker(s) is substantially dissimilar to the level of expression of the biomarker(s) in a cell or tissue (e.g., a tumor tissue) known to be sensitive to treatment with daratumumab.

Additionally, one or more biomarkers of sensitivity (e.g., one or more of NR3C1, PARP8, PSMB10, NR3C1, ZNF593, ZPR1, TXNIP, ARHGEF2, IL10RA, TMEM123, TRIB2, LINC00623/LINC00869, TAGLN2, MCCC2, LOC101928789/SP140, SP110, MBP, STAT6, HIVEP2, SP110, SEL1L3, SP110, EVI2B, NUP88, SEL1L3, and KIAA0247) and one or more biomarkers of resistance (e.g., one or more of CCND1, PSMC3IP, SF3B1, SF3B1, CCL20, UPP1, KPNB1, TMEM2, FKBP9, PDIA4, SMYD2, PTN, GPR171, SLC20A1, TMEM97, GUSB,

HYPK/MIR1282/SERF2/SERF2-C15ORF63, IL6, CAV1, KPNB1, CAV1, MFGE8, TRAF3, RPS6KA3, CALU, and TRBC1) may be used in combination to determine the responsiveness of a cancer patient (e.g., a patient with cancer recurrence) to treatment with daratumumab. For example, the predicted responsiveness of a cancer patient may be determined from, e.g., the difference score, which may be defined as the difference between the mean score (i.e., mean of the levels of expression) of the one or more biomarkers of sensitivity of Table 1 (e.g., NR3C1 (SEQ ID NO: 1)) and the mean score (i.e., mean of the levels of expression) of the one or more biomarkers of resistance of Table 2 (e.g., CCND1 (SEQ ID NO: 28)).

The difference score of the cancer patient can then be compared to the difference score in a cell (e.g., a cancer cell) or tissue (e.g., a tumor tissue) known to be sensitive or resistant to treatment with daratumumab. In particular, the patient may be responsive to treatment with daratumumab if the difference score is substantially similar to the difference score in a cell or tissue (e.g., a tumor tissue) known to be sensitive to treatment with daratumumab. Additionally, the patient may be responsive to treatment with daratumumab if the difference score is substantially dissimilar to the difference score in a cell or tissue (e.g., a tumor tissue) known to be resistant to treatment with daratumumab. Alternatively, the patient may be non-responsive to treatment with daratumumab if the difference score is substantially similar to the difference score in a cell or tissue (e.g., a tumor tissue) known to be resistant to treatment with daratumumab. Moreover, the patient may be non-responsive to treatment with daratumumab if the difference score is substantially dissimilar to the difference score in a cell or tissue (e.g., a tumor tissue) known to be sensitive to treatment with daratumumab.

Additionally, the patient may be responsive to treatment with daratumumab if the difference score is substantially similar to the difference score in a cell or tissue (e.g., a tumor tissue) known to be sensitive to treatment with daratumumab, relative to the difference score in a cell or tissue (e.g., a tumor tissue) known to be resistant to treatment with daratumumab. Also, the patient may be responsive to treatment with daratumumab if the difference score is substantially dissimilar to the difference score in a cell or tissue (e.g., a tumor tissue) known to be resistant to treatment with daratumumab, relative to the difference score in a cell or tissue (e.g., a tumor tissue) known to be sensitive to treatment with daratumumab. Alternatively, the patient may be non-responsive to treatment with daratumumab if the difference score is substantially similar to the difference score in a cell or tissue (e.g., a tumor tissue) known to be resistant to treatment with daratumumab, relative to the difference score in a cell or tissue (e.g., a tumor tissue) known to be sensitive to treatment with daratumumab. Furthermore, the patient may be non-responsive to treatment with daratumumab if the difference score is substantially dissimilar to the difference score in a cell or tissue (e.g., a tumor tissue) known to be sensitive to treatment with daratumumab, relative to the difference score in a cell or tissue (e.g., a tumor tissue) known to be resistant to treatment with daratumumab.

Additionally, the cancer patient (e.g., a patient with cancer recurrence) may be determined to be responsive to treatment with daratumumab if the difference score is compared to a difference score from a reference population (e.g., a score determined using a tumor sample(s) from subjects diagnosed with the same type(s) of tumor as the subject), in which a difference score at the 50^{th} percentile of the reference population, or the 60^{th} percentile, or the 70^{th} percentile, or the 80^{th} percentile, or the 90^{th} percentile, or greater, (e.g., of the difference scores determined for the individuals in the reference sample) indicates that the sample (or the subject from whom the sample was taken) is predicted to be responsive to treatment with daratumumab. The confidence of the prediction increases as the percentile level increases (e.g., a difference score above the 90^{th} percentile of a reference population indicates a greater likelihood of treatment responsiveness than a difference score at the 50^{th} percentile). Conversely, a difference score in the tested sample of below the 50^{th} percentile of the difference score determined for individuals of the reference population (e.g., 40^{th}, percentile, 30^{th} percentile, 20^{th} percentile, or 10^{th} percentile, or below) indicates that the sample (or the subject from whom the sample was taken) is predicted to be non-responsive to treatment with daratumumab.

Additionally, the mean score (e.g., the mean of the levels of expression) of the one or more biomarkers of sensitivity of Table 1 and/or the mean score (e.g., the mean of the levels of expression) of the one or more biomarkers of resistance of Table 2 can be used to predict responsiveness of a cancer patient (e.g., a patient with cancer recurrence) to daratumumab. After determining the mean score of the biomarker(s) in a sample (e.g., a tumor sample) from the cancer patient, the mean score of the biomarker(s) in the sample may be compared to the mean score of the biomarker(s) in a cell (e.g., a cancer cell) or tissue (e.g., a tumor tissue) known to be sensitive to treatment with daratumumab. If the mean score of the biomarker(s) in the sample from the cancer patient is substantially similar to the mean score of the biomarker(s) in the cell or tissue (e.g., a tumor tissue) known to be sensitive to daratumumab, then the cancer patient is predicted to be responsive to treatment with daratumumab. For example, a patient may be deemed sensitive to treatment with daratumumab if the mean score of the biomarker(s) of sensitivity in a cell or tissue (e.g., a tumor tissue) is at or above a cutoff value of the 50^{th} percentile in a cell or tissue (e.g., the same type of cell or tissue as tested in the patient) of the mean score determined for a reference population of individuals having the same cancer as the subject, or greater (e.g., 50^{th} percentile, 60^{th} percentile, 70^{th} percentile, 80t^{h} percentile, or 90^{th} percentile, or greater).

Alternatively, if the mean score of the biomarker(s) in the sample from the cancer patient is substantially dissimilar to the mean score of the biomarker(s) in the cell or tissue (e.g., a tumor tissue) known to be sensitive to daratumumab, then the cancer patient is predicted to be non-responsive to treatment with daratumumab. As an example, a patient may be deemed resistant to treatment with daratumumab if the mean score of the biomarker(s) of sensitivity in a cell or tissue (e.g., a cancer cell or tumor tissue) is below a cutoff value of the 50^{th} percentile or less (e.g., 40^{th} percentile, 30^{th} percentile, 20^{th} percentile, or 10^{th} percentile, or less) in a cell or tissue (e.g., the same type of cell or tissue as tested in the patient) of the mean score determined for a reference population of individuals having the same cancer as the subject.

The mean score (e.g., the mean of the levels of expression) of the one or more biomarkers of sensitivity of Table 1 and/or the mean score (e.g., the mean of the levels of expression) of the one or more biomarkers of resistance of Table 2 in a sample from the cancer patient may also be compared to the mean score of the biomarker(s) in a cell (e.g., a cancer cell) or tissue (e.g., a tumor tissue) known to be resistant to treatment with daratumumab. If the mean score of the biomarker(s) in the sample from the cancer patient is substantially similar to the mean score of the biomarker(s) in the cell or tissue (e.g., a tumor tissue) known to be resistant to daratumumab, then the cancer patient is predicted to be non-responsive to treatment with daratumumab. Alternatively, if the mean score of the biomarker(s) in the sample from the cancer patient is substantially dissimilar to the mean score of the biomarker(s) in the cell or tissue (e.g., a tumor tissue) known to be resistant to daratumumab, then the cancer patient is predicted to be responsive to treatment with daratumumab.

The responsiveness of a cancer patient (e.g., a patient with cancer recurrence) to daratumumab can also be predicted by comparing the mean score (e.g., the mean of the levels of expression) of the one or more biomarkers of sensitivity of Table 1 and/or the mean score (e.g., the mean of the levels of expression) of the one or more biomarkers of resistance of Table 2 in a sample from the cancer patient to the mean score of the biomarker(s) in one or more cells or tissues (e.g., from a cancer patient population) known to be sensitive to treatment with daratumumab and one or more cells or tissues (e.g., from a cancer patient population) known to be resistant to treatment with daratumumab. In particular, the patient may be responsive to treatment with daratumumab if the mean score of the biomarker(s) is substantially similar to the mean score of the biomarker(s) in a cell or tissue (e.g., a tumor tissue) known to be sensitive to treatment with daratumumab, relative to the mean score in a cell or tissue (e.g., a tumor tissue) known to be resistant to treatment with daratumumab. Additionally, the patient may be responsive to treatment with daratumumab if the mean score of the biomarker(s) is substantially dissimilar to the mean score of the biomarker(s) in a cell or tissue (e.g., a tumor tissue) known to be resistant to treatment with daratumumab, relative to the mean score in a cell or tissue (e.g., a tumor tissue) known to be sensitive to treatment with daratumumab.

Additionally, a patient may be deemed sensitive to treatment with daratumumab if the mean score of the biomarker(s) of resistance in the sample is below a cutoff value of the 50^{th} percentile of the mean score determined for the mean score determined for a reference population having the same cancer as the patient, or less (e.g., 40^{th} percentile, 30^{th} percentile, 20^{th} percentile, or 10^{th} percentile, or less). Alternatively, the patient may be non-responsive to treatment with daratumumab if the mean score of the biomarker(s) is substantially similar to the mean score of the biomarker(s) in a cell or tissue (e.g., a tumor tissue) known to be resistant to treatment with daratumumab, relative to the mean score in a cell or tissue (e.g., a tumor tissue) known to be sensitive to treatment with daratumumab. Furthermore, the patient may be non-responsive to treatment with daratumumab if the mean score of the biomarker(s) is substantially dissimilar to the mean score of the biomarker(s) in a cell or tissue (e.g., a tumor tissue) known to be sensitive to treatment with daratumumab, relative to the mean score in a cell or tissue (e.g., a tumor tissue) known to be resistant to treatment with daratumumab.

In addition, the cancer patient (e.g., a patient with cancer recurrence) may be determined to be responsive to treatment with daratumumab if the mean score (i.e., mean of the levels of expression) of the one or more biomarkers of sensitivity of Table 1 is compared to a mean score from a reference population (e.g., a mean score determined using a tumor sample(s) from subjects diagnosed with the same type(s) of tumor as the subject), in which a mean score at the 50^{th} percentile of the mean score determined for the individuals of the reference population, or the 60^{th} percentile, or the 70^{th} percentile, or the 80^{th} percentile, or the 90^{th} percentile, or greater, indicates that the sample (or the subject from whom the sample was taken) is predicted to be responsive to treatment with daratumumab. The confidence of the prediction increases as the percentile level increases (e.g., a mean score above the 90^{th} percentile of a reference population indicates a greater likelihood of treatment responsiveness than a level of expression at the 50^{th} percentile). Conversely, a mean score in the tested sample of below the 50^{th} percentile of the reference population indicates that the sample (or the subject from whom the sample was taken) is predicted to be non-responsive to treatment with daratumumab.

Additionally, the cancer patient (e.g., a patient with cancer recurrence) may be determined to be non-responsive to treatment with daratumumab if the mean score (i.e., mean of the levels of expression) of the one or more biomarkers of resistance of Table 2 is compared to a mean score from a reference population (e.g., a mean score determined using a tumor sample(s) from subjects diagnosed with the same type(s) of tumor as the subject), in which a level of expression at the 50^{th} percentile of the mean score determined for the individuals of the reference population, or the 60^{th} percentile, or the 70^{th} percentile, or the 80^{th} percentile, or the 90^{th} percentile, or greater, indicates that the sample (or the subject from whom the sample was taken) is predicted to be non-responsive to treatment with daratumumab. The confidence of the prediction increases as the percentile level increases (e.g., a mean score above the 90^{th} percentile of the mean score determined for a reference population indicates a greater likelihood of treatment non-responsiveness than a mean score at the 50^{th} percentile).

One or more biomarkers of sensitivity and/or resistance, identified as described herein, can be used to predict responsiveness to daratumumab by measuring the level of expression of the biomarker(s) in a biological sample obtained from the cancer patient. A single biomarker (e.g., any of the biomarkers of Tables 1 and/or 2, such as (e.g., NR3C1 (SEQ ID NO: 1) or CCND1 (SEQ ID NO: 28)) or a set of biomarkers (e.g., one, two, three, four, five, ten, fifteen, twenty, twenty five, or all of the biomarkers of Tables 1 and/or 2 (e.g., the top one, the top two, the top three, the top four, the top five, the top ten, the top fifteen, the top twenty, the top twenty five, or all of the biomarkers of Tables 1 and/or 2)) may be used to determine the responsiveness of a cancer patient (e.g., a patient with cancer recurrence) to daratumumab. After determining the level of expression of the biomarker(s) in a sample (e.g., a tumor sample) from the cancer patient, the level of expression of the biomarker(s) in the sample may be compared to the level of expression of the biomarker(s) in a cell (e.g., a cancer cell) or tissue (e.g., a tumor tissue) known to be sensitive to treatment with daratumumab. If the level of expression of the biomarker(s) in the sample from the cancer patient corresponds to the level of expression of the biomarker(s) in the cell or tissue (e.g., a tumor tissue) known to be sensitive to daratumumab, then the cancer patient is predicted to be responsive to treatment with daratumumab. Alternatively, if the level of expression of the biomarker(s) in the sample from the cancer patient is substantially dissimilar to the level of expression of the biomarker(s) in the cell or tissue (e.g., a tumor tissue) known to be sensitive to daratumumab, then the cancer patient is predicted to be non-responsive to treatment with daratumumab.

The level of expression of the biomarker(s) (e.g., NR3C1 (SEQ ID NO: 1) or CCND1 (SEQ ID NO: 28)) in a sample from the cancer patient may also be compared to the level of expression of the biomarker(s) in a cell (e.g., a cancer cell) or tissue (e.g., a tumor tissue) known to be resistant to treatment with daratumumab. If the level of expression of the biomarker(s) in the sample from the cancer patient corresponds to the level of expression of the biomarker(s) in the cell or tissue (e.g., a tumor tissue) known to be resistant to daratumumab, then the cancer patient is predicted to be non-responsive to treatment with daratumumab. Alternatively, if the level of expression of the biomarker(s) in the sample from the cancer patient is substantially dissimilar to the level of expression of the biomarker(s) in the cell or tissue (e.g., a tumor tissue) known to be resistant to daratumumab, then the cancer patient is predicted to be responsive to treatment with daratumumab.

The responsiveness of a cancer patient (e.g., a patient with cancer recurrence) to daratumumab can also be predicted by comparing the level of expression of the biomarker(s) (e.g., NR3C1 (SEQ ID NO: 1) or CCND1 (SEQ ID NO: 28)) to the level of expression of the biomarker(s) in one or more cells or tissues (e.g., from a cancer patient population, such as a cancer patient population having the same cancer as the cancer patient) known to be sensitive to treatment with daratumumab, and one or more cells or tissues (e.g., from a cancer patient population, such as a cancer patient population having the same cancer as the cancer patient) known to be resistant to treatment with daratumumab. In particular, the patient may be responsive to treatment with daratumumab if the level of expression of the biomarker(s) corresponds to the level of expression of the biomarker(s) in a cell or tissue (e.g., a tumor tissue) known to be sensitive to treatment with daratumumab, relative to the level of expression of the biomarker(s) in a cell or tissue (e.g., a tumor tissue) known to be resistant to treatment with daratumumab. Moreover, the patient may be responsive to treatment with daratumumab if the level of expression of the biomarker(s) is substantially dissimilar to the level of expression of the biomarker(s) in a cell or tissue (e.g., a tumor tissue) known to be resistant to treatment with daratumumab, relative to the level of expression of the biomarker(s) in a cell or tissue (e.g., a tumor tissue) known to be sensitive to treatment with daratumumab. Alternatively, the patient may be non-responsive to treatment with daratumumab if the level of expression of the biomarker(s) corresponds to the level of expression of the biomarker(s) in a cell or tissue (e.g., a tumor tissue) known to be resistant to treatment with daratumumab, relative to the level of expression of the biomarker(s) in a cell or tissue (e.g., a tumor tissue) known to be sensitive to treatment with daratumumab. Also, the patient may be non-responsive to treatment with daratumumab if the level of expression of the biomarker(s) is substantially dissimilar to the level of expression of the biomarker(s) in a cell or tissue (e.g., a tumor tissue) known to be sensitive to treatment with daratumumab, relative to the level of expression of the biomarker(s) in a cell or tissue (e.g., a tumor tissue) known to be resistant to treatment with daratumumab.

Additionally, one or more biomarkers of sensitivity (e.g., one or more of NR3C1, PARP8, PSMB10, NR3C1, ZNF593, ZPR1, TXNIP, ARHGEF2, IL10RA, TMEM123, TRIB2, LINC00623/LINC00869, TAGLN2, MCCC2, LOC101928789/SP140, SP110, MBP, STAT6, HIVEP2, SP110, SEL1L3, SP110, EVI2B, NUP88, SEL1L3, and KIAA0247) and one or more biomarkers of resistance (e.g., one or more of CCND1, PSMC3IP, SF3B1, SF3B1, CCL20, UPP1, KPNB1, TMEM2, FKBP9, PDIA4, SMYD2, PTN, GPR171, SLC20A1, TMEM97, GUSB, HYPK/MIR1282/SERF2/SERF2-C15ORF63, IL6, CAV1, KPNB1, CAV1, MFGE8, TRAF3, RPS6KA3, CALU, and TRBC1) may be used in combination to determine the responsiveness of a cancer patient (e.g., a patient with cancer recurrence) to treatment with daratumumab. For example, the predicted responsiveness of a cancer patient may be determined from, e.g., the difference score, which may be defined as the difference between the mean score (e.g., the mean of the levels of expression) of the one or more biomarkers of sensitivity of Table 1 (e.g., NR3C1 (SEQ ID NO: 1)) and the mean score (e.g., the mean of the levels of expression) of the one or more biomarkers of resistance of Table 2 (e.g., CCND1 (SEQ ID NO: 28)).

The difference score of the cancer patient can then be compared to the difference score in a cell (e.g., a cancer cell) or tissue (e.g., a tumor tissue) known to be sensitive or resistant to treatment with daratumumab. In particular, the patient may be responsive to treatment with daratumumab if the difference score corresponds to the difference score in a cell or tissue (e.g., a tumor tissue) known to be sensitive to treatment with daratumumab. Additionally, the patient may be responsive to treatment with daratumumab if the difference score is substantially dissimilar to the difference score in a cell or tissue (e.g., a tumor tissue) known to be resistant to treatment with daratumumab. Alternatively, the patient may be non-responsive to treatment with daratumumab if the difference score corresponds to the difference score in a cell or tissue (e.g., a tumor tissue) known to be resistant to treatment with daratumumab. Also, the patient may be non-responsive to treatment with daratumumab if the difference score is substantially dissimilar to the difference score in a cell or tissue (e.g., a tumor tissue) known to be sensitive to treatment with daratumumab.

Additionally, the patient may be responsive to treatment with daratumumab if the difference score corresponds to the difference score in a cell or tissue (e.g., a tumor tissue) known to be sensitive to treatment with daratumumab, relative to the difference score in a cell or tissue (e.g., a tumor tissue) known to be resistant to treatment with daratumumab. Furthermore, the patient may be responsive to treatment with daratumumab if the difference score is substantially dissimilar to the difference score in a cell or tissue (e.g., a tumor tissue) known to be resistant to treatment with daratumumab, relative to the difference score in a cell or tissue (e.g., a tumor tissue) known to be sensitive to treatment with daratumumab. Alternatively, the patient may be non-responsive to treatment with daratumumab if the difference score corresponds to the difference score in a cell or tissue (e.g., a tumor tissue) known to be resistant to treatment with daratumumab, relative to the difference score in a cell or tissue (e.g., a tumor tissue) known to be sensitive to treatment with daratumumab. Additionally, the patient may be non-responsive to treatment with daratumumab if the difference score is substantially dissimilar to the difference score in a cell or tissue (e.g., a tumor tissue) known to be sensitive to treatment with daratumumab, relative to the difference score in a cell or tissue (e.g., a tumor tissue) known to be resistant to treatment with daratumumab.

Alternatively, the patient may be determined to be responsive to treatment with daratumumab if the difference score is above a cutoff value of the 50^{th} percentile in a reference population with the same diagnosis as the patient, or greater (e.g., the difference score is above a cutoff value of the 60^{th} percentile, 70^{th} percentile, 80^{th} percentile, or 90^{th} percentile, or greater).

In any of the above instances, the reference population may include individuals with the same cancer as the subject. The percentiles in a reference population may be the percentiles of a set of reference levels of expression, set of mean scores, or set of difference scores determined for the individuals of the reference population. The set of reference mean scores or the set of reference difference scores is: (a) a set including the mean scores for the one or more biomarkers of sensitivity calculated for each individual of the reference population; (b) a set including the mean scores for the one or more biomarkers of resistance calculated for each individual of the reference population; or (c) a set including the difference scores calculated for each individual of the reference population. The reference mean score or the reference difference score may be determined in a reference bone marrow sample or a reference blood sample obtained from the individuals of the reference population. The type of reference bone marrow sample or reference blood sample may be the same type of sample as the sample obtained from the subject. In some instances, the reference bone marrow sample or the reference blood sample includes plasma cells.

Preferably, the cell or tissue (e.g., a tumor tissue; e.g., a bone marrow sample) known to be either sensitive or resistant to daratumumab or from the individuals of the reference population is of the same cancer type as the cancer patient with an unknown responsiveness to daratumumab. For example, the cancer patient and the cell or tissue (e.g., a tissue sample or a tumor sample; e.g., a biopsy sample; e.g., a bone marrow sample or a bone marrow biopsy sample) known to be either sensitive or resistant to daratumumab or the individuals of the reference population may have the same cancer type, selected from multiple myeloma or non-Hodgkin's lymphoma (e.g., follicular lymphoma, diffuse large B cell lymphoma, mantle cell lymphoma, or Burkitt's lymphoma). The methods, devices, and kits described herein can be used to determine responsiveness of multiple types of cancer disclosed herein to daratumumab.

Machine learning techniques such as Neural Networks, Support Vector Machines, K Nearest Neighbor, and Nearest Centroids may also be employed to develop models that discriminate patients sensitive to treatment with daratumumab from those resistant to treatment with daratumumab using biomarker expression as model variables, which assign each patient a classification as sensitive or resistant to treatment with daratumumab. Machine learning techniques used to classify patients using various measurements are described in U.S. Patent No. 5,822,715; U.S. Patent Application Publication Nos. 2003/0073083, 2005/0227266, 2005/0208512, 2005/0123945, 2003/0129629, and 2002/0006613; and in Vapnik V N. Statistical Learning Theory, John Wiley & Sons, New York, 1998; Hastie et al., 2001, The Elements of Statistical Learning: Data Mining, Inference, and Prediction, Springer, N.Y.; Agresti, 1996, An Introduction to Categorical Data Analysis, John Wiley & Sons, New York; V. Tresp et al., "Neural Network Modeling of Physiological Processes," in Hanson S. J. et al. (Eds.), Computational Learning Theory and Natural Learning Systems 2, MIT Press, 1994, each of which are hereby incorporated by reference in their entirety.

### Biomarkers of Sensitivity and Resistance

The levels of expression of one or more biomarkers of Table(s) 1 and/or 2 can be used to determine responsiveness of a cancer patient to treatment with daratumumab. In certain embodiments, the biomarker(s) of sensitivity can be selected from (a) one or more of SEQ ID NOs: 1-27 from Table 1. Moreover, in certain embodiments, the biomarker(s) of resistance can be selected from (a) one or more of SEQ ID NOs: 28-53 of Table 2. In particular embodiments, at least one (e.g., at least 1, at least 5, at least 10, at least 15, at least 20, at least 25, or at least 27 (e.g., at least the top 1, at least the top 5, at least the top 10, at least the top 15, at least the top 20, at least the top 25, or all 27)) of the biomarkers of sensitivity of Table 1 and/or at least one (e.g., at least 1, at least 5, at least 10, at least 15, at least 20, at least 25, or at least 27 (e.g., at least the top 1, at least the top 5, at least the top 10, at least the top 15, at least the top 20, at least the top 25, or all 26)) of the biomarkers of resistance of Table 2 can be used to determine responsiveness of a cancer patient to treatment with daratumumab. In more specific embodiments, one biomarker of sensitivity from Table 1 (e.g., NR3C1 (SEQ ID NO: 1)), and/or one biomarker of resistance from Table 2 (e.g., CCND1 (SEQ ID NO: 28)) can be used to determine responsiveness of a cancer patient to treatment with daratumumab. Once determined to be sensitive, the patient can be treated with daratumumab.

In particular, the biomarker of SEQ ID NO: 1 may be used to assess the responsiveness of a cancer patient (e.g., a patient with a cancer or a recurrence thereof) to daratumumab. The level of expression of the biomarker of SEQ ID NO: 1 may be assessed using nucleic acid amplification methods (e.g., PCR) or a device (e.g., a microarray). As is described above, the level of expression of the biomarker of SEQ ID NO: 1 in the patient sample may then be compared, e.g., to the level of expression of the biomarker of SEQ ID NO: 1 in a cell (e.g., a cancer cell) or tissue (e.g., a tumor tissue) known to be sensitive or resistant to treatment with daratumumab, or above a percentile cutoff (e.g., the 50^{th} percentile, 60th percentile, 70^{th} percentile, 80^{th} percentile, 90^{th} percentile, or greater) in a population of individuals with the cancer, and used to determine the cancer patient's responsiveness to daratumumab. The biomarker of SEQ ID NO: 1 may be used alone or in combination with one or more additional biomarker(s) (e.g., one, two, three, four, five, ten, fifteen, twenty, twenty five, or all of the biomarkers shown in Tables 1 and/or 2 (e.g., the top one biomarker shown in Tables 1 and/or 2, the top two biomarker shown in Tables 1 and/or 2, the top three biomarkers shown in Tables 1 and/or 2, the top four biomarkers shown in Tables 1 and/or 2, the top five biomarkers shown in Tables 1 and/or 2, the top ten biomarkers shown in Tables 1 and/or 2, the top fifteen biomarkers shown in Tables 1 and/or 2, the top twenty biomarkers shown in Tables 1 and/or 2, the top twenty five biomarkers shown in Tables 1 and/or 2, or all of the biomarkers shown in Tables 1 and/or 2)), such as biomarker(s) of SEQ ID NOs: 2-53 to predict responsiveness of the cancer patient to treatment with daratumumab. The level of expression of the biomarker(s) may be determined using, e.g., a microarray, PCR, or other techniques described herein, for example, using a nucleic acid probe sequence based on the target sequences shown in Tables 1 and 2.

The biomarker of SEQ ID NO: 2 may be used to assess the responsiveness of a cancer patient (e.g., a patient with a cancer or a recurrence thereof) to daratumumab. The level of expression of the biomarker of SEQ ID NO: 2 may be assessed using nucleic acid amplification methods (e.g., PCR) or a device (e.g., a microarray). As is described above, the level of expression of the biomarker of SEQ ID NO: 2 in the patient sample may then be compared, e.g., to the level of expression of the biomarker of SEQ ID NO: 2 in a cell (e.g., a cancer cell) or tissue (e.g., a tumor tissue) known to be sensitive or resistant to treatment with daratumumab, or above a percentile cutoff (e.g., the 50^{th} percentile, 60th percentile, 70^{th} percentile, 80^{th} percentile, 90^{th} percentile, or greater) in a population of individuals with the cancer, and used to determine the cancer patient's responsiveness to daratumumab. The biomarker of SEQ ID NO: 2 may be used alone or in combination with one or more additional biomarker(s) (e.g., one, two, three, four, five, ten, fifteen, twenty, twenty five, or all of the biomarkers shown in Tables 1 and/or 2 (e.g., the top one biomarker shown in Tables 1 and/or 2, the top two biomarker shown in Tables 1 and/or 2, the top three biomarkers shown in Tables 1 and/or 2, the top four biomarkers shown in Tables 1 and/or 2, the top five biomarkers shown in Tables 1 and/or 2, the top ten biomarkers shown in Tables 1 and/or 2, the top fifteen biomarkers shown in Tables 1 and/or 2, the top twenty biomarkers shown in Tables 1 and/or 2, the top twenty five biomarkers shown in Tables 1 and/or 2, or all of the biomarkers shown in Tables 1 and/or 2)), such as biomarker(s) of SEQ ID NOs: 1, 3-53 to predict responsiveness of the cancer patient to treatment with daratumumab. The level of expression of the biomarker(s) may be determined using, e.g., a microarray, PCR, or other techniques described herein, for example, using a nucleic acid probe sequence based on the target sequences shown in Tables 1 and 2.

The biomarker of SEQ ID NO: 3 may be used to assess the responsiveness of a cancer patient (e.g., a patient with a cancer or a recurrence thereof) to daratumumab. The level of expression of the biomarker of SEQ ID NO: 3 may be assessed using nucleic acid amplification methods (e.g., PCR) or a device (e.g., a microarray). As is described above, the level of expression of the biomarker of SEQ ID NO: 3 in the patient sample may then be compared, e.g., to the level of expression of the biomarker of SEQ ID NO: 3 in a cell (e.g., a cancer cell) or tissue (e.g., a tumor tissue) known to be sensitive or resistant to treatment with daratumumab, or above a percentile cutoff (e.g., the 50^{th} percentile, 60th percentile, 70^{th} percentile, 80^{th} percentile, 90^{th} percentile, or greater) in a population of individuals with the cancer, and used to determine the cancer patient's responsiveness to daratumumab. The biomarker of SEQ ID NO: 3 may be used alone or in combination with one or more additional biomarker(s) (e.g., one, two, three, four, five, ten, fifteen, twenty, twenty five, or all of the biomarkers shown in Tables 1 and/or 2 (e.g., the top one biomarker shown in Tables 1 and/or 2, the top two biomarker shown in Tables 1 and/or 2, the top three biomarkers shown in Tables 1 and/or 2, the top four biomarkers shown in Tables 1 and/or 2, the top five biomarkers shown in Tables 1 and/or 2, the top ten biomarkers shown in Tables 1 and/or 2, the top fifteen biomarkers shown in Tables 1 and/or 2, the top twenty biomarkers shown in Tables 1 and/or 2, the top twenty five biomarkers shown in Tables 1 and/or 2, or all of the biomarkers shown in Tables 1 and/or 2)), such as biomarker(s) of SEQ ID NOs: 1, 2, 4-53 to predict responsiveness of the cancer patient to treatment with daratumumab. The level of expression of the biomarker(s) may be determined using, e.g., a microarray, PCR, or other techniques described herein, for example, using a nucleic acid probe sequence based on the target sequences shown in Tables 1 and 2.

The biomarker of SEQ ID NO: 4 may be used to assess the responsiveness of a cancer patient (e.g., a patient with a cancer or a recurrence thereof) to daratumumab. The level of expression of the biomarker of SEQ ID NO: 4 may be assessed using nucleic acid amplification methods (e.g., PCR) or a device (e.g., a microarray). As is described above, the level of expression of the biomarker of SEQ ID NO: 4 in the patient sample may then be compared, e.g., to the level of expression of the biomarker of SEQ ID NO: 4 in a cell (e.g., a cancer cell) or tissue (e.g., a tumor tissue) known to be sensitive or resistant to treatment with daratumumab, or above a percentile cutoff (e.g., the 50^{th} percentile, 60th percentile, 70^{th} percentile, 80^{th} percentile, 90^{th} percentile, or greater) in a population of individuals with the cancer, and used to determine the cancer patient's responsiveness to daratumumab. The biomarker of SEQ ID NO: 4 may be used alone or in combination with one or more additional biomarker(s) (e.g., one, two, three, four, five, ten, fifteen, twenty, twenty five, or all of the biomarkers shown in Tables 1 and/or 2 (e.g., the top one biomarker shown in Tables 1 and/or 2, the top two biomarker shown in Tables 1 and/or 2, the top three biomarkers shown in Tables 1 and/or 2, the top four biomarkers shown in Tables 1 and/or 2, the top five biomarkers shown in Tables 1 and/or 2, the top ten biomarkers shown in Tables 1 and/or 2, the top fifteen biomarkers shown in Tables 1 and/or 2, the top twenty biomarkers shown in Tables 1 and/or 2, the top twenty five biomarkers shown in Tables 1 and/or 2, or all of the biomarkers shown in Tables 1 and/or 2)), such as biomarker(s) of SEQ ID NOs: 1-3, 5-53 to predict responsiveness of the cancer patient to treatment with daratumumab. The level of expression of the biomarker(s) may be determined using, e.g., a microarray, PCR, or other techniques described herein, for example, using a nucleic acid probe sequence based on the target sequences shown in Tables 1 and 2.

The biomarker of SEQ ID NO: 5 may be used to assess the responsiveness of a cancer patient (e.g., a patient with a cancer or a recurrence thereof) to daratumumab. The level of expression of the biomarker of SEQ ID NO: 5 may be assessed using nucleic acid amplification methods (e.g., PCR) or a device (e.g., a microarray). As is described above, the level of expression of the biomarker of SEQ ID NO: 5 in the patient sample may then be compared, e.g., to the level of expression of the biomarker of SEQ ID NO: 5 in a cell (e.g., a cancer cell) or tissue (e.g., a tumor tissue) known to be sensitive or resistant to treatment with daratumumab, or above a percentile cutoff (e.g., the 50^{th} percentile, 60th percentile, 70^{th} percentile, 80^{th} percentile, 90^{th} percentile, or greater) in a population of individuals with the cancer, and used to determine the cancer patient's responsiveness to daratumumab. The biomarker of SEQ ID NO: 5 may be used alone or in combination with one or more additional biomarker(s) (e.g., one, two, three, four, five, ten, fifteen, twenty, twenty five, or all of the biomarkers shown in Tables 1 and/or 2 (e.g., the top one biomarker shown in Tables 1 and/or 2, the top two biomarker shown in Tables 1 and/or 2, the top three biomarkers shown in Tables 1 and/or 2, the top four biomarkers shown in Tables 1 and/or 2, the top five biomarkers shown in Tables 1 and/or 2, the top ten biomarkers shown in Tables 1 and/or 2, the top fifteen biomarkers shown in Tables 1 and/or 2, the top twenty biomarkers shown in Tables 1 and/or 2, the top twenty five biomarkers shown in Tables 1 and/or 2, or all of the biomarkers shown in Tables 1 and/or 2)), such as biomarker(s) of SEQ ID NOs: 1-4, 6-53 to predict responsiveness of the cancer patient to treatment with daratumumab. The level of expression of the biomarker(s) may be determined using, e.g., a microarray, PCR, or other techniques described herein, for example, using a nucleic acid probe sequence based on the target sequences shown in Tables 1 and 2.

The biomarker of SEQ ID NO: 6 may be used to assess the responsiveness of a cancer patient (e.g., a patient with a cancer or a recurrence thereof) to daratumumab. The level of expression of the biomarker of SEQ ID NO: 6 may be assessed using nucleic acid amplification methods (e.g., PCR) or a device (e.g., a microarray). As is described above, the level of expression of the biomarker of SEQ ID NO: 6 in the patient sample may then be compared, e.g., to the level of expression of the biomarker of SEQ ID NO: 6 in a cell (e.g., a cancer cell) or tissue (e.g., a tumor tissue) known to be sensitive or resistant to treatment with daratumumab, or above a percentile cutoff (e.g., the 50^{th} percentile, 60th percentile, 70^{th} percentile, 80^{th} percentile, 90^{th} percentile, or greater) in a population of individuals with the cancer, and used to determine the cancer patient's responsiveness to daratumumab. The biomarker of SEQ ID NO: 6 may be used alone or in combination with one or more additional biomarker(s) (e.g., one, two, three, four, five, ten, fifteen, twenty, twenty five, or all of the biomarkers shown in Tables 1 and/or 2 (e.g., the top one biomarker shown in Tables 1 and/or 2, the top two biomarker shown in Tables 1 and/or 2, the top three biomarkers shown in Tables 1 and/or 2, the top four biomarkers shown in Tables 1 and/or 2, the top five biomarkers shown in Tables 1 and/or 2, the top ten biomarkers shown in Tables 1 and/or 2, the top fifteen biomarkers shown in Tables 1 and/or 2, the top twenty biomarkers shown in Tables 1 and/or 2, the top twenty five biomarkers shown in Tables 1 and/or 2, or all of the biomarkers shown in Tables 1 and/or 2)), such as biomarker(s) of SEQ ID NOs: 1-5, 7-53 to predict responsiveness of the cancer patient to treatment with daratumumab. The level of expression of the biomarker(s) may be determined using, e.g., a microarray, PCR, or other techniques described herein, for example, using a nucleic acid probe sequence based on the target sequences shown in Tables 1 and 2.

The biomarker of SEQ ID NO: 7 may be used to assess the responsiveness of a cancer patient (e.g., a patient with a cancer or a recurrence thereof) to daratumumab. The level of expression of the biomarker of SEQ ID NO: 7 may be assessed using nucleic acid amplification methods (e.g., PCR) or a device (e.g., a microarray). As is described above, the level of expression of the biomarker of SEQ ID NO: 7 in the patient sample may then be compared, e.g., to the level of expression of the biomarker of SEQ ID NO: 7 in a cell (e.g., a cancer cell) or tissue (e.g., a tumor tissue) known to be sensitive or resistant to treatment with daratumumab, or above a percentile cutoff (e.g., the 50^{th} percentile, 60th percentile, 70^{th} percentile, 80^{th} percentile, 90^{th} percentile, or greater) in a population of individuals with the cancer, and used to determine the cancer patient's responsiveness to daratumumab. The biomarker of SEQ ID NO: 7 may be used alone or in combination with one or more additional biomarker(s) (e.g., one, two, three, four, five, ten, fifteen, twenty, twenty five, or all of the biomarkers shown in Tables 1 and/or 2 (e.g., the top one biomarker shown in Tables 1 and/or 2, the top two biomarker shown in Tables 1 and/or 2, the top three biomarkers shown in Tables 1 and/or 2, the top four biomarkers shown in Tables 1 and/or 2, the top five biomarkers shown in Tables 1 and/or 2, the top ten biomarkers shown in Tables 1 and/or 2, the top fifteen biomarkers shown in Tables 1 and/or 2, the top twenty biomarkers shown in Tables 1 and/or 2, the top twenty five biomarkers shown in Tables 1 and/or 2, or all of the biomarkers shown in Tables 1 and/or 2)), such as biomarker(s) of SEQ ID NOs: 1-6, 8-53 to predict responsiveness of the cancer patient to treatment with daratumumab. The level of expression of the biomarker(s) may be determined using, e.g., a microarray, PCR, or other techniques described herein, for example, using a nucleic acid probe sequence based on the target sequences shown in Tables 1 and 2.

The biomarker of SEQ ID NO: 8 may be used to assess the responsiveness of a cancer patient (e.g., a patient with a cancer or a recurrence thereof) to daratumumab. The level of expression of the biomarker of SEQ ID NO: 8 may be assessed using nucleic acid amplification methods (e.g., PCR) or a device (e.g., a microarray). As is described above, the level of expression of the biomarker of SEQ ID NO: 8 in the patient sample may then be compared, e.g., to the level of expression of the biomarker of SEQ ID NO: 8 in a cell (e.g., a cancer cell) or tissue (e.g., a tumor tissue) known to be sensitive or resistant to treatment with daratumumab, or above a percentile cutoff (e.g., the 50^{th} percentile, 60th percentile, 70^{th} percentile, 80^{th} percentile, 90^{th} percentile, or greater) in a population of individuals with the cancer, and used to determine the cancer patient's responsiveness to daratumumab. The biomarker of SEQ ID NO: 8 may be used alone or in combination with one or more additional biomarker(s) (e.g., one, two, three, four, five, ten, fifteen, twenty, twenty five, or all of the biomarkers shown in Tables 1 and/or 2 (e.g., the top one biomarker shown in Tables 1 and/or 2, the top two biomarker shown in Tables 1 and/or 2, the top three biomarkers shown in Tables 1 and/or 2, the top four biomarkers shown in Tables 1 and/or 2, the top five biomarkers shown in Tables 1 and/or 2, the top ten biomarkers shown in Tables 1 and/or 2, the top fifteen biomarkers shown in Tables 1 and/or 2, the top twenty biomarkers shown in Tables 1 and/or 2, the top twenty five biomarkers shown in Tables 1 and/or 2, or all of the biomarkers shown in Tables 1 and/or 2)), such as biomarker(s) of SEQ ID NOs: 1-7, 9-53 to predict responsiveness of the cancer patient to treatment with daratumumab. The level of expression of the biomarker(s) may be determined using, e.g., a microarray, PCR, or other techniques described herein, for example, using a nucleic acid probe sequence based on the target sequences shown in Tables 1 and 2.

The biomarker of SEQ ID NO: 9 may be used to assess the responsiveness of a cancer patient (e.g., a patient with a cancer or a recurrence thereof) to daratumumab. The level of expression of the biomarker of SEQ ID NO: 9 may be assessed using nucleic acid amplification methods (e.g., PCR) or a device (e.g., a microarray). As is described above, the level of expression of the biomarker of SEQ ID NO: 9 in the patient sample may then be compared, e.g., to the level of expression of the biomarker of SEQ ID NO: 9 in a cell (e.g., a cancer cell) or tissue (e.g., a tumor tissue) known to be sensitive or resistant to treatment with daratumumab, or above a percentile cutoff (e.g., the 50^{th} percentile, 60th percentile, 70^{th} percentile, 80^{th} percentile, 90^{th} percentile, or greater) in a population of individuals with the cancer, and used to determine the cancer patient's responsiveness to daratumumab. The biomarker of SEQ ID NO: 9 may be used alone or in combination with one or more additional biomarker(s) (e.g., one, two, three, four, five, ten, fifteen, twenty, twenty five, or all of the biomarkers shown in Tables 1 and/or 2 (e.g., the top one biomarker shown in Tables 1 and/or 2, the top two biomarker shown in Tables 1 and/or 2, the top three biomarkers shown in Tables 1 and/or 2, the top four biomarkers shown in Tables 1 and/or 2, the top five biomarkers shown in Tables 1 and/or 2, the top ten biomarkers shown in Tables 1 and/or 2, the top fifteen biomarkers shown in Tables 1 and/or 2, the top twenty biomarkers shown in Tables 1 and/or 2, the top twenty five biomarkers shown in Tables 1 and/or 2, or all of the biomarkers shown in Tables 1 and/or 2)), such as biomarker(s) of SEQ ID NOs: 1-8, 10-53 to predict responsiveness of the cancer patient to treatment with daratumumab. The level of expression of the biomarker(s) may be determined using, e.g., a microarray, PCR, or other techniques described herein, for example, using a nucleic acid probe sequence based on the target sequences shown in Tables 1 and 2.

The biomarker of SEQ ID NO: 10 may be used to assess the responsiveness of a cancer patient (e.g., a patient with a cancer or a recurrence thereof) to daratumumab. The level of expression of the biomarker of SEQ ID NO: 10 may be assessed using nucleic acid amplification methods (e.g., PCR) or a device (e.g., a microarray). As is described above, the level of expression of the biomarker of SEQ ID NO: 10 in the patient sample may then be compared, e.g., to the level of expression of the biomarker of SEQ ID NO: 10 in a cell (e.g., a cancer cell) or tissue (e.g., a tumor tissue) known to be sensitive or resistant to treatment with daratumumab, or above a percentile cutoff (e.g., the 50^{th} percentile, 60th percentile, 70^{th} percentile, 80^{th} percentile, 90^{th} percentile, or greater) in a population of individuals with the cancer, and used to determine the cancer patient's responsiveness to daratumumab. The biomarker of SEQ ID NO: 10 may be used alone or in combination with one or more additional biomarker(s) (e.g., one, two, three, four, five, ten, fifteen, twenty, twenty five, or all of the biomarkers shown in Tables 1 and/or 2 (e.g., the top one biomarker shown in Tables 1 and/or 2, the top two biomarker shown in Tables 1 and/or 2, the top three biomarkers shown in Tables 1 and/or 2, the top four biomarkers shown in Tables 1 and/or 2, the top five biomarkers shown in Tables 1 and/or 2, the top ten biomarkers shown in Tables 1 and/or 2, the top fifteen biomarkers shown in Tables 1 and/or 2, the top twenty biomarkers shown in Tables 1 and/or 2, the top twenty five biomarkers shown in Tables 1 and/or 2, or all of the biomarkers shown in Tables 1 and/or 2)), such as biomarker(s) of SEQ ID NOs: 1-9, 11-53 to predict responsiveness of the cancer patient to treatment with daratumumab. The level of expression of the biomarker(s) may be determined using, e.g., a microarray, PCR, or other techniques described herein, for example, using a nucleic acid probe sequence based on the target sequences shown in Tables 1 and 2.

The biomarker of SEQ ID NO: 11 may be used to assess the responsiveness of a cancer patient (e.g., a patient with a cancer or a recurrence thereof) to daratumumab. The level of expression of the biomarker of SEQ ID NO: 11 may be assessed using nucleic acid amplification methods (e.g., PCR) or a device (e.g., a microarray). As is described above, the level of expression of the biomarker of SEQ ID NO: 11 in the patient sample may then be compared, e.g., to the level of expression of the biomarker of SEQ ID NO: 11 in a cell (e.g., a cancer cell) or tissue (e.g., a tumor tissue) known to be sensitive or resistant to treatment with daratumumab, or above a percentile cutoff (e.g., the 50^{th} percentile, 60th percentile, 70^{th} percentile, 80^{th} percentile, 90^{th} percentile, or greater) in a population of individuals with the cancer, and used to determine the cancer patient's responsiveness to daratumumab. The biomarker of SEQ ID NO: 11 may be used alone or in combination with one or more additional biomarker(s) (e.g., one, two, three, four, five, ten, fifteen, twenty, twenty five, or all of the biomarkers shown in Tables 1 and/or 2 (e.g., the top one biomarker shown in Tables 1 and/or 2, the top two biomarker shown in Tables 1 and/or 2, the top three biomarkers shown in Tables 1 and/or 2, the top four biomarkers shown in Tables 1 and/or 2, the top five biomarkers shown in Tables 1 and/or 2, the top ten biomarkers shown in Tables 1 and/or 2, the top fifteen biomarkers shown in Tables 1 and/or 2, the top twenty biomarkers shown in Tables 1 and/or 2, the top twenty five biomarkers shown in Tables 1 and/or 2, or all of the biomarkers shown in Tables 1 and/or 2)), such as biomarker(s) of SEQ ID NOs: 1-10, 12-53 to predict responsiveness of the cancer patient to treatment with daratumumab. The level of expression of the biomarker(s) may be determined using, e.g., a microarray, PCR, or other techniques described herein, for example, using a nucleic acid probe sequence based on the target sequences shown in Tables 1 and 2.

The biomarker of SEQ ID NO: 12 may be used to assess the responsiveness of a cancer patient (e.g., a patient with a cancer or a recurrence thereof) to daratumumab. The level of expression of the biomarker of SEQ ID NO: 12 may be assessed using nucleic acid amplification methods (e.g., PCR) or a device (e.g., a microarray). As is described above, the level of expression of the biomarker of SEQ ID NO: 12 in the patient sample may then be compared, e.g., to the level of expression of the biomarker of SEQ ID NO: 12 in a cell (e.g., a cancer cell) or tissue (e.g., a tumor tissue) known to be sensitive or resistant to treatment with daratumumab, or above a percentile cutoff (e.g., the 50^{th} percentile, 60th percentile, 70^{th} percentile, 80^{th} percentile, 90^{th} percentile, or greater) in a population of individuals with the cancer, and used to determine the cancer patient's responsiveness to daratumumab. The biomarker of SEQ ID NO: 12 may be used alone or in combination with one or more additional biomarker(s) (e.g., one, two, three, four, five, ten, fifteen, twenty, twenty five, or all of the biomarkers shown in Tables 1 and/or 2 (e.g., the top one biomarker shown in Tables 1 and/or 2, the top two biomarker shown in Tables 1 and/or 2, the top three biomarkers shown in Tables 1 and/or 2, the top four biomarkers shown in Tables 1 and/or 2, the top five biomarkers shown in Tables 1 and/or 2, the top ten biomarkers shown in Tables 1 and/or 2, the top fifteen biomarkers shown in Tables 1 and/or 2, the top twenty biomarkers shown in Tables 1 and/or 2, the top twenty five biomarkers shown in Tables 1 and/or 2, or all of the biomarkers shown in Tables 1 and/or 2)), such as biomarker(s) of SEQ ID NOs: 1-11, 13-53 to predict responsiveness of the cancer patient to treatment with daratumumab. The level of expression of the biomarker(s) may be determined using, e.g., a microarray, PCR, or other techniques described herein, for example, using a nucleic acid probe sequence based on the target sequences shown in Tables 1 and 2.

The biomarker of SEQ ID NO: 13 may be used to assess the responsiveness of a cancer patient (e.g., a patient with a cancer or a recurrence thereof) to daratumumab. The level of expression of the biomarker of SEQ ID NO: 13 may be assessed using nucleic acid amplification methods (e.g., PCR) or a device (e.g., a microarray). As is described above, the level of expression of the biomarker of SEQ ID NO: 13 in the patient sample may then be compared, e.g., to the level of expression of the biomarker of SEQ ID NO: 13 in a cell (e.g., a cancer cell) or tissue (e.g., a tumor tissue) known to be sensitive or resistant to treatment with daratumumab, or above a percentile cutoff (e.g., the 50^{th} percentile, 60th percentile, 70^{th} percentile, 80^{th} percentile, 90^{th} percentile, or greater) in a population of individuals with the cancer, and used to determine the cancer patient's responsiveness to daratumumab. The biomarker of SEQ ID NO: 13 may be used alone or in combination with one or more additional biomarker(s) (e.g., one, two, three, four, five, ten, fifteen, twenty, twenty five, or all of the biomarkers shown in Tables 1 and/or 2 (e.g., the top one biomarker shown in Tables 1 and/or 2, the top two biomarker shown in Tables 1 and/or 2, the top three biomarkers shown in Tables 1 and/or 2, the top four biomarkers shown in Tables 1 and/or 2, the top five biomarkers shown in Tables 1 and/or 2, the top ten biomarkers shown in Tables 1 and/or 2, the top fifteen biomarkers shown in Tables 1 and/or 2, the top twenty biomarkers shown in Tables 1 and/or 2, the top twenty five biomarkers shown in Tables 1 and/or 2, or all of the biomarkers shown in Tables 1 and/or 2)), such as biomarker(s) of SEQ ID NOs: 1-12, 14-53 to predict responsiveness of the cancer patient to treatment with daratumumab. The level of expression of the biomarker(s) may be determined using, e.g., a microarray, PCR, or other techniques described herein, for example, using a nucleic acid probe sequence based on the target sequences shown in Tables 1 and 2.

The biomarker of SEQ ID NO: 14 may be used to assess the responsiveness of a cancer patient (e.g., a patient with a cancer or a recurrence thereof) to daratumumab. The level of expression of the biomarker of SEQ ID NO: 14 may be assessed using nucleic acid amplification methods (e.g., PCR) or a device (e.g., a microarray). As is described above, the level of expression of the biomarker of SEQ ID NO: 14 in the patient sample may then be compared, e.g., to the level of expression of the biomarker of SEQ ID NO: 14 in a cell (e.g., a cancer cell) or tissue (e.g., a tumor tissue) known to be sensitive or resistant to treatment with daratumumab, or above a percentile cutoff (e.g., the 50^{th} percentile, 60th percentile, 70^{th} percentile, 80^{th} percentile, 90^{th} percentile, or greater) in a population of individuals with the cancer, and used to determine the cancer patient's responsiveness to daratumumab. The biomarker of SEQ ID NO: 14 may be used alone or in combination with one or more additional biomarker(s) (e.g., one, two, three, four, five, ten, fifteen, twenty, twenty five, or all of the biomarkers shown in Tables 1 and/or 2 (e.g., the top one biomarker shown in Tables 1 and/or 2, the top two biomarker shown in Tables 1 and/or 2, the top three biomarkers shown in Tables 1 and/or 2, the top four biomarkers shown in Tables 1 and/or 2, the top five biomarkers shown in Tables 1 and/or 2, the top ten biomarkers shown in Tables 1 and/or 2, the top fifteen biomarkers shown in Tables 1 and/or 2, the top twenty biomarkers shown in Tables 1 and/or 2, the top twenty five biomarkers shown in Tables 1 and/or 2, or all of the biomarkers shown in Tables 1 and/or 2)), such as biomarker(s) of SEQ ID NOs: 1-13, 15-53 to predict responsiveness of the cancer patient to treatment with daratumumab. The level of expression of the biomarker(s) may be determined using, e.g., a microarray, PCR, or other techniques described herein, for example, using a nucleic acid probe sequence based on the target sequences shown in Tables 1 and 2.

The biomarker of SEQ ID NO: 15 may be used to assess the responsiveness of a cancer patient (e.g., a patient with a cancer or a recurrence thereof) to daratumumab. The level of expression of the biomarker of SEQ ID NO: 15 may be assessed using nucleic acid amplification methods (e.g., PCR) or a device (e.g., a microarray). As is described above, the level of expression of the biomarker of SEQ ID NO: 15 in the patient sample may then be compared, e.g., to the level of expression of the biomarker of SEQ ID NO: 15 in a cell (e.g., a cancer cell) or tissue (e.g., a tumor tissue) known to be sensitive or resistant to treatment with daratumumab, or above a percentile cutoff (e.g., the 50^{th} percentile, 60th percentile, 70^{th} percentile, 80^{th} percentile, 90^{th} percentile, or greater) in a population of individuals with the cancer, and used to determine the cancer patient's responsiveness to daratumumab. The biomarker of SEQ ID NO: 15 may be used alone or in combination with one or more additional biomarker(s) (e.g., one, two, three, four, five, ten, fifteen, twenty, twenty five, or all of the biomarkers shown in Tables 1 and/or 2 (e.g., the top one biomarker shown in Tables 1 and/or 2, the top two biomarker shown in Tables 1 and/or 2, the top three biomarkers shown in Tables 1 and/or 2, the top four biomarkers shown in Tables 1 and/or 2, the top five biomarkers shown in Tables 1 and/or 2, the top ten biomarkers shown in Tables 1 and/or 2, the top fifteen biomarkers shown in Tables 1 and/or 2, the top twenty biomarkers shown in Tables 1 and/or 2, the top twenty five biomarkers shown in Tables 1 and/or 2, or all of the biomarkers shown in Tables 1 and/or 2)), such as biomarker(s) of SEQ ID NOs: 1-14, 16-53 to predict responsiveness of the cancer patient to treatment with daratumumab. The level of expression of the biomarker(s) may be determined using, e.g., a microarray, PCR, or other techniques described herein, for example, using a nucleic acid probe sequence based on the target sequences shown in Tables 1 and 2.

The biomarker of SEQ ID NO: 16 may be used to assess the responsiveness of a cancer patient (e.g., a patient with a cancer or a recurrence thereof) to daratumumab. The level of expression of the biomarker of SEQ ID NO: 16 may be assessed using nucleic acid amplification methods (e.g., PCR) or a device (e.g., a microarray). As is described above, the level of expression of the biomarker of SEQ ID NO: 16 in the patient sample may then be compared, e.g., to the level of expression of the biomarker of SEQ ID NO: 16 in a cell (e.g., a cancer cell) or tissue (e.g., a tumor tissue) known to be sensitive or resistant to treatment with daratumumab, or above a percentile cutoff (e.g., the 50^{th} percentile, 60th percentile, 70^{th} percentile, 80^{th} percentile, 90^{th} percentile, or greater) in a population of individuals with the cancer, and used to determine the cancer patient's responsiveness to daratumumab. The biomarker of SEQ ID NO: 16 may be used alone or in combination with one or more additional biomarker(s) (e.g., one, two, three, four, five, ten, fifteen, twenty, twenty five, or all of the biomarkers shown in Tables 1 and/or 2 (e.g., the top one biomarker shown in Tables 1 and/or 2, the top two biomarker shown in Tables 1 and/or 2, the top three biomarkers shown in Tables 1 and/or 2, the top four biomarkers shown in Tables 1 and/or 2, the top five biomarkers shown in Tables 1 and/or 2, the top ten biomarkers shown in Tables 1 and/or 2, the top fifteen biomarkers shown in Tables 1 and/or 2, the top twenty biomarkers shown in Tables 1 and/or 2, the top twenty five biomarkers shown in Tables 1 and/or 2, or all of the biomarkers shown in Tables 1 and/or 2)), such as biomarker(s) of SEQ ID NOs: 1-15, 17-53 to predict responsiveness of the cancer patient to treatment with daratumumab. The level of expression of the biomarker(s) may be determined using, e.g., a microarray, PCR, or other techniques described herein, for example, using a nucleic acid probe sequence based on the target sequences shown in Tables 1 and 2.

The biomarker of SEQ ID NO: 17 may be used to assess the responsiveness of a cancer patient (e.g., a patient with a cancer or a recurrence thereof) to daratumumab. The level of expression of the biomarker of SEQ ID NO: 17 may be assessed using nucleic acid amplification methods (e.g., PCR) or a device (e.g., a microarray). As is described above, the level of expression of the biomarker of SEQ ID NO: 17 in the patient sample may then be compared, e.g., to the level of expression of the biomarker of SEQ ID NO: 17 in a cell (e.g., a cancer cell) or tissue (e.g., a tumor tissue) known to be sensitive or resistant to treatment with daratumumab, or above a percentile cutoff (e.g., the 50^{th} percentile, 60th percentile, 70^{th} percentile, 80^{th} percentile, 90^{th} percentile, or greater) in a population of individuals with the cancer, and used to determine the cancer patient's responsiveness to daratumumab. The biomarker of SEQ ID NO: 17 may be used alone or in combination with one or more additional biomarker(s) (e.g., one, two, three, four, five, ten, fifteen, twenty, twenty five, or all of the biomarkers shown in Tables 1 and/or 2 (e.g., the top one biomarker shown in Tables 1 and/or 2, the top two biomarker shown in Tables 1 and/or 2, the top three biomarkers shown in Tables 1 and/or 2, the top four biomarkers shown in Tables 1 and/or 2, the top five biomarkers shown in Tables 1 and/or 2, the top ten biomarkers shown in Tables 1 and/or 2, the top fifteen biomarkers shown in Tables 1 and/or 2, the top twenty biomarkers shown in Tables 1 and/or 2, the top twenty five biomarkers shown in Tables 1 and/or 2, or all of the biomarkers shown in Tables 1 and/or 2)), such as biomarker(s) of SEQ ID NOs: 1-16, 18-53 to predict responsiveness of the cancer patient to treatment with daratumumab. The level of expression of the biomarker(s) may be determined using, e.g., a microarray, PCR, or other techniques described herein, for example, using a nucleic acid probe sequence based on the target sequences shown in Tables 1 and 2.

The biomarker of SEQ ID NO: 18 may be used to assess the responsiveness of a cancer patient (e.g., a patient with a cancer or a recurrence thereof) to daratumumab. The level of expression of the biomarker of SEQ ID NO: 18 may be assessed using nucleic acid amplification methods (e.g., PCR) or a device (e.g., a microarray). As is described above, the level of expression of the biomarker of SEQ ID NO: 18 in the patient sample may then be compared, e.g., to the level of expression of the biomarker of SEQ ID NO: 18 in a cell (e.g., a cancer cell) or tissue (e.g., a tumor tissue) known to be sensitive or resistant to treatment with daratumumab, or above a percentile cutoff (e.g., the 50^{th} percentile, 60th percentile, 70^{th} percentile, 80^{th} percentile, 90^{th} percentile, or greater) in a population of individuals with the cancer, and used to determine the cancer patient's responsiveness to daratumumab. The biomarker of SEQ ID NO: 18 may be used alone or in combination with one or more additional biomarker(s) (e.g., one, two, three, four, five, ten, fifteen, twenty, twenty five, or all of the biomarkers shown in Tables 1 and/or 2 (e.g., the top one biomarker shown in Tables 1 and/or 2, the top two biomarker shown in Tables 1 and/or 2, the top three biomarkers shown in Tables 1 and/or 2, the top four biomarkers shown in Tables 1 and/or 2, the top five biomarkers shown in Tables 1 and/or 2, the top ten biomarkers shown in Tables 1 and/or 2, the top fifteen biomarkers shown in Tables 1 and/or 2, the top twenty biomarkers shown in Tables 1 and/or 2, the top twenty five biomarkers shown in Tables 1 and/or 2, or all of the biomarkers shown in Tables 1 and/or 2)), such as biomarker(s) of SEQ ID NOs: 1-17, 19-53 to predict responsiveness of the cancer patient to treatment with daratumumab. The level of expression of the biomarker(s) may be determined using, e.g., a microarray, PCR, or other techniques described herein, for example, using a nucleic acid probe sequence based on the target sequences shown in Tables 1 and 2.

The biomarker of SEQ ID NO: 19 may be used to assess the responsiveness of a cancer patient (e.g., a patient with a cancer or a recurrence thereof) to daratumumab. The level of expression of the biomarker of SEQ ID NO: 19 may be assessed using nucleic acid amplification methods (e.g., PCR) or a device (e.g., a microarray). As is described above, the level of expression of the biomarker of SEQ ID NO: 19 in the patient sample may then be compared, e.g., to the level of expression of the biomarker of SEQ ID NO: 19 in a cell (e.g., a cancer cell) or tissue (e.g., a tumor tissue) known to be sensitive or resistant to treatment with daratumumab, or above a percentile cutoff (e.g., the 50^{th} percentile, 60th percentile, 70^{th} percentile, 80^{th} percentile, 90^{th} percentile, or greater) in a population of individuals with the cancer, and used to determine the cancer patient's responsiveness to daratumumab. The biomarker of SEQ ID NO: 19 may be used alone or in combination with one or more additional biomarker(s) (e.g., one, two, three, four, five, ten, fifteen, twenty, twenty five, or all of the biomarkers shown in Tables 1 and/or 2 (e.g., the top one biomarker shown in Tables 1 and/or 2, the top two biomarker shown in Tables 1 and/or 2, the top three biomarkers shown in Tables 1 and/or 2, the top four biomarkers shown in Tables 1 and/or 2, the top five biomarkers shown in Tables 1 and/or 2, the top ten biomarkers shown in Tables 1 and/or 2, the top fifteen biomarkers shown in Tables 1 and/or 2, the top twenty biomarkers shown in Tables 1 and/or 2, the top twenty five biomarkers shown in Tables 1 and/or 2, or all of the biomarkers shown in Tables 1 and/or 2)), such as biomarker(s) of SEQ ID NOs: 1-18, 20-53 to predict responsiveness of the cancer patient to treatment with daratumumab. The level of expression of the biomarker(s) may be determined using, e.g., a microarray, PCR, or other techniques described herein, for example, using a nucleic acid probe sequence based on the target sequences shown in Tables 1 and 2.

The biomarker of SEQ ID NO: 20 may be used to assess the responsiveness of a cancer patient (e.g., a patient with a cancer or a recurrence thereof) to daratumumab. The level of expression of the biomarker of SEQ ID NO: 20 may be assessed using nucleic acid amplification methods (e.g., PCR) or a device (e.g., a microarray). As is described above, the level of expression of the biomarker of SEQ ID NO: 20 in the patient sample may then be compared, e.g., to the level of expression of the biomarker of SEQ ID NO: 20 in a cell (e.g., a cancer cell) or tissue (e.g., a tumor tissue) known to be sensitive or resistant to treatment with daratumumab, or above a percentile cutoff (e.g., the 50^{th} percentile, 60th percentile, 70^{th} percentile, 80^{th} percentile, 90^{th} percentile, or greater) in a population of individuals with the cancer, and used to determine the cancer patient's responsiveness to daratumumab. The biomarker of SEQ ID NO: 20 may be used alone or in combination with one or more additional biomarker(s) (e.g., one, two, three, four, five, ten, fifteen, twenty, twenty five, or all of the biomarkers shown in Tables 1 and/or 2 (e.g., the top one biomarker shown in Tables 1 and/or 2, the top two biomarker shown in Tables 1 and/or 2, the top three biomarkers shown in Tables 1 and/or 2, the top four biomarkers shown in Tables 1 and/or 2, the top five biomarkers shown in Tables 1 and/or 2, the top ten biomarkers shown in Tables 1 and/or 2, the top fifteen biomarkers shown in Tables 1 and/or 2, the top twenty biomarkers shown in Tables 1 and/or 2, the top twenty five biomarkers shown in Tables 1 and/or 2, or all of the biomarkers shown in Tables 1 and/or 2)), such as biomarker(s) of SEQ ID NOs: 1-19, 21-53 to predict responsiveness of the cancer patient to treatment with daratumumab. The level of expression of the biomarker(s) may be determined using, e.g., a microarray, PCR, or other techniques described herein, for example, using a nucleic acid probe sequence based on the target sequences shown in Tables 1 and 2.

The biomarker of SEQ ID NO: 21 may be used to assess the responsiveness of a cancer patient (e.g., a patient with a cancer or a recurrence thereof) to daratumumab. The level of expression of the biomarker of SEQ ID NO: 21 may be assessed using nucleic acid amplification methods (e.g., PCR) or a device (e.g., a microarray). As is described above, the level of expression of the biomarker of SEQ ID NO: 21 in the patient sample may then be compared, e.g., to the level of expression of the biomarker of SEQ ID NO: 21 in a cell (e.g., a cancer cell) or tissue (e.g., a tumor tissue) known to be sensitive or resistant to treatment with daratumumab, or above a percentile cutoff (e.g., the 50^{th} percentile, 60th percentile, 70^{th} percentile, 80^{th} percentile, 90^{th} percentile, or greater) in a population of individuals with the cancer, and used to determine the cancer patient's responsiveness to daratumumab. The biomarker of SEQ ID NO: 21 may be used alone or in combination with one or more additional biomarker(s) (e.g., one, two, three, four, five, ten, fifteen, twenty, twenty five, or all of the biomarkers shown in Tables 1 and/or 2 (e.g., the top one biomarker shown in Tables 1 and/or 2, the top two biomarker shown in Tables 1 and/or 2, the top three biomarkers shown in Tables 1 and/or 2, the top four biomarkers shown in Tables 1 and/or 2, the top five biomarkers shown in Tables 1 and/or 2, the top ten biomarkers shown in Tables 1 and/or 2, the top fifteen biomarkers shown in Tables 1 and/or 2, the top twenty biomarkers shown in Tables 1 and/or 2, the top twenty five biomarkers shown in Tables 1 and/or 2, or all of the biomarkers shown in Tables 1 and/or 2)), such as biomarker(s) of SEQ ID NOs: 1-20, 22-53 to predict responsiveness of the cancer patient to treatment with daratumumab. The level of expression of the biomarker(s) may be determined using, e.g., a microarray, PCR, or other techniques described herein, for example, using a nucleic acid probe sequence based on the target sequences shown in Tables 1 and 2.

The biomarker of SEQ ID NO: 22 may be used to assess the responsiveness of a cancer patient (e.g., a patient with a cancer or a recurrence thereof) to daratumumab. The level of expression of the biomarker of SEQ ID NO: 22 may be assessed using nucleic acid amplification methods (e.g., PCR) or a device (e.g., a microarray). As is described above, the level of expression of the biomarker of SEQ ID NO: 22 in the patient sample may then be compared, e.g., to the level of expression of the biomarker of SEQ ID NO: 22 in a cell (e.g., a cancer cell) or tissue (e.g., a tumor tissue) known to be sensitive or resistant to treatment with daratumumab, or above a percentile cutoff (e.g., the 50^{th} percentile, 60th percentile, 70^{th} percentile, 80^{th} percentile, 90^{th} percentile, or greater) in a population of individuals with the cancer, and used to determine the cancer patient's responsiveness to daratumumab. The biomarker of SEQ ID NO: 22 may be used alone or in combination with one or more additional biomarker(s) (e.g., one, two, three, four, five, ten, fifteen, twenty, twenty five, or all of the biomarkers shown in Tables 1 and/or 2 (e.g., the top one biomarker shown in Tables 1 and/or 2, the top two biomarker shown in Tables 1 and/or 2, the top three biomarkers shown in Tables 1 and/or 2, the top four biomarkers shown in Tables 1 and/or 2, the top five biomarkers shown in Tables 1 and/or 2, the top ten biomarkers shown in Tables 1 and/or 2, the top fifteen biomarkers shown in Tables 1 and/or 2, the top twenty biomarkers shown in Tables 1 and/or 2, the top twenty five biomarkers shown in Tables 1 and/or 2, or all of the biomarkers shown in Tables 1 and/or 2)), such as biomarker(s) of SEQ ID NOs: 1-21, 23-53 to predict responsiveness of the cancer patient to treatment with daratumumab. The level of expression of the biomarker(s) may be determined using, e.g., a microarray, PCR, or other techniques described herein, for example, using a nucleic acid probe sequence based on the target sequences shown in Tables 1 and 2.

The biomarker of SEQ ID NO: 23 may be used to assess the responsiveness of a cancer patient (e.g., a patient with a cancer or a recurrence thereof) to daratumumab. The level of expression of the biomarker of SEQ ID NO: 23 may be assessed using nucleic acid amplification methods (e.g., PCR) or a device (e.g., a microarray). As is described above, the level of expression of the biomarker of SEQ ID NO: 23 in the patient sample may then be compared, e.g., to the level of expression of the biomarker of SEQ ID NO: 23 in a cell (e.g., a cancer cell) or tissue (e.g., a tumor tissue) known to be sensitive or resistant to treatment with daratumumab, or above a percentile cutoff (e.g., the 50^{th} percentile, 60th percentile, 70^{th} percentile, 80^{th} percentile, 90^{th} percentile, or greater) in a population of individuals with the cancer, and used to determine the cancer patient's responsiveness to daratumumab. The biomarker of SEQ ID NO: 23 may be used alone or in combination with one or more additional biomarker(s) (e.g., one, two, three, four, five, ten, fifteen, twenty, twenty five, or all of the biomarkers shown in Tables 1 and/or 2 (e.g., the top one biomarker shown in Tables 1 and/or 2, the top two biomarker shown in Tables 1 and/or 2, the top three biomarkers shown in Tables 1 and/or 2, the top four biomarkers shown in Tables 1 and/or 2, the top five biomarkers shown in Tables 1 and/or 2, the top ten biomarkers shown in Tables 1 and/or 2, the top fifteen biomarkers shown in Tables 1 and/or 2, the top twenty biomarkers shown in Tables 1 and/or 2, the top twenty five biomarkers shown in Tables 1 and/or 2, or all of the biomarkers shown in Tables 1 and/or 2)), such as biomarker(s) of SEQ ID NOs: 1-22, 24-53 to predict responsiveness of the cancer patient to treatment with daratumumab. The level of expression of the biomarker(s) may be determined using, e.g., a microarray, PCR, or other techniques described herein, for example, using a nucleic acid probe sequence based on the target sequences shown in Tables 1 and 2.

The biomarker of SEQ ID NO: 24 may be used to assess the responsiveness of a cancer patient (e.g., a patient with a cancer or a recurrence thereof) to daratumumab. The level of expression of the biomarker of SEQ ID NO: 24 may be assessed using nucleic acid amplification methods (e.g., PCR) or a device (e.g., a microarray). As is described above, the level of expression of the biomarker of SEQ ID NO: 24 in the patient sample may then be compared, e.g., to the level of expression of the biomarker of SEQ ID NO: 24 in a cell (e.g., a cancer cell) or tissue (e.g., a tumor tissue) known to be sensitive or resistant to treatment with daratumumab, or above a percentile cutoff (e.g., the 50^{th} percentile, 60th percentile, 70^{th} percentile, 80^{th} percentile, 90^{th} percentile, or greater) in a population of individuals with the cancer, and used to determine the cancer patient's responsiveness to daratumumab. The biomarker of SEQ ID NO: 24 may be used alone or in combination with one or more additional biomarker(s) (e.g., one, two, three, four, five, ten, fifteen, twenty, twenty five, or all of the biomarkers shown in Tables 1 and/or 2 (e.g., the top one biomarker shown in Tables 1 and/or 2, the top two biomarker shown in Tables 1 and/or 2, the top three biomarkers shown in Tables 1 and/or 2, the top four biomarkers shown in Tables 1 and/or 2, the top five biomarkers shown in Tables 1 and/or 2, the top ten biomarkers shown in Tables 1 and/or 2, the top fifteen biomarkers shown in Tables 1 and/or 2, the top twenty biomarkers shown in Tables 1 and/or 2, the top twenty five biomarkers shown in Tables 1 and/or 2, or all of the biomarkers shown in Tables 1 and/or 2)), such as biomarker(s) of SEQ ID NOs: 1-23, 25-53 to predict responsiveness of the cancer patient to treatment with daratumumab. The level of expression of the biomarker(s) may be determined using, e.g., a microarray, PCR, or other techniques described herein, for example, using a nucleic acid probe sequence based on the target sequences shown in Tables 1 and 2.

The biomarker of SEQ ID NO: 25 may be used to assess the responsiveness of a cancer patient (e.g., a patient with a cancer or a recurrence thereof) to daratumumab. The level of expression of the biomarker of SEQ ID NO: 25 may be assessed using nucleic acid amplification methods (e.g., PCR) or a device (e.g., a microarray). As is described above, the level of expression of the biomarker of SEQ ID NO: 25 in the patient sample may then be compared, e.g., to the level of expression of the biomarker of SEQ ID NO: 25 in a cell (e.g., a cancer cell) or tissue (e.g., a tumor tissue) known to be sensitive or resistant to treatment with daratumumab, or above a percentile cutoff (e.g., the 50^{th} percentile, 60th percentile, 70^{th} percentile, 80^{th} percentile, 90^{th} percentile, or greater) in a population of individuals with the cancer, and used to determine the cancer patient's responsiveness to daratumumab. The biomarker of SEQ ID NO: 25 may be used alone or in combination with one or more additional biomarker(s) (e.g., one, two, three, four, five, ten, fifteen, twenty, twenty five, or all of the biomarkers shown in Tables 1 and/or 2 (e.g., the top one biomarker shown in Tables 1 and/or 2, the top two biomarker shown in Tables 1 and/or 2, the top three biomarkers shown in Tables 1 and/or 2, the top four biomarkers shown in Tables 1 and/or 2, the top five biomarkers shown in Tables 1 and/or 2, the top ten biomarkers shown in Tables 1 and/or 2, the top fifteen biomarkers shown in Tables 1 and/or 2, the top twenty biomarkers shown in Tables 1 and/or 2, the top twenty five biomarkers shown in Tables 1 and/or 2, or all of the biomarkers shown in Tables 1 and/or 2)), such as biomarker(s) of SEQ ID NOs: 1-24, 26-53 to predict responsiveness of the cancer patient to treatment with daratumumab. The level of expression of the biomarker(s) may be determined using, e.g., a microarray, PCR, or other techniques described herein, for example, using a nucleic acid probe sequence based on the target sequences shown in Tables 1 and 2.

The biomarker of SEQ ID NO: 26 may be used to assess the responsiveness of a cancer patient (e.g., a patient with a cancer or a recurrence thereof) to daratumumab. The level of expression of the biomarker of SEQ ID NO: 26 may be assessed using nucleic acid amplification methods (e.g., PCR) or a device (e.g., a microarray). As is described above, the level of expression of the biomarker of SEQ ID NO: 26 in the patient sample may then be compared, e.g., to the level of expression of the biomarker of SEQ ID NO: 26 in a cell (e.g., a cancer cell) or tissue (e.g., a tumor tissue) known to be sensitive or resistant to treatment with daratumumab, or above a percentile cutoff (e.g., the 50^{th} percentile, 60th percentile, 70^{th} percentile, 80^{th} percentile, 90^{th} percentile, or greater) in a population of individuals with the cancer, and used to determine the cancer patient's responsiveness to daratumumab. The biomarker of SEQ ID NO: 26 may be used alone or in combination with one or more additional biomarker(s) (e.g., one, two, three, four, five, ten, fifteen, twenty, twenty five, or all of the biomarkers shown in Tables 1 and/or 2 (e.g., the top one biomarker shown in Tables 1 and/or 2, the top two biomarker shown in Tables 1 and/or 2, the top three biomarkers shown in Tables 1 and/or 2, the top four biomarkers shown in Tables 1 and/or 2, the top five biomarkers shown in Tables 1 and/or 2, the top ten biomarkers shown in Tables 1 and/or 2, the top fifteen biomarkers shown in Tables 1 and/or 2, the top twenty biomarkers shown in Tables 1 and/or 2, the top twenty five biomarkers shown in Tables 1 and/or 2, or all of the biomarkers shown in Tables 1 and/or 2)), such as biomarker(s) of SEQ ID NOs: 1-25, 27-53 to predict responsiveness of the cancer patient to treatment with daratumumab. The level of expression of the biomarker(s) may be determined using, e.g., a microarray, PCR, or other techniques described herein, for example, using a nucleic acid probe sequence based on the target sequences shown in Tables 1 and 2.

The biomarker of SEQ ID NO: 27 may be used to assess the responsiveness of a cancer patient (e.g., a patient with a cancer or a recurrence thereof) to daratumumab. The level of expression of the biomarker of SEQ ID NO: 27 may be assessed using nucleic acid amplification methods (e.g., PCR) or a device (e.g., a microarray). As is described above, the level of expression of the biomarker of SEQ ID NO: 27 in the patient sample may then be compared, e.g., to the level of expression of the biomarker of SEQ ID NO: 27 in a cell (e.g., a cancer cell) or tissue (e.g., a tumor tissue) known to be sensitive or resistant to treatment with daratumumab, or above a percentile cutoff (e.g., the 50^{th} percentile, 60th percentile, 70^{th} percentile, 80^{th} percentile, 90^{th} percentile, or greater) in a population of individuals with the cancer, and used to determine the cancer patient's responsiveness to daratumumab. The biomarker of SEQ ID NO: 27 may be used alone or in combination with one or more additional biomarker(s) (e.g., one, two, three, four, five, ten, fifteen, twenty, twenty five, or all of the biomarkers shown in Tables 1 and/or 2 (e.g., the top one biomarker shown in Tables 1 and/or 2, the top two biomarker shown in Tables 1 and/or 2, the top three biomarkers shown in Tables 1 and/or 2, the top four biomarkers shown in Tables 1 and/or 2, the top five biomarkers shown in Tables 1 and/or 2, the top ten biomarkers shown in Tables 1 and/or 2, the top fifteen biomarkers shown in Tables 1 and/or 2, the top twenty biomarkers shown in Tables 1 and/or 2, the top twenty five biomarkers shown in Tables 1 and/or 2, or all of the biomarkers shown in Tables 1 and/or 2)), such as biomarker(s) of SEQ ID NOs: 1-26, 28-53 to predict responsiveness of the cancer patient to treatment with daratumumab. The level of expression of the biomarker(s) may be determined using, e.g., a microarray, PCR, or other techniques described herein, for example, using a nucleic acid probe sequence based on the target sequences shown in Tables 1 and 2.

The biomarker of SEQ ID NO: 28 may be used to assess the responsiveness of a cancer patient (e.g., a patient with a cancer or a recurrence thereof) to daratumumab. The level of expression of the biomarker of SEQ ID NO: 28 may be assessed using nucleic acid amplification methods (e.g., PCR) or a device (e.g., a microarray). As is described above, the level of expression of the biomarker of SEQ ID NO: 28 in the patient sample may then be compared, e.g., to the level of expression of the biomarker of SEQ ID NO: 28 in a cell (e.g., a cancer cell) or tissue (e.g., a tumor tissue) known to be sensitive or resistant to treatment with daratumumab, or above a percentile cutoff (e.g., the 50^{th} percentile, 60th percentile, 70^{th} percentile, 80^{th} percentile, 90^{th} percentile, or greater) in a population of individuals with the cancer, and used to determine the cancer patient's responsiveness to daratumumab. The biomarker of SEQ ID NO: 28 may be used alone or in combination with one or more additional biomarker(s) (e.g., one, two, three, four, five, ten, fifteen, twenty, twenty five, or all of the biomarkers shown in Tables 1 and/or 2 (e.g., the top one biomarker shown in Tables 1 and/or 2, the top two biomarker shown in Tables 1 and/or 2, the top three biomarkers shown in Tables 1 and/or 2, the top four biomarkers shown in Tables 1 and/or 2, the top five biomarkers shown in Tables 1 and/or 2, the top ten biomarkers shown in Tables 1 and/or 2, the top fifteen biomarkers shown in Tables 1 and/or 2, the top twenty biomarkers shown in Tables 1 and/or 2, the top twenty five biomarkers shown in Tables 1 and/or 2, or all of the biomarkers shown in Tables 1 and/or 2)), such as biomarker(s) of SEQ ID NOs: 1-27, 29-53 to predict responsiveness of the cancer patient to treatment with daratumumab. The level of expression of the biomarker(s) may be determined using, e.g., a microarray, PCR, or other techniques described herein, for example, using a nucleic acid probe sequence based on the target sequences shown in Tables 1 and 2.

The biomarker of SEQ ID NO: 29 may be used to assess the responsiveness of a cancer patient (e.g., a patient with a cancer or a recurrence thereof) to daratumumab. The level of expression of the biomarker of SEQ ID NO: 29 may be assessed using nucleic acid amplification methods (e.g., PCR) or a device (e.g., a microarray). As is described above, the level of expression of the biomarker of SEQ ID NO: 29 in the patient sample may then be compared, e.g., to the level of expression of the biomarker of SEQ ID NO: 29 in a cell (e.g., a cancer cell) or tissue (e.g., a tumor tissue) known to be sensitive or resistant to treatment with daratumumab, or above a percentile cutoff (e.g., the 50^{th} percentile, 60th percentile, 70^{th} percentile, 80^{th} percentile, 90^{th} percentile, or greater) in a population of individuals with the cancer, and used to determine the cancer patient's responsiveness to daratumumab. The biomarker of SEQ ID NO: 29 may be used alone or in combination with one or more additional biomarker(s) (e.g., one, two, three, four, five, ten, fifteen, twenty, twenty five, or all of the biomarkers shown in Tables 1 and/or 2 (e.g., the top one biomarker shown in Tables 1 and/or 2, the top two biomarker shown in Tables 1 and/or 2, the top three biomarkers shown in Tables 1 and/or 2, the top four biomarkers shown in Tables 1 and/or 2, the top five biomarkers shown in Tables 1 and/or 2, the top ten biomarkers shown in Tables 1 and/or 2, the top fifteen biomarkers shown in Tables 1 and/or 2, the top twenty biomarkers shown in Tables 1 and/or 2, the top twenty five biomarkers shown in Tables 1 and/or 2, or all of the biomarkers shown in Tables 1 and/or 2)), such as biomarker(s) of SEQ ID NOs: 1-28, 30-53 to predict responsiveness of the cancer patient to treatment with daratumumab. The level of expression of the biomarker(s) may be determined using, e.g., a microarray, PCR, or other techniques described herein, for example, using a nucleic acid probe sequence based on the target sequences shown in Tables 1 and 2.

The biomarker of SEQ ID NO: 30 may be used to assess the responsiveness of a cancer patient (e.g., a patient with a cancer or a recurrence thereof) to daratumumab. The level of expression of the biomarker of SEQ ID NO: 30 may be assessed using nucleic acid amplification methods (e.g., PCR) or a device (e.g., a microarray). As is described above, the level of expression of the biomarker of SEQ ID NO: 30 in the patient sample may then be compared, e.g., to the level of expression of the biomarker of SEQ ID NO: 30 in a cell (e.g., a cancer cell) or tissue (e.g., a tumor tissue) known to be sensitive or resistant to treatment with daratumumab, or above a percentile cutoff (e.g., the 50^{th} percentile, 60th percentile, 70^{th} percentile, 80^{th} percentile, 90^{th} percentile, or greater) in a population of individuals with the cancer, and used to determine the cancer patient's responsiveness to daratumumab. The biomarker of SEQ ID NO: 30 may be used alone or in combination with one or more additional biomarker(s) (e.g., one, two, three, four, five, ten, fifteen, twenty, twenty five, or all of the biomarkers shown in Tables 1 and/or 2 (e.g., the top one biomarker shown in Tables 1 and/or 2, the top two biomarker shown in Tables 1 and/or 2, the top three biomarkers shown in Tables 1 and/or 2, the top four biomarkers shown in Tables 1 and/or 2, the top five biomarkers shown in Tables 1 and/or 2, the top ten biomarkers shown in Tables 1 and/or 2, the top fifteen biomarkers shown in Tables 1 and/or 2, the top twenty biomarkers shown in Tables 1 and/or 2, the top twenty five biomarkers shown in Tables 1 and/or 2, or all of the biomarkers shown in Tables 1 and/or 2)), such as biomarker(s) of SEQ ID NOs: 1-29, 31-53 to predict responsiveness of the cancer patient to treatment with daratumumab. The level of expression of the biomarker(s) may be determined using, e.g., a microarray, PCR, or other techniques described herein, for example, using a nucleic acid probe sequence based on the target sequences shown in Tables 1 and 2.

The biomarker of SEQ ID NO: 31 may be used to assess the responsiveness of a cancer patient (e.g., a patient with a cancer or a recurrence thereof) to daratumumab. The level of expression of the biomarker of SEQ ID NO: 31 may be assessed using nucleic acid amplification methods (e.g., PCR) or a device (e.g., a microarray). As is described above, the level of expression of the biomarker of SEQ ID NO: 31 in the patient sample may then be compared, e.g., to the level of expression of the biomarker of SEQ ID NO: 31 in a cell (e.g., a cancer cell) or tissue (e.g., a tumor tissue) known to be sensitive or resistant to treatment with daratumumab, or above a percentile cutoff (e.g., the 50^{th} percentile, 60th percentile, 70^{th} percentile, 80^{th} percentile, 90^{th} percentile, or greater) in a population of individuals with the cancer, and used to determine the cancer patient's responsiveness to daratumumab. The biomarker of SEQ ID NO: 31 may be used alone or in combination with one or more additional biomarker(s) (e.g., one, two, three, four, five, ten, fifteen, twenty, twenty five, or all of the biomarkers shown in Tables 1 and/or 2 (e.g., the top one biomarker shown in Tables 1 and/or 2, the top two biomarker shown in Tables 1 and/or 2, the top three biomarkers shown in Tables 1 and/or 2, the top four biomarkers shown in Tables 1 and/or 2, the top five biomarkers shown in Tables 1 and/or 2, the top ten biomarkers shown in Tables 1 and/or 2, the top fifteen biomarkers shown in Tables 1 and/or 2, the top twenty biomarkers shown in Tables 1 and/or 2, the top twenty five biomarkers shown in Tables 1 and/or 2, or all of the biomarkers shown in Tables 1 and/or 2)), such as biomarker(s) of SEQ ID NOs: 1-30, 32-53 to predict responsiveness of the cancer patient to treatment with daratumumab. The level of expression of the biomarker(s) may be determined using, e.g., a microarray, PCR, or other techniques described herein, for example, using a nucleic acid probe sequence based on the target sequences shown in Tables 1 and 2.

The biomarker of SEQ ID NO: 32 may be used to assess the responsiveness of a cancer patient (e.g., a patient with a cancer or a recurrence thereof) to daratumumab. The level of expression of the biomarker of SEQ ID NO: 32 may be assessed using nucleic acid amplification methods (e.g., PCR) or a device (e.g., a microarray). As is described above, the level of expression of the biomarker of SEQ ID NO: 32 in the patient sample may then be compared, e.g., to the level of expression of the biomarker of SEQ ID NO: 32 in a cell (e.g., a cancer cell) or tissue (e.g., a tumor tissue) known to be sensitive or resistant to treatment with daratumumab, or above a percentile cutoff (e.g., the 50^{th} percentile, 60th percentile, 70^{th} percentile, 80^{th} percentile, 90^{th} percentile, or greater) in a population of individuals with the cancer, and used to determine the cancer patient's responsiveness to daratumumab. The biomarker of SEQ ID NO: 32 may be used alone or in combination with one or more additional biomarker(s) (e.g., one, two, three, four, five, ten, fifteen, twenty, twenty five, or all of the biomarkers shown in Tables 1 and/or 2 (e.g., the top one biomarker shown in Tables 1 and/or 2, the top two biomarker shown in Tables 1 and/or 2, the top three biomarkers shown in Tables 1 and/or 2, the top four biomarkers shown in Tables 1 and/or 2, the top five biomarkers shown in Tables 1 and/or 2, the top ten biomarkers shown in Tables 1 and/or 2, the top fifteen biomarkers shown in Tables 1 and/or 2, the top twenty biomarkers shown in Tables 1 and/or 2, the top twenty five biomarkers shown in Tables 1 and/or 2, or all of the biomarkers shown in Tables 1 and/or 2)), such as biomarker(s) of SEQ ID NOs: 1-31, 33-53 to predict responsiveness of the cancer patient to treatment with daratumumab. The level of expression of the biomarker(s) may be determined using, e.g., a microarray, PCR, or other techniques described herein, for example, using a nucleic acid probe sequence based on the target sequences shown in Tables 1 and 2.

The biomarker of SEQ ID NO: 33 may be used to assess the responsiveness of a cancer patient (e.g., a patient with a cancer or a recurrence thereof) to daratumumab. The level of expression of the biomarker of SEQ ID NO: 33 may be assessed using nucleic acid amplification methods (e.g., PCR) or a device (e.g., a microarray). As is described above, the level of expression of the biomarker of SEQ ID NO: 33 in the patient sample may then be compared, e.g., to the level of expression of the biomarker of SEQ ID NO: 33 in a cell (e.g., a cancer cell) or tissue (e.g., a tumor tissue) known to be sensitive or resistant to treatment with daratumumab, or above a percentile cutoff (e.g., the 50^{th} percentile, 60th percentile, 70^{th} percentile, 80^{th} percentile, 90^{th} percentile, or greater) in a population of individuals with the cancer, and used to determine the cancer patient's responsiveness to daratumumab. The biomarker of SEQ ID NO: 33 may be used alone or in combination with one or more additional biomarker(s) (e.g., one, two, three, four, five, ten, fifteen, twenty, twenty five, or all of the biomarkers shown in Tables 1 and/or 2 (e.g., the top one biomarker shown in Tables 1 and/or 2, the top two biomarker shown in Tables 1 and/or 2, the top three biomarkers shown in Tables 1 and/or 2, the top four biomarkers shown in Tables 1 and/or 2, the top five biomarkers shown in Tables 1 and/or 2, the top ten biomarkers shown in Tables 1 and/or 2, the top fifteen biomarkers shown in Tables 1 and/or 2, the top twenty biomarkers shown in Tables 1 and/or 2, the top twenty five biomarkers shown in Tables 1 and/or 2, or all of the biomarkers shown in Tables 1 and/or 2)), such as biomarker(s) of SEQ ID NOs: 1-32, 34-53 to predict responsiveness of the cancer patient to treatment with daratumumab. The level of expression of the biomarker(s) may be determined using, e.g., a microarray, PCR, or other techniques described herein, for example, using a nucleic acid probe sequence based on the target sequences shown in Tables 1 and 2.

The biomarker of SEQ ID NO: 34 may be used to assess the responsiveness of a cancer patient (e.g., a patient with a cancer or a recurrence thereof) to daratumumab. The level of expression of the biomarker of SEQ ID NO: 34 may be assessed using nucleic acid amplification methods (e.g., PCR) or a device (e.g., a microarray). As is described above, the level of expression of the biomarker of SEQ ID NO: 34 in the patient sample may then be compared, e.g., to the level of expression of the biomarker of SEQ ID NO: 34 in a cell (e.g., a cancer cell) or tissue (e.g., a tumor tissue) known to be sensitive or resistant to treatment with daratumumab, or above a percentile cutoff (e.g., the 50^{th} percentile, 60th percentile, 70^{th} percentile, 80^{th} percentile, 90^{th} percentile, or greater) in a population of individuals with the cancer, and used to determine the cancer patient's responsiveness to daratumumab. The biomarker of SEQ ID NO: 34 may be used alone or in combination with one or more additional biomarker(s) (e.g., one, two, three, four, five, ten, fifteen, twenty, twenty five, or all of the biomarkers shown in Tables 1 and/or 2 (e.g., the top one biomarker shown in Tables 1 and/or 2, the top two biomarker shown in Tables 1 and/or 2, the top three biomarkers shown in Tables 1 and/or 2, the top four biomarkers shown in Tables 1 and/or 2, the top five biomarkers shown in Tables 1 and/or 2, the top ten biomarkers shown in Tables 1 and/or 2, the top fifteen biomarkers shown in Tables 1 and/or 2, the top twenty biomarkers shown in Tables 1 and/or 2, the top twenty five biomarkers shown in Tables 1 and/or 2, or all of the biomarkers shown in Tables 1 and/or 2)), such as biomarker(s) of SEQ ID NOs: 1-33, 35-53 to predict responsiveness of the cancer patient to treatment with daratumumab. The level of expression of the biomarker(s) may be determined using, e.g., a microarray, PCR, or other techniques described herein, for example, using a nucleic acid probe sequence based on the target sequences shown in Tables 1 and 2.

The biomarker of SEQ ID NO: 35 may be used to assess the responsiveness of a cancer patient (e.g., a patient with a cancer or a recurrence thereof) to daratumumab. The level of expression of the biomarker of SEQ ID NO: 35 may be assessed using nucleic acid amplification methods (e.g., PCR) or a device (e.g., a microarray). As is described above, the level of expression of the biomarker of SEQ ID NO: 35 in the patient sample may then be compared, e.g., to the level of expression of the biomarker of SEQ ID NO: 35 in a cell (e.g., a cancer cell) or tissue (e.g., a tumor tissue) known to be sensitive or resistant to treatment with daratumumab, or above a percentile cutoff (e.g., the 50^{th} percentile, 60th percentile, 70^{th} percentile, 80^{th} percentile, 90^{th} percentile, or greater) in a population of individuals with the cancer, and used to determine the cancer patient's responsiveness to daratumumab. The biomarker of SEQ ID NO: 35 may be used alone or in combination with one or more additional biomarker(s) (e.g., one, two, three, four, five, ten, fifteen, twenty, twenty five, or all of the biomarkers shown in Tables 1 and/or 2 (e.g., the top one biomarker shown in Tables 1 and/or 2, the top two biomarker shown in Tables 1 and/or 2, the top three biomarkers shown in Tables 1 and/or 2, the top four biomarkers shown in Tables 1 and/or 2, the top five biomarkers shown in Tables 1 and/or 2, the top ten biomarkers shown in Tables 1 and/or 2, the top fifteen biomarkers shown in Tables 1 and/or 2, the top twenty biomarkers shown in Tables 1 and/or 2, the top twenty five biomarkers shown in Tables 1 and/or 2, or all of the biomarkers shown in Tables 1 and/or 2)), such as biomarker(s) of SEQ ID NOs: 1-34, 36-53 to predict responsiveness of the cancer patient to treatment with daratumumab. The level of expression of the biomarker(s) may be determined using, e.g., a microarray, PCR, or other techniques described herein, for example, using a nucleic acid probe sequence based on the target sequences shown in Tables 1 and 2.

The biomarker of SEQ ID NO: 36 may be used to assess the responsiveness of a cancer patient (e.g., a patient with a cancer or a recurrence thereof) to daratumumab. The level of expression of the biomarker of SEQ ID NO: 36 may be assessed using nucleic acid amplification methods (e.g., PCR) or a device (e.g., a microarray). As is described above, the level of expression of the biomarker of SEQ ID NO: 36 in the patient sample may then be compared, e.g., to the level of expression of the biomarker of SEQ ID NO: 36 in a cell (e.g., a cancer cell) or tissue (e.g., a tumor tissue) known to be sensitive or resistant to treatment with daratumumab, or above a percentile cutoff (e.g., the 50^{th} percentile, 60th percentile, 70^{th} percentile, 80^{th} percentile, 90^{th} percentile, or greater) in a population of individuals with the cancer, and used to determine the cancer patient's responsiveness to daratumumab. The biomarker of SEQ ID NO: 36 may be used alone or in combination with one or more additional biomarker(s) (e.g., one, two, three, four, five, ten, fifteen, twenty, twenty five, or all of the biomarkers shown in Tables 1 and/or 2 (e.g., the top one biomarker shown in Tables 1 and/or 2, the top two biomarker shown in Tables 1 and/or 2, the top three biomarkers shown in Tables 1 and/or 2, the top four biomarkers shown in Tables 1 and/or 2, the top five biomarkers shown in Tables 1 and/or 2, the top ten biomarkers shown in Tables 1 and/or 2, the top fifteen biomarkers shown in Tables 1 and/or 2, the top twenty biomarkers shown in Tables 1 and/or 2, the top twenty five biomarkers shown in Tables 1 and/or 2, or all of the biomarkers shown in Tables 1 and/or 2)), such as biomarker(s) of SEQ ID NOs: 1-35, 37-53 to predict responsiveness of the cancer patient to treatment with daratumumab. The level of expression of the biomarker(s) may be determined using, e.g., a microarray, PCR, or other techniques described herein, for example, using a nucleic acid probe sequence based on the target sequences shown in Tables 1 and 2.

The biomarker of SEQ ID NO: 37 may be used to assess the responsiveness of a cancer patient (e.g., a patient with a cancer or a recurrence thereof) to daratumumab. The level of expression of the biomarker of SEQ ID NO: 37 may be assessed using nucleic acid amplification methods (e.g., PCR) or a device (e.g., a microarray). As is described above, the level of expression of the biomarker of SEQ ID NO: 37 in the patient sample may then be compared, e.g., to the level of expression of the biomarker of SEQ ID NO: 37 in a cell (e.g., a cancer cell) or tissue (e.g., a tumor tissue) known to be sensitive or resistant to treatment with daratumumab, or above a percentile cutoff (e.g., the 50^{th} percentile, 60th percentile, 70^{th} percentile, 80^{th} percentile, 90^{th} percentile, or greater) in a population of individuals with the cancer, and used to determine the cancer patient's responsiveness to daratumumab. The biomarker of SEQ ID NO: 37 may be used alone or in combination with one or more additional biomarker(s) (e.g., one, two, three, four, five, ten, fifteen, twenty, twenty five, or all of the biomarkers shown in Tables 1 and/or 2 (e.g., the top one biomarker shown in Tables 1 and/or 2, the top two biomarker shown in Tables 1 and/or 2, the top three biomarkers shown in Tables 1 and/or 2, the top four biomarkers shown in Tables 1 and/or 2, the top five biomarkers shown in Tables 1 and/or 2, the top ten biomarkers shown in Tables 1 and/or 2, the top fifteen biomarkers shown in Tables 1 and/or 2, the top twenty biomarkers shown in Tables 1 and/or 2, the top twenty five biomarkers shown in Tables 1 and/or 2, or all of the biomarkers shown in Tables 1 and/or 2)), such as biomarker(s) of SEQ ID NOs: 1-36, 38-53 to predict responsiveness of the cancer patient to treatment with daratumumab. The level of expression of the biomarker(s) may be determined using, e.g., a microarray, PCR, or other techniques described herein, for example, using a nucleic acid probe sequence based on the target sequences shown in Tables 1 and 2.

The biomarker of SEQ ID NO: 38 may be used to assess the responsiveness of a cancer patient (e.g., a patient with a cancer or a recurrence thereof) to daratumumab. The level of expression of the biomarker of SEQ ID NO: 38 may be assessed using nucleic acid amplification methods (e.g., PCR) or a device (e.g., a microarray). As is described above, the level of expression of the biomarker of SEQ ID NO: 38 in the patient sample may then be compared, e.g., to the level of expression of the biomarker of SEQ ID NO: 38 in a cell (e.g., a cancer cell) or tissue (e.g., a tumor tissue) known to be sensitive or resistant to treatment with daratumumab, or above a percentile cutoff (e.g., the 50^{th} percentile, 60th percentile, 70^{th} percentile, 80^{th} percentile, 90^{th} percentile, or greater) in a population of individuals with the cancer, and used to determine the cancer patient's responsiveness to daratumumab. The biomarker of SEQ ID NO: 38 may be used alone or in combination with one or more additional biomarker(s) (e.g., one, two, three, four, five, ten, fifteen, twenty, twenty five, or all of the biomarkers shown in Tables 1 and/or 2 (e.g., the top one biomarker shown in Tables 1 and/or 2, the top two biomarker shown in Tables 1 and/or 2, the top three biomarkers shown in Tables 1 and/or 2, the top four biomarkers shown in Tables 1 and/or 2, the top five biomarkers shown in Tables 1 and/or 2, the top ten biomarkers shown in Tables 1 and/or 2, the top fifteen biomarkers shown in Tables 1 and/or 2, the top twenty biomarkers shown in Tables 1 and/or 2, the top twenty five biomarkers shown in Tables 1 and/or 2, or all of the biomarkers shown in Tables 1 and/or 2)), such as biomarker(s) of SEQ ID NOs: 1-37, 39-53 to predict responsiveness of the cancer patient to treatment with daratumumab. The level of expression of the biomarker(s) may be determined using, e.g., a microarray, PCR, or other techniques described herein, for example, using a nucleic acid probe sequence based on the target sequences shown in Tables 1 and 2.

The biomarker of SEQ ID NO: 39 may be used to assess the responsiveness of a cancer patient (e.g., a patient with a cancer or a recurrence thereof) to daratumumab. The level of expression of the biomarker of SEQ ID NO: 39 may be assessed using nucleic acid amplification methods (e.g., PCR) or a device (e.g., a microarray). As is described above, the level of expression of the biomarker of SEQ ID NO: 39 in the patient sample may then be compared, e.g., to the level of expression of the biomarker of SEQ ID NO: 39 in a cell (e.g., a cancer cell) or tissue (e.g., a tumor tissue) known to be sensitive or resistant to treatment with daratumumab, or above a percentile cutoff (e.g., the 50^{th} percentile, 60th percentile, 70^{th} percentile, 80^{th} percentile, 90^{th} percentile, or greater) in a population of individuals with the cancer, and used to determine the cancer patient's responsiveness to daratumumab. The biomarker of SEQ ID NO: 39 may be used alone or in combination with one or more additional biomarker(s) (e.g., one, two, three, four, five, ten, fifteen, twenty, twenty five, or all of the biomarkers shown in Tables 1 and/or 2 (e.g., the top one biomarker shown in Tables 1 and/or 2, the top two biomarker shown in Tables 1 and/or 2, the top three biomarkers shown in Tables 1 and/or 2, the top four biomarkers shown in Tables 1 and/or 2, the top five biomarkers shown in Tables 1 and/or 2, the top ten biomarkers shown in Tables 1 and/or 2, the top fifteen biomarkers shown in Tables 1 and/or 2, the top twenty biomarkers shown in Tables 1 and/or 2, the top twenty five biomarkers shown in Tables 1 and/or 2, or all of the biomarkers shown in Tables 1 and/or 2)), such as biomarker(s) of SEQ ID NOs: 1-38, 40-53 to predict responsiveness of the cancer patient to treatment with daratumumab. The level of expression of the biomarker(s) may be determined using, e.g., a microarray, PCR, or other techniques described herein, for example, using a nucleic acid probe sequence based on the target sequences shown in Tables 1 and 2.

The biomarker of SEQ ID NO: 40 may be used to assess the responsiveness of a cancer patient (e.g., a patient with a cancer or a recurrence thereof) to daratumumab. The level of expression of the biomarker of SEQ ID NO: 40 may be assessed using nucleic acid amplification methods (e.g., PCR) or a device (e.g., a microarray). As is described above, the level of expression of the biomarker of SEQ ID NO: 40 in the patient sample may then be compared, e.g., to the level of expression of the biomarker of SEQ ID NO: 40 in a cell (e.g., a cancer cell) or tissue (e.g., a tumor tissue) known to be sensitive or resistant to treatment with daratumumab, or above a percentile cutoff (e.g., the 50^{th} percentile, 60th percentile, 70^{th} percentile, 80^{th} percentile, 90^{th} percentile, or greater) in a population of individuals with the cancer, and used to determine the cancer patient's responsiveness to daratumumab. The biomarker of SEQ ID NO: 40 may be used alone or in combination with one or more additional biomarker(s) (e.g., one, two, three, four, five, ten, fifteen, twenty, twenty five, or all of the biomarkers shown in Tables 1 and/or 2 (e.g., the top one biomarker shown in Tables 1 and/or 2, the top two biomarker shown in Tables 1 and/or 2, the top three biomarkers shown in Tables 1 and/or 2, the top four biomarkers shown in Tables 1 and/or 2, the top five biomarkers shown in Tables 1 and/or 2, the top ten biomarkers shown in Tables 1 and/or 2, the top fifteen biomarkers shown in Tables 1 and/or 2, the top twenty biomarkers shown in Tables 1 and/or 2, the top twenty five biomarkers shown in Tables 1 and/or 2, or all of the biomarkers shown in Tables 1 and/or 2)), such as biomarker(s) of SEQ ID NOs: 1-39, 41-53 to predict responsiveness of the cancer patient to treatment with daratumumab. The level of expression of the biomarker(s) may be determined using, e.g., a microarray, PCR, or other techniques described herein, for example, using a nucleic acid probe sequence based on the target sequences shown in Tables 1 and 2.

The biomarker of SEQ ID NO: 41 may be used to assess the responsiveness of a cancer patient (e.g., a patient with a cancer or a recurrence thereof) to daratumumab. The level of expression of the biomarker of SEQ ID NO: 41 may be assessed using nucleic acid amplification methods (e.g., PCR) or a device (e.g., a microarray). As is described above, the level of expression of the biomarker of SEQ ID NO: 41 in the patient sample may then be compared, e.g., to the level of expression of the biomarker of SEQ ID NO: 41 in a cell (e.g., a cancer cell) or tissue (e.g., a tumor tissue) known to be sensitive or resistant to treatment with daratumumab, or above a percentile cutoff (e.g., the 50^{th} percentile, 60th percentile, 70^{th} percentile, 80^{th} percentile, 90^{th} percentile, or greater) in a population of individuals with the cancer, and used to determine the cancer patient's responsiveness to daratumumab. The biomarker of SEQ ID NO: 41 may be used alone or in combination with one or more additional biomarker(s) (e.g., one, two, three, four, five, ten, fifteen, twenty, twenty five, or all of the biomarkers shown in Tables 1 and/or 2 (e.g., the top one biomarker shown in Tables 1 and/or 2, the top two biomarker shown in Tables 1 and/or 2, the top three biomarkers shown in Tables 1 and/or 2, the top four biomarkers shown in Tables 1 and/or 2, the top five biomarkers shown in Tables 1 and/or 2, the top ten biomarkers shown in Tables 1 and/or 2, the top fifteen biomarkers shown in Tables 1 and/or 2, the top twenty biomarkers shown in Tables 1 and/or 2, the top twenty five biomarkers shown in Tables 1 and/or 2, or all of the biomarkers shown in Tables 1 and/or 2)), such as biomarker(s) of SEQ ID NOs: 1-40, 42-53 to predict responsiveness of the cancer patient to treatment with daratumumab. The level of expression of the biomarker(s) may be determined using, e.g., a microarray, PCR, or other techniques described herein, for example, using a nucleic acid probe sequence based on the target sequences shown in Tables 1 and 2.

The biomarker of SEQ ID NO: 42 may be used to assess the responsiveness of a cancer patient (e.g., a patient with a cancer or a recurrence thereof) to daratumumab. The level of expression of the biomarker of SEQ ID NO: 42 may be assessed using nucleic acid amplification methods (e.g., PCR) or a device (e.g., a microarray). As is described above, the level of expression of the biomarker of SEQ ID NO: 42 in the patient sample may then be compared, e.g., to the level of expression of the biomarker of SEQ ID NO: 42 in a cell (e.g., a cancer cell) or tissue (e.g., a tumor tissue) known to be sensitive or resistant to treatment with daratumumab, or above a percentile cutoff (e.g., the 50^{th} percentile, 60th percentile, 70^{th} percentile, 80^{th} percentile, 90^{th} percentile, or greater) in a population of individuals with the cancer, and used to determine the cancer patient's responsiveness to daratumumab. The biomarker of SEQ ID NO: 42 may be used alone or in combination with one or more additional biomarker(s) (e.g., one, two, three, four, five, ten, fifteen, twenty, twenty five, or all of the biomarkers shown in Tables 1 and/or 2 (e.g., the top one biomarker shown in Tables 1 and/or 2, the top two biomarker shown in Tables 1 and/or 2, the top three biomarkers shown in Tables 1 and/or 2, the top four biomarkers shown in Tables 1 and/or 2, the top five biomarkers shown in Tables 1 and/or 2, the top ten biomarkers shown in Tables 1 and/or 2, the top fifteen biomarkers shown in Tables 1 and/or 2, the top twenty biomarkers shown in Tables 1 and/or 2, the top twenty five biomarkers shown in Tables 1 and/or 2, or all of the biomarkers shown in Tables 1 and/or 2)), such as biomarker(s) of SEQ ID NOs: 1-41, 43-53 to predict responsiveness of the cancer patient to treatment with daratumumab. The level of expression of the biomarker(s) may be determined using, e.g., a microarray, PCR, or other techniques described herein, for example, using a nucleic acid probe sequence based on the target sequences shown in Tables 1 and 2.

The biomarker of SEQ ID NO: 43 may be used to assess the responsiveness of a cancer patient (e.g., a patient with a cancer or a recurrence thereof) to daratumumab. The level of expression of the biomarker of SEQ ID NO: 43 may be assessed using nucleic acid amplification methods (e.g., PCR) or a device (e.g., a microarray). As is described above, the level of expression of the biomarker of SEQ ID NO: 43 in the patient sample may then be compared, e.g., to the level of expression of the biomarker of SEQ ID NO: 43 in a cell (e.g., a cancer cell) or tissue (e.g., a tumor tissue) known to be sensitive or resistant to treatment with daratumumab, or above a percentile cutoff (e.g., the 50^{th} percentile, 60th percentile, 70^{th} percentile, 80^{th} percentile, 90^{th} percentile, or greater) in a population of individuals with the cancer, and used to determine the cancer patient's responsiveness to daratumumab. The biomarker of SEQ ID NO: 43 may be used alone or in combination with one or more additional biomarker(s) (e.g., one, two, three, four, five, ten, fifteen, twenty, twenty five, or all of the biomarkers shown in Tables 1 and/or 2 (e.g., the top one biomarker shown in Tables 1 and/or 2, the top two biomarker shown in Tables 1 and/or 2, the top three biomarkers shown in Tables 1 and/or 2, the top four biomarkers shown in Tables 1 and/or 2, the top five biomarkers shown in Tables 1 and/or 2, the top ten biomarkers shown in Tables 1 and/or 2, the top fifteen biomarkers shown in Tables 1 and/or 2, the top twenty biomarkers shown in Tables 1 and/or 2, the top twenty five biomarkers shown in Tables 1 and/or 2, or all of the biomarkers shown in Tables 1 and/or 2)), such as biomarker(s) of SEQ ID NOs: 1-42, 44-53 to predict responsiveness of the cancer patient to treatment with daratumumab. The level of expression of the biomarker(s) may be determined using, e.g., a microarray, PCR, or other techniques described herein, for example, using a nucleic acid probe sequence based on the target sequences shown in Tables 1 and 2.

The biomarker of SEQ ID NO: 44 may be used to assess the responsiveness of a cancer patient (e.g., a patient with a cancer or a recurrence thereof) to daratumumab. The level of expression of the biomarker of SEQ ID NO: 44 may be assessed using nucleic acid amplification methods (e.g., PCR) or a device (e.g., a microarray). As is described above, the level of expression of the biomarker of SEQ ID NO: 44 in the patient sample may then be compared, e.g., to the level of expression of the biomarker of SEQ ID NO: 44 in a cell (e.g., a cancer cell) or tissue (e.g., a tumor tissue) known to be sensitive or resistant to treatment with daratumumab, or above a percentile cutoff (e.g., the 50^{th} percentile, 60th percentile, 70^{th} percentile, 80^{th} percentile, 90^{th} percentile, or greater) in a population of individuals with the cancer, and used to determine the cancer patient's responsiveness to daratumumab. The biomarker of SEQ ID NO: 44 may be used alone or in combination with one or more additional biomarker(s) (e.g., one, two, three, four, five, ten, fifteen, twenty, twenty five, or all of the biomarkers shown in Tables 1 and/or 2 (e.g., the top one biomarker shown in Tables 1 and/or 2, the top two biomarker shown in Tables 1 and/or 2, the top three biomarkers shown in Tables 1 and/or 2, the top four biomarkers shown in Tables 1 and/or 2, the top five biomarkers shown in Tables 1 and/or 2, the top ten biomarkers shown in Tables 1 and/or 2, the top fifteen biomarkers shown in Tables 1 and/or 2, the top twenty biomarkers shown in Tables 1 and/or 2, the top twenty five biomarkers shown in Tables 1 and/or 2, or all of the biomarkers shown in Tables 1 and/or 2)), such as biomarker(s) of SEQ ID NOs: 1-43, 45-53 to predict responsiveness of the cancer patient to treatment with daratumumab. The level of expression of the biomarker(s) may be determined using, e.g., a microarray, PCR, or other techniques described herein, for example, using a nucleic acid probe sequence based on the target sequences shown in Tables 1 and 2.

The biomarker of SEQ ID NO: 45 may be used to assess the responsiveness of a cancer patient (e.g., a patient with a cancer or a recurrence thereof) to daratumumab. The level of expression of the biomarker of SEQ ID NO: 45 may be assessed using nucleic acid amplification methods (e.g., PCR) or a device (e.g., a microarray). As is described above, the level of expression of the biomarker of SEQ ID NO: 45 in the patient sample may then be compared, e.g., to the level of expression of the biomarker of SEQ ID NO: 45 in a cell (e.g., a cancer cell) or tissue (e.g., a tumor tissue) known to be sensitive or resistant to treatment with daratumumab, or above a percentile cutoff (e.g., the 50^{th} percentile, 60th percentile, 70^{th} percentile, 80^{th} percentile, 90^{th} percentile, or greater) in a population of individuals with the cancer, and used to determine the cancer patient's responsiveness to daratumumab. The biomarker of SEQ ID NO: 45 may be used alone or in combination with one or more additional biomarker(s) (e.g., one, two, three, four, five, ten, fifteen, twenty, twenty five, or all of the biomarkers shown in Tables 1 and/or 2 (e.g., the top one biomarker shown in Tables 1 and/or 2, the top two biomarker shown in Tables 1 and/or 2, the top three biomarkers shown in Tables 1 and/or 2, the top four biomarkers shown in Tables 1 and/or 2, the top five biomarkers shown in Tables 1 and/or 2, the top ten biomarkers shown in Tables 1 and/or 2, the top fifteen biomarkers shown in Tables 1 and/or 2, the top twenty biomarkers shown in Tables 1 and/or 2, the top twenty five biomarkers shown in Tables 1 and/or 2, or all of the biomarkers shown in Tables 1 and/or 2)), such as biomarker(s) of SEQ ID NOs: 1-44, 46-53 to predict responsiveness of the cancer patient to treatment with daratumumab. The level of expression of the biomarker(s) may be determined using, e.g., a microarray, PCR, or other techniques described herein, for example, using a nucleic acid probe sequence based on the target sequences shown in Tables 1 and 2.

The biomarker of SEQ ID NO: 46 may be used to assess the responsiveness of a cancer patient (e.g., a patient with a cancer or a recurrence thereof) to daratumumab. The level of expression of the biomarker of SEQ ID NO: 46 may be assessed using nucleic acid amplification methods (e.g., PCR) or a device (e.g., a microarray). As is described above, the level of expression of the biomarker of SEQ ID NO: 46 in the patient sample may then be compared, e.g., to the level of expression of the biomarker of SEQ ID NO: 46 in a cell (e.g., a cancer cell) or tissue (e.g., a tumor tissue) known to be sensitive or resistant to treatment with daratumumab, or above a percentile cutoff (e.g., the 50^{th} percentile, 60th percentile, 70^{th} percentile, 80^{th} percentile, 90^{th} percentile, or greater) in a population of individuals with the cancer, and used to determine the cancer patient's responsiveness to daratumumab. The biomarker of SEQ ID NO: 46 may be used alone or in combination with one or more additional biomarker(s) (e.g., one, two, three, four, five, ten, fifteen, twenty, twenty five, or all of the biomarkers shown in Tables 1 and/or 2 (e.g., the top one biomarker shown in Tables 1 and/or 2, the top two biomarker shown in Tables 1 and/or 2, the top three biomarkers shown in Tables 1 and/or 2, the top four biomarkers shown in Tables 1 and/or 2, the top five biomarkers shown in Tables 1 and/or 2, the top ten biomarkers shown in Tables 1 and/or 2, the top fifteen biomarkers shown in Tables 1 and/or 2, the top twenty biomarkers shown in Tables 1 and/or 2, the top twenty five biomarkers shown in Tables 1 and/or 2, or all of the biomarkers shown in Tables 1 and/or 2)), such as biomarker(s) of SEQ ID NOs: 1-45, 47-53 to predict responsiveness of the cancer patient to treatment with daratumumab. The level of expression of the biomarker(s) may be determined using, e.g., a microarray, PCR, or other techniques described herein, for example, using a nucleic acid probe sequence based on the target sequences shown in Tables 1 and 2.

The biomarker of SEQ ID NO: 47 may be used to assess the responsiveness of a cancer patient (e.g., a patient with a cancer or a recurrence thereof) to daratumumab. The level of expression of the biomarker of SEQ ID NO: 47 may be assessed using nucleic acid amplification methods (e.g., PCR) or a device (e.g., a microarray). As is described above, the level of expression of the biomarker of SEQ ID NO: 47 in the patient sample may then be compared, e.g., to the level of expression of the biomarker of SEQ ID NO: 47 in a cell (e.g., a cancer cell) or tissue (e.g., a tumor tissue) known to be sensitive or resistant to treatment with daratumumab, or above a percentile cutoff (e.g., the 50^{th} percentile, 60th percentile, 70^{th} percentile, 80^{th} percentile, 90^{th} percentile, or greater) in a population of individuals with the cancer, and used to determine the cancer patient's responsiveness to daratumumab. The biomarker of SEQ ID NO: 47 may be used alone or in combination with one or more additional biomarker(s) (e.g., one, two, three, four, five, ten, fifteen, twenty, twenty five, or all of the biomarkers shown in Tables 1 and/or 2 (e.g., the top one biomarker shown in Tables 1 and/or 2, the top two biomarker shown in Tables 1 and/or 2, the top three biomarkers shown in Tables 1 and/or 2, the top four biomarkers shown in Tables 1 and/or 2, the top five biomarkers shown in Tables 1 and/or 2, the top ten biomarkers shown in Tables 1 and/or 2, the top fifteen biomarkers shown in Tables 1 and/or 2, the top twenty biomarkers shown in Tables 1 and/or 2, the top twenty five biomarkers shown in Tables 1 and/or 2, or all of the biomarkers shown in Tables 1 and/or 2)), such as biomarker(s) of SEQ ID NOs: 1-46, 48-53 to predict responsiveness of the cancer patient to treatment with daratumumab. The level of expression of the biomarker(s) may be determined using, e.g., a microarray, PCR, or other techniques described herein, for example, using a nucleic acid probe sequence based on the target sequences shown in Tables 1 and 2.

The biomarker of SEQ ID NO: 48 may be used to assess the responsiveness of a cancer patient (e.g., a patient with a cancer or a recurrence thereof) to daratumumab. The level of expression of the biomarker of SEQ ID NO: 48 may be assessed using nucleic acid amplification methods (e.g., PCR) or a device (e.g., a microarray). As is described above, the level of expression of the biomarker of SEQ ID NO: 48 in the patient sample may then be compared, e.g., to the level of expression of the biomarker of SEQ ID NO: 48 in a cell (e.g., a cancer cell) or tissue (e.g., a tumor tissue) known to be sensitive or resistant to treatment with daratumumab, or above a percentile cutoff (e.g., the 50^{th} percentile, 60th percentile, 70^{th} percentile, 80^{th} percentile, 90^{th} percentile, or greater) in a population of individuals with the cancer, and used to determine the cancer patient's responsiveness to daratumumab. The biomarker of SEQ ID NO: 48 may be used alone or in combination with one or more additional biomarker(s) (e.g., one, two, three, four, five, ten, fifteen, twenty, twenty five, or all of the biomarkers shown in Tables 1 and/or 2 (e.g., the top one biomarker shown in Tables 1 and/or 2, the top two biomarker shown in Tables 1 and/or 2, the top three biomarkers shown in Tables 1 and/or 2, the top four biomarkers shown in Tables 1 and/or 2, the top five biomarkers shown in Tables 1 and/or 2, the top ten biomarkers shown in Tables 1 and/or 2, the top fifteen biomarkers shown in Tables 1 and/or 2, the top twenty biomarkers shown in Tables 1 and/or 2, the top twenty five biomarkers shown in Tables 1 and/or 2, or all of the biomarkers shown in Tables 1 and/or 2)), such as biomarker(s) of SEQ ID NOs: 1-47, 49-53 to predict responsiveness of the cancer patient to treatment with daratumumab. The level of expression of the biomarker(s) may be determined using, e.g., a microarray, PCR, or other techniques described herein, for example, using a nucleic acid probe sequence based on the target sequences shown in Tables 1 and 2.

The biomarker of SEQ ID NO: 49 may be used to assess the responsiveness of a cancer patient (e.g., a patient with a cancer or a recurrence thereof) to daratumumab. The level of expression of the biomarker of SEQ ID NO: 49 may be assessed using nucleic acid amplification methods (e.g., PCR) or a device (e.g., a microarray). As is described above, the level of expression of the biomarker of SEQ ID NO: 49 in the patient sample may then be compared, e.g., to the level of expression of the biomarker of SEQ ID NO: 49 in a cell (e.g., a cancer cell) or tissue (e.g., a tumor tissue) known to be sensitive or resistant to treatment with daratumumab, or above a percentile cutoff (e.g., the 50^{th} percentile, 60th percentile, 70^{th} percentile, 80^{th} percentile, 90^{th} percentile, or greater) in a population of individuals with the cancer, and used to determine the cancer patient's responsiveness to daratumumab. The biomarker of SEQ ID NO: 49 may be used alone or in combination with one or more additional biomarker(s) (e.g., one, two, three, four, five, ten, fifteen, twenty, twenty five, or all of the biomarkers shown in Tables 1 and/or 2 (e.g., the top one biomarker shown in Tables 1 and/or 2, the top two biomarker shown in Tables 1 and/or 2, the top three biomarkers shown in Tables 1 and/or 2, the top four biomarkers shown in Tables 1 and/or 2, the top five biomarkers shown in Tables 1 and/or 2, the top ten biomarkers shown in Tables 1 and/or 2, the top fifteen biomarkers shown in Tables 1 and/or 2, the top twenty biomarkers shown in Tables 1 and/or 2, the top twenty five biomarkers shown in Tables 1 and/or 2, or all of the biomarkers shown in Tables 1 and/or 2)), such as biomarker(s) of SEQ ID NOs: 1-48, 50-53 to predict responsiveness of the cancer patient to treatment with daratumumab. The level of expression of the biomarker(s) may be determined using, e.g., a microarray, PCR, or other techniques described herein, for example, using a nucleic acid probe sequence based on the target sequences shown in Tables 1 and 2.

The biomarker of SEQ ID NO: 50 may be used to assess the responsiveness of a cancer patient (e.g., a patient with a cancer or a recurrence thereof) to daratumumab. The level of expression of the biomarker of SEQ ID NO: 50 may be assessed using nucleic acid amplification methods (e.g., PCR) or a device (e.g., a microarray). As is described above, the level of expression of the biomarker of SEQ ID NO: 50 in the patient sample may then be compared, e.g., to the level of expression of the biomarker of SEQ ID NO: 50 in a cell (e.g., a cancer cell) or tissue (e.g., a tumor tissue) known to be sensitive or resistant to treatment with daratumumab, or above a percentile cutoff (e.g., the 50^{th} percentile, 60th percentile, 70^{th} percentile, 80^{th} percentile, 90^{th} percentile, or greater) in a population of individuals with the cancer, and used to determine the cancer patient's responsiveness to daratumumab. The biomarker of SEQ ID NO: 50 may be used alone or in combination with one or more additional biomarker(s) (e.g., one, two, three, four, five, ten, fifteen, twenty, twenty five, or all of the biomarkers shown in Tables 1 and/or 2 (e.g., the top one biomarker shown in Tables 1 and/or 2, the top two biomarker shown in Tables 1 and/or 2, the top three biomarkers shown in Tables 1 and/or 2, the top four biomarkers shown in Tables 1 and/or 2, the top five biomarkers shown in Tables 1 and/or 2, the top ten biomarkers shown in Tables 1 and/or 2, the top fifteen biomarkers shown in Tables 1 and/or 2, the top twenty biomarkers shown in Tables 1 and/or 2, the top twenty five biomarkers shown in Tables 1 and/or 2, or all of the biomarkers shown in Tables 1 and/or 2)), such as biomarker(s) of SEQ ID NOs: 1-49, 51-53 to predict responsiveness of the cancer patient to treatment with daratumumab. The level of expression of the biomarker(s) may be determined using, e.g., a microarray, PCR, or other techniques described herein, for example, using a nucleic acid probe sequence based on the target sequences shown in Tables 1 and 2.

The biomarker of SEQ ID NO: 51 may be used to assess the responsiveness of a cancer patient (e.g., a patient with a cancer or a recurrence thereof) to daratumumab. The level of expression of the biomarker of SEQ ID NO: 51 may be assessed using nucleic acid amplification methods (e.g., PCR) or a device (e.g., a microarray). As is described above, the level of expression of the biomarker of SEQ ID NO: 51 in the patient sample may then be compared, e.g., to the level of expression of the biomarker of SEQ ID NO: 51 in a cell (e.g., a cancer cell) or tissue (e.g., a tumor tissue) known to be sensitive or resistant to treatment with daratumumab, or above a percentile cutoff (e.g., the 50^{th} percentile, 60th percentile, 70^{th} percentile, 80^{th} percentile, 90^{th} percentile, or greater) in a population of individuals with the cancer, and used to determine the cancer patient's responsiveness to daratumumab. The biomarker of SEQ ID NO: 51 may be used alone or in combination with one or more additional biomarker(s) (e.g., one, two, three, four, five, ten, fifteen, twenty, twenty five, or all of the biomarkers shown in Tables 1 and/or 2 (e.g., the top one biomarker shown in Tables 1 and/or 2, the top two biomarker shown in Tables 1 and/or 2, the top three biomarkers shown in Tables 1 and/or 2, the top four biomarkers shown in Tables 1 and/or 2, the top five biomarkers shown in Tables 1 and/or 2, the top ten biomarkers shown in Tables 1 and/or 2, the top fifteen biomarkers shown in Tables 1 and/or 2, the top twenty biomarkers shown in Tables 1 and/or 2, the top twenty five biomarkers shown in Tables 1 and/or 2, or all of the biomarkers shown in Tables 1 and/or 2)), such as biomarker(s) of SEQ ID NOs: 1-50, 52, and 53 to predict responsiveness of the cancer patient to treatment with daratumumab. The level of expression of the biomarker(s) may be determined using, e.g., a microarray, PCR, or other techniques described herein, for example, using a nucleic acid probe sequence based on the target sequences shown in Tables 1 and 2.

The biomarker of SEQ ID NO: 52 may be used to assess the responsiveness of a cancer patient (e.g., a patient with a cancer or a recurrence thereof) to daratumumab. The level of expression of the biomarker of SEQ ID NO: 52 may be assessed using nucleic acid amplification methods (e.g., PCR) or a device (e.g., a microarray). As is described above, the level of expression of the biomarker of SEQ ID NO: 52 in the patient sample may then be compared, e.g., to the level of expression of the biomarker of SEQ ID NO: 52 in a cell (e.g., a cancer cell) or tissue (e.g., a tumor tissue) known to be sensitive or resistant to treatment with daratumumab, or above a percentile cutoff (e.g., the 50^{th} percentile, 60th percentile, 70^{th} percentile, 80^{th} percentile, 90^{th} percentile, or greater) in a population of individuals with the cancer, and used to determine the cancer patient's responsiveness to daratumumab. The biomarker of SEQ ID NO: 52 may be used alone or in combination with one or more additional biomarker(s) (e.g., one, two, three, four, five, ten, fifteen, twenty, twenty five, or all of the biomarkers shown in Tables 1 and/or 2 (e.g., the top one biomarker shown in Tables 1 and/or 2, the top two biomarker shown in Tables 1 and/or 2, the top three biomarkers shown in Tables 1 and/or 2, the top four biomarkers shown in Tables 1 and/or 2, the top five biomarkers shown in Tables 1 and/or 2, the top ten biomarkers shown in Tables 1 and/or 2, the top fifteen biomarkers shown in Tables 1 and/or 2, the top twenty biomarkers shown in Tables 1 and/or 2, the top twenty five biomarkers shown in Tables 1 and/or 2, or all of the biomarkers shown in Tables 1 and/or 2)), such as biomarker(s) of SEQ ID NOs: 1-51 and 53 to predict responsiveness of the cancer patient to treatment with daratumumab. The level of expression of the biomarker(s) may be determined using, e.g., a microarray, PCR, or other techniques described herein, for example, using a nucleic acid probe sequence based on the target sequences shown in Tables 1 and 2.

The biomarker of SEQ ID NO: 53 may be used to assess the responsiveness of a cancer patient (e.g., a patient with a cancer or a recurrence thereof) to daratumumab. The level of expression of the biomarker of SEQ ID NO: 53 may be assessed using nucleic acid amplification methods (e.g., PCR) or a device (e.g., a microarray). As is described above, the level of expression of the biomarker of SEQ ID NO: 53 in the patient sample may then be compared, e.g., to the level of expression of the biomarker of SEQ ID NO: 53 in a cell (e.g., a cancer cell) or tissue (e.g., a tumor tissue) known to be sensitive or resistant to treatment with daratumumab, or above a percentile cutoff (e.g., the 50^{th} percentile, 60th percentile, 70^{th} percentile, 80^{th} percentile, 90^{th} percentile, or greater) in a population of individuals with the cancer, and used to determine the cancer patient's responsiveness to daratumumab. The biomarker of SEQ ID NO: 53 may be used alone or in combination with one or more additional biomarker(s) (e.g., one, two, three, four, five, ten, fifteen, twenty, twenty five, or all of the biomarkers shown in Tables 1 and/or 2 (e.g., the top one biomarker shown in Tables 1 and/or 2, the top two biomarker shown in Tables 1 and/or 2, the top three biomarkers shown in Tables 1 and/or 2, the top four biomarkers shown in Tables 1 and/or 2, the top five biomarkers shown in Tables 1 and/or 2, the top ten biomarkers shown in Tables 1 and/or 2, the top fifteen biomarkers shown in Tables 1 and/or 2, the top twenty biomarkers shown in Tables 1 and/or 2, the top twenty five biomarkers shown in Tables 1 and/or 2, or all of the biomarkers shown in Tables 1 and/or 2)), such as biomarker(s) of SEQ ID NOs: 1-52 to predict responsiveness of the cancer patient to treatment with daratumumab. The level of expression of the biomarker(s) may be determined using, e.g., a microarray, PCR, or other techniques described herein, for example, using a nucleic acid probe sequence based on the target sequences shown in Tables 1 and 2.

### Methods of Treatment

The methods of the disclosure permit the assessment of whether a subject with a cancer (e.g., MM or NHL; e.g., MM) is likely to be responsive to treatment with daratumumab and, thus, can be used to direct the subject's treatment (e.g., as a first line therapy and/or as a second or third line therapy). A subject to be treated or tested for responsiveness to daratumumab according to the methods may include, e.g., a subject that has been diagnosed with a cancer, e.g., multiple myeloma and non-Hodgkin's lymphoma (e.g., follicular lymphoma, diffuse large B cell lymphoma, mantle cell lymphoma, and Burkitt's lymphoma). In some instances, the subject receiving daratumumab for treatment has not received prior therapy for the multiple myeloma. In some instances, the subject has relapsed after or is refractory to at least one prior therapy for multiple myeloma. In some instances, the at least one prior therapy includes an alkylating agent, a proteasome inhibitor, and/or an immunomodulatory agent. In some instances, the at least one prior therapy includes bortezomib, lenalidomide, pomalidomide, carfilzomib, autologous stem cell transplant therapy, or a combination thereof. In some instances, the subject has relapsed after or is refractory to one, two, or three prior lines of therapy.

A subject found to be responsive to daratumumab according to the methods of the disclosure may be selected for treatment with daratumumab. For example, a subject can be identified as responsive to daratumumab by determining the level of expression of one or more biomarkers (e.g., one or more of the biomarkers shown in Table(s) 1 and/or 2, such as NR3C1 (SEQ ID NO: 1) and/or CCND1 (SEQ ID NO: 28)) in a biological sample (e.g., a tissue sample; e.g., a biopsy sample; e.g., a bone marrow sample or a bone marrow biopsy sample) obtained from the subject, and subsequently administered daratumumab. One or more additional therapies (e.g., surgery, radiation, or a therapeutic agent) may also be administered to the subject prior to, concurrently with, or after administration of daratumumab. A therapeutic agent that can be administered to the subject prior to, concurrently with, or after administration of daratumumab includes a corticosteroid, an antipyretic, an antihistamine, a chemotherapeutic agent, or a combination thereof. In some instances, (a) the corticosteroid is methylprednisolone or dexamethasone; (b) the antipyretic is acetaminophen/paracetamol; and/or (c) the antihistamine is diphenhydramine. Alternatively, a therapeutic agent that can be administered to the subject prior to, concurrently with, or after administration of daratumumab includes lenalidomide, dexamethasone, bortezomib, melphalan, prednisone, thalidomide, pomalidomide, or a combination thereof. In particular, the following combinations of therapeutic agents are possible: (a) daratumumab and lenalidomide and dexamethasone; (b) daratumumab and bortezomib and melphalan and prednisone; (c) daratumumab and bortezomib and thalidomide and dexamethasone; (d) daratumumab and bortezomib and dexamethasone; or (e) daratumumab and pomalidomide and dexamethasone.

Alternatively, a subject can be, e.g., identified as less likely to be responsive to daratumumab by determining the level of expression of one or more biomarkers (e.g., one or more of the biomarkers shown in Table(s) 1 and/or 2, such as NR3C1 (SEQ ID NO: 1) and/or CCND1 (SEQ ID NO: 28)) in a biological sample obtained from the subject. If the subject exhibits levels of expression of one or more biomarkers indicative of non-responsiveness to daratumumab, the subject can be administered a treatment other than daratumumab (e.g., surgery, radiation, or a therapeutic agent). In particular, the therapeutic agent may be one or more of lenalidomide, dexamethasone, bortezomib, melphalan, prednisone, thalidomide, pomalidomide, or a combination thereof.

### Administration of daratumumab

Once the subject has been determined to be responsive to daratumumab, according to the methods described herein, daratumumab may be administered to the subject, for example, by way of intravenous infusion. Daratumumab may be administered by way of intravenous infusion at a rate of 50 mL/hr, 100 mL/hr, 150 mL/hr, or 200 mL/hr and/or over a period of 3.25-6.5 hours. Daratumumab may be administered once a week, once every two weeks, once every three weeks, and/or once every four weeks. Daratumumab may be administered to the subject at a dose of about 16 mg/kg. Daratumumab may be administered as two sub-doses, wherein each sub-dose is half the amount of the dose, i.e., 8 mg/kg each. In some instances, the second sub-dose may be administered one day after administration of the first sub-dose.

Daratumumab may be administered according to one of the following dosing regimens: (a)1 dose per week for weeks 1-8, followed by 1 dose every two weeks starting on week 9 for weeks 9-24, and then followed by 1 dose every four weeks thereafter starting on week 25; (b) 1 dose per week for weeks 1-6, followed by 1 dose every three weeks starting on week 7 for weeks 7-54 starting on week 7, and then followed by 1 dose every four weeks thereafter starting on week 55; (c) 1 dose per week for weeks 1-9, followed by 1 dose every three weeks starting on week 10 for weeks 10-24, and then followed by 1 dose every four weeks thereafter starting on week 25; (d) 1 dose per week for weeks 1-8, followed by 1 dose every two weeks starting on week 9 for weeks 9-16, followed by a pause, and then followed by 1 dose every two weeks for weeks 1-8 after reinitiation of dosing; or (e) 1 dose split into two sub-doses, each comprising half the amount of the 1 dose, administered on Days 1 and 2 of week 1, 1 dose per week for weeks 2-8, followed by 1 dose every two weeks starting on week 9 for weeks 9-24, and then followed by 1 dose every four weeks thereafter starting on week 25.

Daratumumab can be administered in a pharmaceutical composition that includes one or more pharmaceutically acceptable carriers, excipients, or diluents. Examples of suitable carriers, excipients, or diluents of daratumumab include, e.g., saline, sterile water, polyalkylene glycols, oils of vegetable origin, hydrogenated napthalenes, suitable buffer, 1,3-butanediol, Ringer's solution and/or sodium chloride solution. Exemplary formulations for parenteral administration can include solutions prepared in water suitably mixed with a surfactant, e.g., hydroxypropylcellulose. Dispersions can also be prepared in glycerol, liquid polyethylene glycols, DMSO and mixtures thereof with or without alcohol, and in oils. Under ordinary conditions of storage and use, these preparations may contain a preservative to prevent the growth of microorganisms. Other exemplary carriers, excipients, or diluents are described in the Handbook of Pharmaceutical Excipients, 6th Edition, Rowe et al., Eds., Pharmaceutical Press (2009), hereby incorporated by reference in its entirety.

A pharmaceutical composition can be formulated to be compatible with its intended route of administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Daratumumab may be formulated as a 100 mg/5 mL (20 mg/mL) solution in a single-dose vial or a 400 mg/20 mL (20 mg/mL) solution in a single-dose vial. Exemplary formulations for daratumumab include: 400 mg daratumumab, glacial acetic acid (3.7 mg), mannitol (510 mg), polysorbate 20 (8 mg), sodium acetate trihydrate (59.3 mg), sodium chloride (70.1 mg), and Water for Injection, USP, in a 20 mL single-dose vial; 400 mg daratumumab, L-histidine (7 mg), L-histidine hydrochloride monohydrate (32.6 mg), L-methionine (20 mg), polysorbate 20 (8 mg), sorbitol (1093 mg), and Water for Injection, USP, in a 20 mL single-dose vial; 100 mg daratumumab, glacial acetic acid (0.9 mg), mannitol (127.5 mg), polysorbate 20 (2 mg), sodium acetate trihydrate (14.8 mg), sodium chloride (17.5 mg), and Water for Injection, USP, in a 5 mL single-dose vial; or 100 mg daratumumab, L-histidine (1.8 mg), L-histidine hydrochloride monohydrate (8.2 mg), L-methionine (5 mg), polysorbate 20 (2 mg), sorbitol (273.3 mg), and Water for Injection, USP, in a 5 mL single-dose vial.

Pharmaceutical compositions suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, or phosphate buffered saline (PBS). In all cases, the composition must be sterile and should be fluid to the extent that easy syringability exists. It must be stable under the conditions of manufacture and storage and must be preserved against the contaminating action of microorganisms, such as bacteria and fungi. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as mannitol, sorbitol, and sodium chloride in the composition. Prolonged absorption of the injectable compositions can be brought about by including in the composition an agent which delays absorption, for example, aluminum monostearate and gelatin.

Sterile injectable solutions can be prepared by incorporating daratumumab in the required amount in an appropriate solvent with one or a combination of ingredients enumerated above, as required, followed by filtered sterilization. Dispersions can be prepared by incorporating daratumumab into a sterile vehicle which contains a basic dispersion medium and the required other ingredients from those enumerated above. In the case of sterile powders for the preparation of sterile injectable solutions, the preferred methods of preparation can be vacuum drying and freeze-drying which yields lyophilized daratumumab plus any additional desired ingredient from a previously sterile-filtered solution thereof.

The pharmaceutical compositions can be included in a container, pack, or dispenser together with instructions for administration.

Methods of formulating pharmaceutical agents are known in the art, e.g., Niazi, Handbook of Pharmaceutical Manufacturing Formulations (Second Edition), CRC Press 2009*,* describes formulation development for liquid, sterile, compressed, semi-compressed and OTC forms. Transdermal and mucosal delivery, lymphatic system delivery, nanoparticles, controlled drug release systems, theranostics, protein and peptide drugs, and biologics delivery are described in Wang et al., Drug Delivery: Principles and Applications (Second Edition), Wiley 2016; formulation and delivery of peptide and protein agent is described, e.g., in Banga, Therapeutic Peptides and Proteins: Formulation, Processing, and Delivery Systems (Third Edition), CRC Press 2015*.*

### Kits

Kits of the disclosure can be used for determining the responsiveness of a cancer patient (e.g., multiple myeloma or non-Hodgkin's lymphoma (e.g., a diffuse large B cell lymphoma, follicular lymphoma, mantle cell lymphoma, or Burkitt's lymphoma)) to daratumumab. Kits of the disclosure can include reagents and/or materials for, e.g., collecting and/or purifying nucleic acids from biological samples (such as those obtained from a patient to be treated with daratumumab), reagents for amplifying such nucleic acids to produce an amplified sample, and/or at least one device of the invention. Reagents for amplifying nucleic acids may include, e.g., PCR reagents, including but not limited to DNA polymerase, RNA polymerase, PCR buffer, magnesium chloride solutions, nucleic acid primers (e.g., primers designed to target particular biomarkers of responsiveness to daratumumab), and/or any other PCR reagents as are well known in the art. In particular, kits useful in the method may include includes one or more of the following: a kit for RNA extraction from tumors (e.g., Trizol for mRNA, mirVana miRNA isolation kit from Ambion Inc), a kit for RNA labeling (e.g., MessageAmp from Ambion Inc., FlashTag from Genisphere Inc), a microarray for measuring biomarker expression (e.g., HG-U133A, HG-U133_Plus2 or miRNA-1.0 from Affymetrix Inc), a microarray hybridization station and scanner (e.g., GeneChip System 3000Dx from Affymetrix Inc), and/or software for analyzing the expression of biomarker genes or RNAs (e.g., miRNAs) as described in herein (e.g., implemented in R from R-Project or S-Plus from Insightful Corp.).

For example, a kit of the disclosure can include one or more probes capable of detecting one or more biomarkers of Table(s) 1 and/or 2 (e.g., the kit may include probes for the biomarkers of Tables 1 and 2). Such probes can, for example, include nucleic acids capable of hybridizing to the biomarker based on nucleic acid sequence complementarity. In particular, a probe has at least 85% sequence identity (e.g., 85%, 90%, 95%, 97%, 98%, 99%, or 100% sequence identity) to a nucleic acid sequence that is complementary or identical to at least 5 (e.g., at least 15) consecutive nucleotides of one or more biomarkers. The probes can be attached a solid surface, such as a microarray. The kit may include NanoString capture probes, NanoString reporter probes, and/or one or more nCounter cartridges. The kit may include reagents for next generation sequencing, including but not limited to poly(T) oligonucleotides, dye terminators, sequencing adapters, adapter ligation reagents, reverse transcriptase, primers (e.g., random primers), DNA-cleaving enzymes, polymerases, and/or any combination thereof. The kit may also be one that includes a protein array and/or reagents for detection of the polypeptide product(s) of one or more biomarkers of Table(s) 1 and/or 2.

### EXAMPLES

### Example 1. Identification of biomarkers of sensitivity and resistance to daratumumab and development of a drug response predictor (DRP) for daratumumab

Sixteen multiple myeloma cell lines were treated with daratumumab in the presence of peripheral blood mononuclear cells (PBMCs) enriched for natural killer (NK) cells as external effectors and antibody-dependent cellular cytotoxicity (ADCC) quantified by calcein release assay (Table 3).

**Table 3. Daratumumab induces ADCC in CD38-expressing multiple myeloma (MM) cell lines. Data show maximal killing at 0.1 µg/ml obtained in a representative experiment. The experiment was repeated two to three times, depending on the cell line tested. Data from Michel de Weers et al., J Immunol. 186(3): 1840-1848, 2011.**

| **Cell Line** | **Daratumumab (% Lysis)** |
|---|---|
| Daudi | 61.5 |
| MM1R | 43.2 |
| MM1S | 47.5 |
| 28BM | 23.8 |
| 28PE | 6.1 |
| 12BM | 15.4 |
| 12PE | 39.1 |
| RPMI 8226 | 54.2 |
| LR5 | 39.2 |
| DOX40 | 39.3 |
| L363 | 52.1 |
| H929 | 65.2 |
| INA6 | 18.0 |
| LP-1 | 54.6 |
| UM-9 | 27.2 |
| U266 | 0 |

Fifteen B-cell non-Hodgkin lymphoma (NHL) cell lines were treated with daratumumab in the presence of peripheral blood mononuclear cells (PBMC) from healthy donors as external effectors and antibody-dependent cellular cytotoxicity (ADCC) quantified by calcein release assay (Table 4).

**Table 4. Daratumumab-induced ADCC in B-cell NHL cell lines. Data from Anna Vidal-Crespo et al., Haematologica.;105(4):1032-1041, 2019.**

| **Cell line** | **Subtype** | **%ADCC** |
|---|---|---|
| Jeko | MCL | 58 |
| REC-1 | MCL | 60 |
| HBL2 | MCL | 28 |
| Mino | MCL | 32 |
| UPN1 | MCL | 36 |
| ZI138 | MCL | 53 |
| DOHH2 | FL | 25 |
| SC-1 | FL | 41 |
| RL | FL | 52 |
| WSU-FSCCL | FL | 67 |
| Toledo | DLBCL | 34 |
| WSU-DLCL2 | DLBCL | 45 |
| SU-DHL-6 | DLBCL | 64 |
| SU-DHL-4 | DLBCL | 61 |
| Daudi | BL | 63 |

The 15 NHL and 16 multiple myeloma cell lines were matched against the Cancer Cell Line Encyclopedia (CCLE) repository of Affymetrix^{®} baseline analysis of cell lines (in the absence of daratumumab), and transcription was matched for 19 cell lines (MM1R; 28PE; 12PE; LR5; DOX40; INA6; UM-9; HBL2; UPN1; Z138; SC-1; and WSU-FSCCL have not been assayed in CCLE, Daudi cell line was present in both panels and gave almost identical % lysis in the two experiments: 62% and 63%).

The % lysis numbers for each cell line were used together with the Affymetrix^{®} CEL files for each cell to identify genes correlated to daratumumab sensitivity (correlation coefficient (CC) > 0.45) and genes correlated to daratumumab resistance (CC < -0.45). The resulting genes are shown in Table 1 (biomarkers of daratumumab sensitivity) and Table 2 (biomarkers of daratumumab resistance), above:

### In Vitro Validation

To test the extent to which the genes identified in Table 1 and 2 are predictive of in vitro sensitivity to daratumumab, three tests were performed. First the final DRP with 53 (27+26) genes was applied to the 19 cell lines with gene expression, and the predicted sensitivity was compared to the measured % lysis. The correlation was 0.86 (95% confidence interval (CI): 0.65-0.94), but this is an overestimate of predictive performance, because the set of 19 cell lines used to identify the 53 genes is identical to the 19 cell lines used to evaluate their performance. A more realistic test of performance, as well as data integrity and comparability of in vitro results, used the 16-7=9 myeloma cell lines to develop a DRP and then applied it to predict the % lysis in the 15-5=10 B-cell NHL cell lines. The Pearson correlation was 0.319 (95% CI: -0.28-1.0).

### Retrospective clinical validation

### Clinical Dataset

The KYDAR trial, published in Nature Medicine (Yael et al., Nature Medicine. 27(3):491-503, 2021) recruited 41 newly diagnosed MM subjects, who either failed to respond or experienced early relapse after a bortezomib-containing induction regimen, were enrolled to evaluate the safety and efficacy of a daratumumab, carfilzomib, lenalidomide and dexamethasone combination. The primary clinical endpoint was safety and tolerability. Secondary endpoints included overall response rate, progression-free survival and overall survival BM cells from relapse-refractory patients were stained with magnetic CD138+ bead enrichment (Miltenyi).

### Results

The 53 genes in the DRP-Daratumumab were converted to gene symbols and matched 51 genes in the KYDAR scRNAseq data.

DRPs for carfilzomib, lenalidomide, and dexamethasone were converted as well and resulted in a combined DRP for all four drugs with 303 genes that matched the scRNAseq data (133 positively correlated and 170 negatively correlated). If the same gene appeared in two DRPs, it was counted twice in the final combined DRP.

A difference score was calculated for each subject (having outcome information) by subtracting the mean of the z-scores from the negatively correlated genes from the mean of the z-scores from the positively correlated genes. The z-scores were taken from clone 1 for each subject in Table S2 of Yael et al. (the KYDAR Nature Medicine paper). Z-scores for clone 1 of each subject were used because clone 1 was the dominant clone at baseline (see Fig 5b of Yael et al. (*supra*), which is partially reproduced as **FIG. 1** herein).

There are only two outcome measures listed in Figure 4e (reproduced as **FIG. 2** herein) of the Nature Medicine paper: IMWG response at cycle 4, and whether or not PR was achieved in the 6 months since treatment start (responder/nonresponder). When these are encoded numerically (CR = 1, VGPR = 2, PR = 3, SD = 4, PD=5) and (responder = 2, nonresponder = 2), the Pearson correlation to the raw DRP difference score can be calculated for the combined DRP of 4 drugs (Table 5). In addition, Table 5 shows a sensitivity analysis where one DRP is removed at a time (-Dara, -Dex, etc.). The sensitivity analysis shows that the only contribution to prediction power is DRP-Daratumumab (-Dara, Dara only. It is no surprise then, that DRP-Daratumumab alone is the best predictor of response to the daratumumab combination treatment.

**Table 5. Pearson correlation between DRP score and numerically encoded treatment outcome. Correlations in bold are statistically significant.**

| | 4 DRPs | -Dara | -Dex | -Len | -Car | Dara only |
|---|---|---|---|---|---|---|
| Response at cycle 4 | 0.29 | 0.38 | -0.15 | 0.26 | 0.27 | -0.26 |
| PR within 6 months | -0.03 | 0.10 | -0.06 | -0.11 | -0.14 | -0.59 |

Additional results and analyses are shown in **FIG. 2** - **FIG. 4**. **FIG. 2** shows the DRP-Daratumumab scores of evaluable KYDAR subjects. Raw difference scores are linearly transformed to a scale from 0 to 100 based on the extremes in the entire KYDAR population. With the exception of subjects with PR at cycle 4, the expected negative trend from CR, over VGPR to PD is evident. The Nature Medicine paper says that some cycle 4 PR patients showed transient response and are not counted as responders. **FIG. 4** shows the responders versus nonresponders. At a cutoff of 20, almost half of the nonresponders are excluded, while all responders are included. At a cutoff of 60, all subjects respond to treatment. **FIG. 5** shows a receiver operating characteristic for DRP-Daratumumab, showing that cutoff 22 and 68 both are optimal in this dataset. Cutoff 22 is preferred for clinical reasons if the DRP-Daratumumab is used in isolation. If may DRPs are applied to the same patient, it is possible that a different cutoff may be relevant to select between treatments.

The analysis was repeated with clone 2 in subjects where clone 2 has been sequenced. The results are similar to the results obtained with clone 1.

### Example 2. Treating a cancer patient with daratumumab

A cancer patient (e.g., one diagnosed with multiple myeloma (MM) or a non-Hodgkin's lymphoma (NHL); e.g., one diagnosed with MM) can be treated with daratumumab after being predicted to be responsive to treatment with daratumumab by the methods described herein. In particular, the cancer patient may be one that has not previously received any cancer treatment or one that has received a cancer treatment other than daratumumab. Moreover, the patient may be one diagnosed with a cancer (e.g., MM or NHL; e.g., MM) or with recurrence thereof.

The cancer patient can be determined to be responsive to daratumumab following the methods disclosed herein. Once the patient has been determined to be responsive to daratumumab, according to the methods described herein, daratumumab may be administered to the patient, for example, by way of intravenous infusion. Daratumumab may be administered by way of intravenous infusion at a rate of 50 mL/hr, 100 mL/hr, 150 mL/hr, or 200 mL/hr and/or over a period of 3.25-6.5 hours. Daratumumab may be administered once a week, once every two weeks, once every three weeks, and/or once every four weeks. Daratumumab may be administered to the patient at a dose of about 16 mg/kg. Daratumumab may be administered as two sub-doses, wherein each sub-dose is half the amount of the dose, e.g., 8 mg/kg each. In some instances, the second sub-dose may be administered one day after administration of the first sub-dose.

Daratumumab may be administered according to one of the following dosing regimens: (a)1 dose per week for weeks 1-8, followed by 1 dose every two weeks starting on week 9 for weeks 9-24, and then followed by 1 dose every four weeks thereafter starting on week 25; (b) 1 dose per week for weeks 1-6, followed by 1 dose every three weeks starting on week 7 for weeks 7-54 starting on week 7, and then followed by 1 dose every four weeks thereafter starting on week 55; (c) 1 dose per week for weeks 1-9, followed by 1 dose every three weeks starting on week 10 for weeks 10-24, and then followed by 1 dose every four weeks thereafter starting on week 25; (d) 1 dose per week for weeks 1-8, followed by 1 dose every two weeks starting on week 9 for weeks 9-16, followed by a pause, and then followed by 1 dose every two weeks for weeks 1-8 after reinitiation of dosing; or (e) 1 dose split into two sub-doses, each comprising half the amount of the 1 dose, administered on Days 1 and 2 of week 1, 1 dose per week for weeks 2-8, followed by 1 dose every two weeks starting on week 9 for weeks 9-24, and then followed by 1 dose every four weeks thereafter starting on week 25.

### Example 3. Predicting responsiveness of a multiple myeloma patient to daratumumab using a panel of biomarkers of sensitivity and biomarkers of resistance to daratumumab.

The diagnostic methods described herein can be used to predict the responsiveness of a multiple myeloma patient to treatment with daratumumab. In particular, the multiple myeloma patient may be one that has not previously received any cancer treatment or one that has received a cancer treatment other than daratumumab. Moreover, the patient may be one diagnosed with multiple myeloma or with recurrence of multiple myeloma.

A biological sample (e.g., a bone marrow sample, such as a bone marrow sample obtained by biopsy) may be obtained from the patient through methods well known in the art. The sample may be frozen and/or prepared, e.g., by formalin fixation and paraffin embedding. In particular, mRNA can be isolated from the sample and a gene expression profile can be determined, e.g., using a microarray platform, such as the Affymetrix HG-U133A, for one or more of the biomarkers shown in Table(s) 1 and/or 2. One or more of the biomarkers shown in Table(s) 1 and/or 2 can also be measured, e.g., by sequencing or PCR-based techniques, such as those described herein.

For example, the level of expression of one or more biomarkers of sensitivity to daratumumab can be determined in the sample from the patient, such as one or more biomarkers of SEQ ID NO: 1-27. The level of expression of one or more biomarkers of resistance to daratumumab can also be determined in the sample from the patient, such as one or more of SEQ ID NO: 28-53. The patient's responsiveness to daratumumab can also be assessed by calculating a difference score for the patient (mean of expression of the biomarkers of sensitivity noted above minus the mean of expression of the biomarkers of resistance noted above).

The multiple myeloma patient would be determined to be responsive to daratumumab when the level of expression of the one or more biomarkers of sensitivity is similar to (e.g., substantially similar to) the level of expression of the biomarkers of sensitivity in a cell or tissue (e.g., tumor tissue) known to be sensitive to daratumumab. The multiple myeloma patient would also be determined to be responsive to daratumumab when the level of expression of one or more of the biomarkers of resistance is similar to (e.g., substantially similar to) the level of expression of the biomarkers of resistance in a cell or tissue (e.g., a tumor tissue) known to be sensitive to daratumumab. The multiple myeloma patient would also be determined to be responsive to daratumumab when a difference score for the patient is similar to (e.g., substantially similar to) a difference score in a cell or tissue (e.g., a tumor tissue) known to be sensitive to daratumumab (e.g., the difference score of the patient is similar to the difference score of a reference subject that has the same cancer as the patient and that has been determined to be responsive to daratumumab).

Alternatively, the multiple myeloma patient may be determined to be responsive to daratumumab if the level of expression of one or more of the biomarkers of sensitivity is dissimilar to (e.g., substantially dissimilar to) the level of expression of the biomarkers of sensitivity in a cell or tissue (e.g., a tumor tissue) known to be resistant to daratumumab. The multiple myeloma patient may also be responsive to daratumumab if the level of expression of one or more of the biomarkers of resistance is dissimilar to (e.g., substantially dissimilar to) the level of expression of the biomarkers of resistance in a cell or tissue (e.g., a tumor tissue) known to be resistant to daratumumab. The multiple myeloma patient would also be determined to be responsive to daratumumab when the difference score for the patient is dissimilar to (e.g., substantially dissimilar to) the difference score in a cell or tissue (e.g., a tumor tissue) known to be resistant to daratumumab (e.g., the difference score of the patient is dissimilar to the difference score of a reference subject that has the same cancer as the patient and that has been determined to be resistant to daratumumab).

If the patient is predicted to be responsive, then the patient can be administered daratumumab, such as daratumumab administered as an intravenous infusion at a dose of about 16 mg/kg. Conversely, if the patient is predicted to be non-responsive to daratumumab treatment, then the patient can be administered one or more therapies other than daratumumab, such as radiation or a therapeutic agent (e.g., bortezomib, and/or another therapeutic agent described herein). For example, the patient can be administered about 16 mg/kg of daratumumab by way of intravenous infusion over a period of 3.25-20 (e.g., 3.25-6.5) hours in accordance with any of the dosing regimens described herein. For patients unable to receive treatment with daratumumab, treatment with bortezomib may be possible.

### Example 6. Predicting responsiveness of multiple myeloma patients to daratumumab using a single biomarker of sensitivity and a single biomarker of resistance to daratumumab.

The diagnostic methods described herein can be used to predict the responsiveness of a multiple myeloma patient to treatment with daratumumab. In particular, the multiple myeloma patient may be one that has not previously received any cancer treatment or one that has received a cancer treatment other than daratumumab. Moreover, the patient may be one diagnosed with multiple myeloma or with recurrence of multiple myeloma.

A biological sample (e.g., a bone marrow sample, such as a bone marrow sample obtained by biopsy) may be obtained from the patient through methods well known in the art. The sample may be frozen and/or prepared, e.g., by formalin fixation and paraffin embedding. The sample can be subjected to CD138 positive cell selection using commercially available selection kits. In particular, mRNA can be isolated from the sample and a gene expression profile can be determined, e.g., using a microarray platform, such as the Affymetrix HG-U133A, for one or more of the biomarkers shown in Table(s) 1 and/or 2. One or more of the biomarkers shown in Table(s) 1 and/or 2 can also be measured, e.g., by sequencing or PCR-based techniques, such as those described herein.

In particular, the level of expression of a single biomarker of sensitivity to daratumumab can be determined in the sample from the patient, such as, e.g., the biomarker NR3C1 (SEQ ID NO: 1). The level of expression of a single biomarker of resistance to daratumumab can also be determined in the sample from the patient, such as, e.g., the biomarker CCND1 (SEQ ID NO: 28). The patient's responsiveness to daratumumab can also be assessed by calculating a difference score for the patient (the level of expression of NR3C1 (SEQ ID NO: 1) minus the level of expression of CCND1 (SEQ ID NO: 28)).

The multiple myeloma patient would be determined to be responsive to daratumumab when the level of expression of NR3C1 (SEQ ID NO: 1) is similar to (e.g., substantially similar to) the level of expression of NR3C1 (SEQ ID NO: 1) in a cell or tissue (e.g., tumor tissue) known to be sensitive to daratumumab. The multiple myeloma patient would also be determined to be responsive to daratumumab when the level of expression of CCND1 (SEQ ID NO: 28) is similar to (e.g., substantially similar to) the level of expression of CCND1 (SEQ ID NO: 28) in a cell or tissue (e.g., a tumor tissue) known to be sensitive to daratumumab. The multiple myeloma patient would also be determined to be responsive to daratumumab when the difference score for the patient is similar to (e.g., substantially similar to) the difference score in a cell or tissue (e.g., a tumor tissue) known to be sensitive to daratumumab (e.g., the difference score of the patient is similar to the difference score of a reference subject that has the same cancer as the patient and that has been determined to be responsive to daratumumab).

Alternatively, the multiple myeloma patient may be determined to be responsive to daratumumab if the level of expression of NR3C1 (SEQ ID NO: 1) is dissimilar to (e.g., substantially dissimilar to) the level of expression of NR3C1 (SEQ ID NO: 1) in a cell or tissue (e.g., a tumor tissue) known to be resistant to daratumumab. The multiple myeloma patient may also be responsive to daratumumab if the level of expression of CCND1 (SEQ ID NO: 28) is dissimilar to (e.g., substantially dissimilar to) the level of expression of CCND1 (SEQ ID NO: 28) in a cell or tissue (e.g., a tumor tissue) known to be resistant to daratumumab. The multiple myeloma patient would also be determined to be responsive to daratumumab when difference score for the patient is dissimilar to (e.g., substantially dissimilar to) the difference score in a cell or tissue (e.g., a tumor tissue) known to be resistant to daratumumab (e.g., the difference score of the patient is dissimilar to the difference score of a reference subject that has the same cancer as the patient and that has been determined to be resistant to daratumumab).

If the patient is predicted to be responsive, then the patient can be administered daratumumab, such as daratumumab administered as an intravenous infusion at a dose of about 16 mg/kg. Conversely, if the patient is predicted to be non-responsive to daratumumab treatment, then the patient can be administered one or more therapies other than daratumumab, such as radiation or a therapeutic agent (e.g., bortezomib, and/or another therapeutic agent described herein). For example, the patient can be administered about 16 mg/kg of daratumumab by way of intravenous infusion over a period of 3.25-6.5 hours according to any of the dosing regimens described herein.

### OTHER EMBODIMENTS

All publications, patents, and patent applications mentioned in the above specification are hereby incorporated by reference. Various modifications and variations of the described device and methods of use of the invention will be apparent to those skilled in the art without departing from the scope and spirit of the invention. Although the invention has been described in connection with specific embodiments, it should be understood that the invention as claimed should not be unduly limited to such specific embodiments. Indeed, various modifications of the described modes for carrying out the invention that are obvious to those skilled in the art are intended to be within the scope of the invention. For example, it is anticipated that measuring the level of proteins, metabolites, identifying genetic mutations and DNA copy number variations, all will be useful in determining patient responsiveness.

Some embodiments of the invention described herein can be defined according to any one of the following numbered embodiments.
1. A method of determining responsiveness of a subject with a cancer to daratumumab comprising:
   (a) determining the level of expression of one or more biomarkers of sensitivity selected from the biomarkers of Table 1; and/or
   (b) determining the level of expression of one or more biomarkers of resistance selected from the biomarkers of Table 2.
2. The method of embodiment 1, wherein the subject is determined to be responsive to daratumumab if:
   (a) the level of expression of the one or more biomarkers of sensitivity is substantially similar to the level of expression of the one or more biomarkers of sensitivity in a cell or tissue known to be sensitive to daratumumab;
   (b) the level of expression of the one or more biomarkers of resistance is substantially similar to the level of expression of the one or more biomarkers of resistance in a cell or tissue known to be sensitive to daratumumab;
   (c) the level of expression of the one or more biomarkers of sensitivity is substantially dissimilar to the level of expression of the one or more biomarkers of sensitivity in a cell or tissue known to be resistant to daratumumab; and/or
   (d) the level of expression of the one or more biomarkers of resistance is substantially dissimilar to the level of expression of the one or more biomarkers of resistance in a cell or tissue known to be resistant to daratumumab.
3. The method of embodiment 2, wherein:
   (a) sensitivity of a cell or tissue known to be sensitive to daratumumab is based on Cancer Cell Line Encyclopedia (CCLE) data of B cell lymphoma cell lines; and/or
   (b) resistance of a cell or tissue known to be resistant to daratumumab is based on CCLE data of B cell lymphoma cell lines.
4. The method of any one of embodiments 1-3, further comprising administering daratumumab to the subject determined to be sensitive to daratumumab.
5. A method of treating a cancer in a subject in need thereof comprising administering daratumumab to the subject, wherein the subject has been determined to be responsive to daratumumab according to the method of any one of embodiments 1-3.
6. A method of treating a subject with a cancer comprising:
   (a) determining the level of expression of one or more biomarkers of sensitivity and/or determining the level of expression of one or more biomarkers of resistance; and
   (b) administering daratumumab to the subject based on the level of expression of the one or more biomarkers of sensitivity and/or the one or more biomarkers of resistance.
7. The method of embodiment 6, wherein the subject is administered daratumumab if:
   (a) the level of expression of the one or more biomarkers of sensitivity is substantially similar to the level of expression of the one or more biomarkers of sensitivity in a cell or tissue known to be sensitive to daratumumab;
   (b) the level of expression of the one or more biomarkers of resistance is substantially similar to the level of expression of the one or more biomarkers of resistance in a cell or tissue known to be sensitive to daratumumab;
   (c) the level of expression of the one or more biomarkers of sensitivity is substantially dissimilar to the level of expression of the one or more biomarkers of sensitivity in a cell or tissue known to be resistant to daratumumab; and/or
   (d) the level of expression of the one or more biomarkers of resistance is substantially dissimilar to the level of expression of the one or more biomarkers of resistance in a cell or tissue known to be resistant to daratumumab.
8. The method of embodiment 7, wherein:
   (a) sensitivity of a cell or tissue known to be sensitive to daratumumab is based on CCLE data of B cell lymphoma cell lines; and/or
   (b) resistance of a cell or tissue known to be resistant to daratumumab is based on CCLE data of B cell lymphoma cell lines.
9. The method of any one of embodiments 4-8, further comprising administering one or more therapies other than daratumumab to the subject, wherein, optionally, the one or more therapies comprises surgery, radiation, or a therapeutic agent.
10. The method of embodiment 9, wherein the therapeutic agent comprises a corticosteroid, an antipyretic, an antihistamine, a chemotherapeutic agent, or a combination thereof.
11. The method of embodiment 10, wherein:
   (a) the corticosteroid is methylprednisolone or dexamethasone;
   (b) the antipyretic is acetaminophen/paracetamol; and/or
   (c) the antihistamine is diphenhydramine.
12. The method of any one of embodiments 4-11, further comprising administering to the subject lenalidomide, dexamethasone, bortezomib, melphalan, prednisone, thalidomide, pomalidomide, or a combination thereof.
13. The method of any one of embodiments 4-12, further comprising administering to the subject:
   (a) daratumumab and lenalidomide and dexamethasone;
   (b) daratumumab and bortezomib and melphalan and prednisone;
   (c) daratumumab and bortezomib and thalidomide and dexamethasone;
   (d) daratumumab and bortezomib and dexamethasone; or
   (e) daratumumab and pomalidomide and dexamethasone.
14. The method of any one of embodiments 4-13, wherein daratumumab is administered intravenously.
15. The method of embodiment 14, wherein daratumumab is administered via intravenous infusion.
16. The method of any one of embodiments 4-15, wherein a dose of daratumumab is administered once a week, once every two weeks, once every three weeks, and/or once every four weeks.
17. The method of embodiment 16, wherein the dose of daratumumab administered to the subject is about 16 mg/kg.
18. The method of embodiment 16 or 17, wherein the dose of daratumumab is administered as two sub-doses, wherein each sub-dose is half the amount of the dose.
19. The method of embodiment 18, wherein the second sub-dose is administered one day after administration of the first sub-dose.
20. The method of any one of embodiments 4-19, wherein daratumumab is administered in a dosing schedule comprising:
   (a) 1 dose per week for weeks 1-8, followed by 1 dose every two weeks starting on week 9 for weeks 9-24, and then followed by 1 dose every four weeks thereafter starting on week 25;
   (b) 1 dose per week for weeks 1-6, followed by 1 dose every three weeks starting on week 7 for weeks 7-54 starting on week 7, and then followed by 1 dose every four weeks thereafter starting on week 55;
   (c) 1 dose per week for weeks 1-9, followed by 1 dose every three weeks starting on week 10 for weeks 10-24, and then followed by 1 dose every four weeks thereafter starting on week 25;
   (d) 1 dose per week for weeks 1-8, followed by 1 dose every two weeks starting on week 9 for weeks 9-16, followed by a pause, and then followed by 1 dose every two weeks for weeks 1-8 after reinitiation of dosing; or
   (e) 1 dose split into two sub-doses, each comprising half the amount of the 1 dose, administered on Days 1 and 2 of week 1, 1 dose per week for weeks 2-8, followed by 1 dose every two weeks starting on week 9 for weeks 9-24, and then followed by 1 dose every four weeks thereafter starting on week 25.
21. The method of any one of embodiments 1-20, wherein the level of expression of the one or more biomarkers of sensitivity and/or the level of expression one or more biomarkers of resistance is determined using a device.
22. The method of embodiment 21, wherein the device is a microarray.
23. The method of embodiment 22, wherein the microarray is a deoxyribonucleic acid (DNA)-based platform.
24. The method of embodiment 22 or 23, wherein the device comprises at least two, at least three, at least four, at least five, at least six, at least seven, at least eight, at least nine, at least ten, or more single-stranded nucleic acid molecules of capable of hybridizing to the one or more biomarkers of sensitivity and/or the one or more biomarkers of resistance.
25. The method of embodiment 24, wherein the one or more single-stranded nucleic acid molecules of the device have a length in the range of 10-100 nucleotides.
26. The method of any one of embodiments 1-25, wherein the level of expression of the one or more biomarkers of sensitivity and/or the level of expression of the one or more biomarkers of resistance is determined by microarray analysis, mass spectrometry, a nucleic acid amplification method, a nucleic acid sequencing method, an imaging-based method, or a gel-based method.
27. The method of embodiment 26, wherein:
   (a) the nucleic acid amplification method comprises polymerase chain reaction (PCR), reverse transcriptase PCR (RT-PCR), or quantitative real-time PCR (qPCR); or
   (b) the nucleic acid sequencing method comprises pyrosequencing, nanopore sequencing, sequencing by synthesis, sequencing by expansion, sequencing by ligation, or next generation sequencing;
   (c) the imaging-based method comprises fluorescence *in situ* hybridization, enzyme-linked immunosorbent assay (ELISA), nanostring, or immunohistochemistry; or
   (d) the gel-based method comprises a Northern blot, a Western blot, or a Southern blot.
28. The method of any one of embodiments 1-27, wherein the level of expression of the one or more biomarkers of sensitivity and/or the level of expression of the one or more biomarkers of resistance is an RNA expression level or a protein expression level.
29. The method of embodiment 28, wherein the RNA expression level is an mRNA expression level.
30. The method of any one of embodiments 1-29, wherein the method comprises converting the level of expression of the one or more biomarkers of sensitivity and/or the level of expression of the one or more biomarkers of resistance into a mean score, wherein the mean score indicates the responsiveness of the subject to daratumumab.
31. The method of embodiment 30, further comprising subtracting the mean score for the one or more biomarkers of resistance from the mean score for the one or more biomarkers of sensitivity to obtain a difference score, wherein the difference score indicates the responsiveness of the subject to daratumumab.
32. The method of any one of embodiments 1-31, wherein the level of expression of the one or more biomarkers of sensitivity and/or the level of expression of the one or more biomarkers of resistance is determined in a bone marrow sample or a blood sample obtained from the subject.
33. The method of embodiment 32, wherein the bone marrow sample and/or the blood sample comprises plasma cells.
34. The method of embodiment 32 or 33, wherein the mean score for the one or more biomarkers of sensitivity and/or the difference score above a cutoff value indicates that the subject is responsive to daratumumab.
35. The method of embodiment 34, wherein the cutoff value is established as the 50^{th} percentile, 60th percentile, 70^{th} percentile, 80^{th} percentile, 90^{th} percentile, or greater of a set of reference mean scores or a set of reference difference scores determined in a reference population.
36. The method of embodiment 35, wherein the reference population comprises individuals having the same cancer as the subject.
37. The method of embodiment 35 or 36, wherein the set of reference mean scores or the set of reference difference scores is:
   (a) a set comprising the mean scores for the one or more biomarkers of sensitivity calculated for each individual of the reference population;
   (b) a set comprising the mean scores for the one or more biomarkers of resistance calculated for each individual of the reference population; or
   (c) a set comprising the difference scores calculated for each individual of the reference population.
38. The method of any one of embodiments 35-37, wherein the reference mean score or the reference difference score is determined in a reference bone marrow sample or a reference blood sample obtained from the individuals of the reference population.
39. The method of embodiment 38, wherein the reference bone marrow sample or the reference blood sample comprises plasma cells.
40. The method of any one of embodiments 1-39, wherein:
   (a) the biomarkers of sensitivity are selected from at least 5, at least 10, at least 15, at least 20, at least 25, or all 27 of the biomarkers of Table 1; and/or the biomarkers of resistance are selected from at least 5, at least 10, at least 15, at least 20, at least 25, or all 26 of the biomarkers of Table 2;
   (b) the biomarkers of sensitivity are selected from one or more of SEQ ID NOs: 1-27 and/or the biomarkers of resistance are selected from one or more of SEQ ID NOs: 28-53; and/or
   (c) the biomarker of sensitivity is NR3C1 (SEQ ID NO: 1); and/or the biomarker of resistance is CCND1 (SEQ ID NO: 28).
41. The method of any one of embodiments 1-40, wherein the biomarker of sensitivity is NR3C1 and the biomarker of resistance is CCND1.
42. The method of any one of embodiments 1-41, wherein the cancer is multiple myeloma.
43. The method of embodiment 42, wherein the subject has not received prior therapy for treating the multiple myeloma.
44. The method of embodiment 42, wherein the subject has relapsed after or is refractory to at least one prior therapy for treating the multiple myeloma.
45. The method of embodiment 44, wherein the at least one prior therapy comprises an alkylating agent, a proteasome inhibitor, and/or an immunomodulatory agent.
46. The method of embodiment 44, wherein the at least one prior therapy comprises bortezomib, lenalidomide, pomalidomide, carfilzomib, autologous stem cell transplant therapy, or a combination thereof.
47. The method of embodiment 44, wherein the subject has relapsed after or is refractory to one, two, or three prior lines of therapy.
48. The method of any one of embodiments 1-41, wherein the cancer is a B cell lymphoma.
49. The method of embodiment 48, wherein the B cell lymphoma is a non-Hodgkin's lymphoma (NHL).
50. The method of any one of embodiments 1-49, wherein the subject is ineligible for autologous stem cell transplant therapy.

## Claims

1. A method of determining responsiveness of a subject with a cancer to daratumumab comprising:
(a) determining a level of expression of one or more biomarkers of sensitivity selected from the biomarkers of Table 1; and/or
(b) determining a level of expression of one or more biomarkers of resistance selected from the biomarkers of Table 2.

2. The method of claim 1, wherein the subject is determined to be responsive to daratumumab if:
(a) the level of expression of the one or more biomarkers of sensitivity is substantially similar to the level of expression of the one or more biomarkers of sensitivity in a cell or tissue known to be sensitive to daratumumab;
(b) the level of expression of the one or more biomarkers of resistance is substantially similar to the level of expression of the one or more biomarkers of resistance in a cell or tissue known to be sensitive to daratumumab;
(c) the level of expression of the one or more biomarkers of sensitivity is substantially dissimilar to the level of expression of the one or more biomarkers of sensitivity in a cell or tissue known to be resistant to daratumumab; and/or
(d) the level of expression of the one or more biomarkers of resistance is substantially dissimilar to the level of expression of the one or more biomarkers of resistance in a cell or tissue known to be resistant to daratumumab.

3. The method of claim 2, wherein:
(a) sensitivity of a cell or tissue known to be sensitive to daratumumab is based on Cancer Cell Line Encyclopedia (CCLE) data of B cell lymphoma cell lines; and/or
(b) resistance of a cell or tissue known to be resistant to daratumumab is based on CCLE data of B cell lymphoma cell lines.

4. The method of any one of claims 1-3, further comprising administering daratumumab to the subject determined to be sensitive to daratumumab.

5. A method of treating a cancer in a subject in need thereof comprising administering daratumumab to the subject, wherein the subject has been determined to be responsive to daratumumab according to the method of any one of claims 1-3.

6. A method of treating a subject with a cancer comprising:
(a) determining a level of expression of one or more biomarkers of sensitivity and/or determining a level of expression of one or more biomarkers of resistance; and
(b) administering daratumumab to the subject based on the level of expression of the one or more biomarkers of sensitivity and/or the one or more biomarkers of resistance.

7. The method of claim 6, wherein the subject is administered daratumumab if:
(a) the level of expression of the one or more biomarkers of sensitivity is substantially similar to the level of expression of the one or more biomarkers of sensitivity in a cell or tissue known to be sensitive to daratumumab;
(b) the level of expression of the one or more biomarkers of resistance is substantially similar to the level of expression of the one or more biomarkers of resistance in a cell or tissue known to be sensitive to daratumumab;
(c) the level of expression of the one or more biomarkers of sensitivity is substantially dissimilar to the level of expression of the one or more biomarkers of sensitivity in a cell or tissue known to be resistant to daratumumab; and/or
(d) the level of expression of the one or more biomarkers of resistance is substantially dissimilar to the level of expression of the one or more biomarkers of resistance in a cell or tissue known to be resistant to daratumumab.

8. The method of claim 7, wherein:
(a) sensitivity of a cell or tissue known to be sensitive to daratumumab is based on CCLE data of B cell lymphoma cell lines; and/or
(b) resistance of a cell or tissue known to be resistant to daratumumab is based on CCLE data of B cell lymphoma cell lines.

9. The method of any one of claims 4-8, further comprising administering one or more therapies other than daratumumab to the subject, wherein, optionally, the one or more therapies comprises surgery, radiation, or a therapeutic agent.

10. The method of claim 9, wherein the therapeutic agent comprises a corticosteroid, an antipyretic, an antihistamine, a chemotherapeutic agent, or a combination thereof.

11. The method of claim 10, wherein:
(a) the corticosteroid is methylprednisolone or dexamethasone;
(b) the antipyretic is acetaminophen/paracetamol; and/or
(c) the antihistamine is diphenhydramine.

12. The method of any one of claims 4-11, further comprising administering lenalidomide, dexamethasone, bortezomib, melphalan, prednisone, thalidomide, pomalidomide, or a combination thereof to the subject.

13. The method of any one of claims 4-11, further comprising administering to the subject:
(a) daratumumab and lenalidomide and dexamethasone;
(b) daratumumab and bortezomib and melphalan and prednisone;
(c) daratumumab and bortezomib and thalidomide and dexamethasone;
(d) daratumumab and bortezomib and dexamethasone; or
(e) daratumumab and pomalidomide and dexamethasone.

14. The method of any one of claims 4-13, wherein daratumumab is administered intravenously.

15. The method of claim 14, wherein daratumumab is administered via intravenous infusion.

16. The method of any one of claims 4-15, wherein a dose of daratumumab is administered once a week, once every two weeks, once every three weeks, and/or once every four weeks.

17. The method of claim 16, wherein the dose of daratumumab administered to the subject is about 16 mg/kg.

18. The method of claim 16 or 17, wherein the dose of daratumumab is administered as two sub-doses, wherein each sub-dose is half the amount of the dose.

19. The method of claim 18, wherein the second sub-dose is administered one day after administration of the first sub-dose.

20. The method of any one of claims 4-19, wherein daratumumab is administered in a dosing schedule comprising:
(a) 1 dose per week for weeks 1-8, followed by 1 dose every two weeks starting on week 9 for weeks 9-24, and then followed by 1 dose every four weeks thereafter starting on week 25;
(b) 1 dose per week for weeks 1-6, followed by 1 dose every three weeks starting on week 7 for weeks 7-54, and then followed by 1 dose every four weeks thereafter starting on week 55;
(c) 1 dose per week for weeks 1-9, followed by 1 dose every three weeks starting on week 10 for weeks 10-24, and then followed by 1 dose every four weeks thereafter starting on week 25;
(d) 1 dose per week for weeks 1-8, followed by 1 dose every two weeks starting on week 9 for weeks 9-16, followed by a pause, and then followed by 1 dose every two weeks for weeks 1-8 after reinitiation of dosing; or
(e) 1 dose split into two sub-doses, each comprising half the amount of the 1 dose, administered on Days 1 and 2 of week 1, 1 dose per week for weeks 2-8, followed by 1 dose every two weeks starting on week 9 for weeks 9-24, and then followed by 1 dose every four weeks thereafter starting on week 25.

21. The method of any one of claims 1-20, wherein the level of expression of the one or more biomarkers of sensitivity and/or the level of expression one or more biomarkers of resistance is determined using a device.

22. The method of claim 21, wherein the device is a microarray.

23. The method of claim 22, wherein the microarray is a deoxyribonucleic acid (DNA)-based platform.

24. The method of claim 22 or 23, wherein the device comprises at least two, at least three, at least four, at least five, at least six, at least seven, at least eight, at least nine, at least ten, or more single-stranded nucleic acid molecules of capable of hybridizing to the one or more biomarkers of sensitivity and/or the one or more biomarkers of resistance.

25. The method of claim 24, wherein the one or more single-stranded nucleic acid molecules of the device have a length in the range of 10-100 nucleotides.

26. The method of any one of claims 1-25, wherein the level of expression of the one or more biomarkers of sensitivity and/or the level of expression of the one or more biomarkers of resistance is determined by microarray analysis, mass spectrometry, a nucleic acid amplification method, a nucleic acid sequencing method, an imaging-based method, or a gel-based method.

27. The method of claim 26, wherein:
(a) the nucleic acid amplification method comprises polymerase chain reaction (PCR), reverse transcriptase PCR (RT-PCR), or quantitative real-time PCR (qPCR); or
(b) the nucleic acid sequencing method comprises pyrosequencing, nanopore sequencing, sequencing by synthesis, sequencing by expansion, sequencing by ligation, or next generation sequencing;
(c) the imaging-based method comprises fluorescence *in situ* hybridization, enzyme-linked immunosorbent assay (ELISA), nanostring, or immunohistochemistry; or
(d) the gel-based method comprises a Northern blot, a Western blot, or a Southern blot.

28. The method of any one of claims 1-27, wherein the level of expression of the one or more biomarkers of sensitivity and/or the level of expression of the one or more biomarkers of resistance is an RNA expression level or a protein expression level.

29. The method of claim 28, wherein the RNA expression level is an mRNA expression level.

30. The method of any one of claims 1-29, wherein the method comprises converting the level of expression of the one or more biomarkers of sensitivity and/or the level of expression of the one or more biomarkers of resistance into a mean score, wherein the mean score indicates the responsiveness of the subject to daratumumab.

31. The method of claim 30, further comprising subtracting the mean score for the one or more biomarkers of resistance from the mean score for the one or more biomarkers of sensitivity to obtain a difference score, wherein the difference score indicates the responsiveness of the subject to daratumumab.

32. The method of any one of claims 1-31, wherein the level of expression of the one or more biomarkers of sensitivity and/or the level of expression of the one or more biomarkers of resistance is determined in a bone marrow sample or a blood sample obtained from the subject.

33. The method of claim 32, wherein the bone marrow sample and/or the blood sample comprises plasma cells.

34. The method of any one of claims 30-33, wherein the mean score for the one or more biomarkers of sensitivity and/or the difference score above a cutoff value indicates that the subject is responsive to daratumumab.

35. The method of claim 34, wherein the cutoff value is established as the 50^{th} percentile, 60th percentile, 70^{th} percentile, 80^{th} percentile, 90^{th} percentile, or greater relative to a reference, wherein optionally the reference comprises a set of reference mean scores or a set of reference difference scores determined in a reference population.

36. The method of claim 35, wherein the reference population comprises individuals having the same cancer as the subject.

37. The method of claim 35 or 36, wherein the set of reference mean scores or the set of reference difference scores is:
(a) a set comprising the mean scores for the one or more biomarkers of sensitivity calculated for each individual of the reference population;
(b) a set comprising the mean scores for the one or more biomarkers of resistance calculated for each individual of the reference population; or
(c) a set comprising the difference scores calculated for each individual of the reference population.

38. The method of any one of claims 35-37, wherein the reference mean score or the reference difference score is determined in a reference bone marrow sample or a reference blood sample obtained from the individuals of the reference population.

39. The method of claim 38, wherein the reference bone marrow sample or the reference blood sample comprises plasma cells.

40. The method of any one of claims 1-39, wherein:
(a) the biomarkers of sensitivity are selected from at least 5, at least 10, at least 15, at least 20, at least 25, or all 27 of the biomarkers of Table 1; and/or the biomarkers of resistance are selected from at least 5, at least 10, at least 15, at least 20, at least 25, or all 26 of the biomarkers of Table 2;
(b) the biomarkers of sensitivity are selected from one or more of SEQ ID NOs: 1-27 and/or the biomarkers of resistance are selected from one or more of SEQ ID NOs: 28-53; and/or
(c) the biomarker of sensitivity is NR3C1 (SEQ ID NO: 1); and/or the biomarker of resistance is CCND1 (SEQ ID NO: 28).

41. The method of any one of claims 1-40, wherein the biomarker of sensitivity is NR3C1 and the biomarker of resistance is CCND1.

42. The method of any one of claims 1-41, wherein the cancer is multiple myeloma.

43. The method of claim 42, wherein the subject has not received prior therapy for treating the multiple myeloma.

44. The method of claim 42, wherein the subject has relapsed after or is refractory to at least one prior therapy for treating the multiple myeloma.

45. The method of claim 44, wherein the at least one prior therapy comprises an alkylating agent, a proteasome inhibitor, and/or an immunomodulatory agent.

46. The method of claim 44, wherein the at least one prior therapy comprises bortezomib, lenalidomide, pomalidomide, carfilzomib, autologous stem cell transplant therapy, or a combination thereof.

47. The method of claim 44, wherein the subject has relapsed after or is refractory to one, two, or three prior lines of therapy.

48. The method of any one of claims 1-41, wherein the cancer is a B cell lymphoma.

49. The method of claim 48, wherein the B cell lymphoma is a non-Hodgkin's lymphoma (NHL).

50. The method of any one of claims 1-49, wherein the subject is ineligible for autologous stem cell transplant therapy.
